# EUROPÄISCHE PATENTSCHRIFT

(11) **EP 1 141 716 B1**
(45) Veröffentlichungstag und Bekanntmachung des Hinweises auf die Patenterteilung: **05.11.2008**
(21) Anmeldenummer: 99965576.4
(22) Anmeldetag: 29.12.1999
(51) Int. Cl.: G01N 33/566, C12N 5/10, C12Q 1/02

(54) **METHODE ZUR ZELLULÄREN HIGH-THROUGHPUT-DETEKTION VON REZEPTOR-LIGANDEN-INTERAKTIONEN**
METHOD FOR THE CELLULAR HIGH-THROUGHPUT-DETECTION OF RECEPTOR LIGAND INTERACTIONS
METHODE DE DETECTION CELLULAIRE A HAUT RENDEMENT D'INTERACTIONS ENTRE DES RECEPTEURS NUCLEAIRES ET DES LIGANDS

(30) Priorität: 30.12.1998 DE 19860833
(43) Veröffentlichungstag der Anmeldung: 10.10.2001
(73) Patentinhaber: Forster, Hansjörg, Dr., 79856 Hinterzarten (DE); Zimmermann, André, 72072 Tübingen (DE)
(72) Erfinder: Sippel Albrecht E., 79104 Freiburg (DE); Zimmermann, André, 72072 Freiburg (DE)
(74) Vertreter: Weiss, Wolfgang
(86) Internationale Anmeldenummer: PCT/EP1999/010460
(87) Internationale Veröffentlichungsnummer: WO 2000/040969

(56) Entgegenhaltungen:
- EP-A- 0 863 214
- WO-A-93/07294
- WO-A-94/29458
- WO-A-95/23231
- WO-A-97/46688
- WO-A-97/48820
- WO-A-98/26054
- WO-A-99/02675

## Beschreibung

Die Anmeldung bezieht sich auf das Gebiet der Molekularbiologie. Sie betrifft insbesondere Assayverfahren, die dazu dienen, spezifische Interaktionen zwischen einem extrazellulären Ligand und einem insbesondere membranständigen Rezeptor zu detektieren, und zielt u.a. darauf ab, neuartige, funktionelle Liganden für Rezeptoren zu finden wie auch eine Ligandenbindungsfunktion, die für insbesondere membranständige Rezeptoren charakteristisch ist, bei Polypeptiden oder Proteinen, bei denen eine solche Funktion vermutet wird, gegebenenfalls nachzuweisen. In diesem Zusammenhang betrifft die Anmeldung heterologe Membranrezeptoren, insbesondere Fusionsproteine, Nukleinsäuren, die diese Membranrezeptoren, insbesondere Fusionsproteine kodieren, Vektoren, die diese Nukleinsäuren enthalten, transformierte Hefezellen die diese Membranrezeptoren, insbesondere Fusionsproteine enthalten, sowie Kits, die sämtlich für die erfindungsgemäßen Assayverfahren oder in Zusammenhang mit diesen eingesetzt werden können.

Bei den im folgenden genannten Zellen, die für die Assays dieser Anmeldung eingesetzt bzw. benutzt werden, handelt es sich ausschliesslich um Hefezellen. Alle genannten Membranrezeptoren und Fusionsproteine sind heterolog.

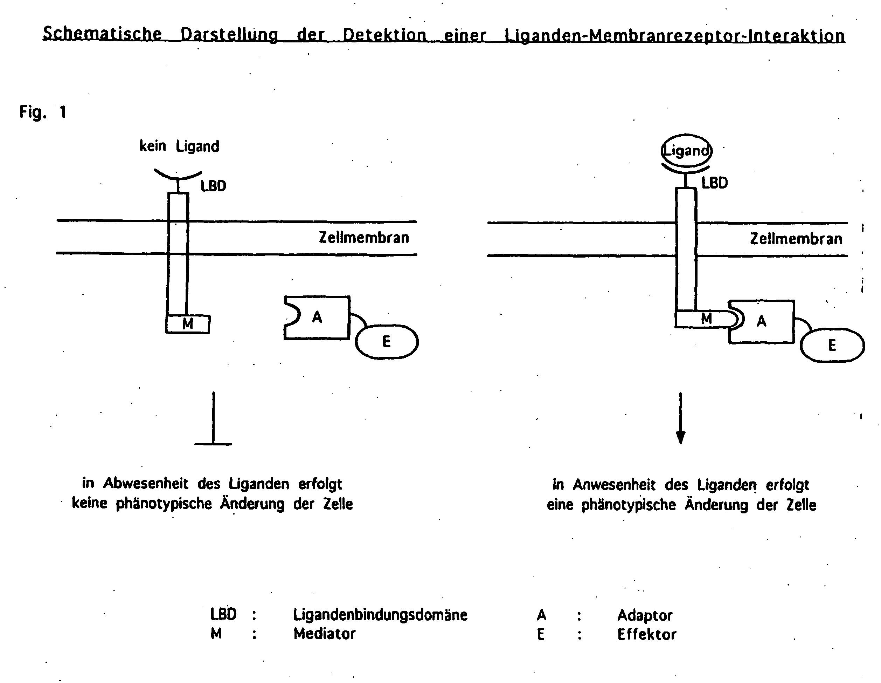

Zellen, vor allem Zellen in vielzelligen Organismen, sind darauf angewiesen, angemessen auf extrazelluläre Signale aus ihrer Umgebung zu reagieren. Signalzielzellen benutzen zur spezifischen Signalaufnahme membranständige Rezeptorproteine. Durch Bindung extrazellulärer Ligandenmoleküle an die extrazellulären Abschnitte des Rezeptors (Ligandenbindungsabschnitt) erfolgt eine konformationsvermittelte Signalweiterleitung über den Transmembranbereich des Rezeptors zum zytoplasmatischen Abschnitt des Rezeptors im Zellinnern (Mediatorabschnitt). So kann auf der Zytoplasmaseite des Rezeptors die Ligandenbindungs-abhängige molekulare Veränderung durch Adaptormoleküle abgegriffen und an innerzelluläre Signaltransduktionswege weitergeleitet werden (Figur 1). Effekte der Rezeptor-vermittelten Signaltransduktion sind rasche Veränderungen an zytoplasmatischen Strukturen und Prozesse der Genaktivitätsveränderung und der Vermehrung oder des Abbaus von genetischem Material im Zellkern.

Membranrezeptoren vermitteln also ihre intrazelluläre Signalweiterleitung über den zytoplasmatischen Mediatorabschnitt des Rezeptors. Je nach der Beschaffenheit dieses Mediatorabschnitts teilt man die Membranrezeptoren in solche ein, bei denen die Signalweiterleitung des Mediators über eine enzymatische Proteinkinasereaktion (engl. enzyme-coupled receptors) oder über sogenannte G-Proteine (engl. G-protein-coupled receptors) erfolgt.

Zu den Enzym-gekoppelten Rezeptoren gehören solche, bei denen der enzymatisch aktive Mediatorabschnitt direkt im selben Proteinmolekül des Rezeptors sitzt, das auch den Ligandenbindungsabschnitt trägt. Paradebeispiel für diesen Rezeptortyp ist der epidermaler wachstumsfaktor ("epidermal growth factor"; EGF)-Rezeptor (Schlessinger und Ulrich, 1992). Bei anderen Enzym-gekoppelten Rezeptoren liegt der enzymatisch aktive Proteinabschnitt in einer zweiten Proteinuntereinheit vor. Paradebeispiele für diesen Typ sind viele Zytokinrezeptoren (Stahl und Yancopoulos, 1993). Gemeinsam ist allen, daß die Proteinkinaseaktivität des Mediatorabschnitts des Rezeptors strikt von der Bindung des Liganden an die Ligandenbindungsdomäne des Rezeptors abhängig ist. Viele der Enzym-gekoppelten Rezeptoren geben ihr Signal über Adaptorproteine und -polypeptide an Mitglieder der Ras-Familie von monomeren GTPasen weiter, die unter anderem eine Serin/Threonin-Phosphorylierungskaskade aktivieren können (Nishida und Gotoh, 1993).

Bei den G-Protein-gekoppelten Rezeptoren wird Ligandenbindungs-abhängig die zytoplasmatische Rezeptorkonfiguration durch einen G-Proteinkomplex abgegriffen, der aus drei Untereinheiten besteht und der das Signal durch Dissoziation in einen aktivierten membrangebunden βγ-Teil und in eine aktivierte Gα-Proteinuntereinheit weiterleitet. Dabei tauscht die Gα-Proteinuntereinheit ihren für den inaktiven Zustand charakteristischen Kofaktor-GDP gegen den für den aktivierten Zustand charakteristischen Kofaktor-GTP aus, der nach GTP zu GDP-Hydrolyse die Gα-Proteinuntereinheit wieder inaktiviert. An diesen Kreislauf sind über die aktivierte βγ-Untereinheit und/oder die aktivierte α-Untereinheit des G-Proteinkomplexes als Mediatoren die verschiedensten Signaltransduktionswege angekoppelt. Zu den G-Proteingekoppelten Rezeptoren gehört die große Familie der 7-Transmembranrezeptoren. Diese umfassen z. B. die Toxinrezeptoren, die Geruchsrezeptoren, Neurotransmitterrezeptoren und viele Hundert mehr (Dohlman et al.; 1991, Leurs et al., 1998). Adaptorproteine für die G-Protein-Mediatoren sind z. B. Adenylatzyklasen, die Phospholipase C und andere G-Protein gekoppelte Rezeptorkinasen, sowie bestimmte Ionenkanäle.

Fusionsproteine, welche aus einem Effektorabschnitt und einem Adaptorprotein bestehen und Membranrezeptor-vermittelte Signaltransduktionswege in einer Zelle aktivieren können, sind im Stand der Technik, bekannt. So beschreiben Cheng et al. (Cell (1998), 95, 793-803) im Rahmen von Untersuchungen zur biologischen Funktion von SH2/SH3-Adaptoren während der Embryonalentwicklung bzw. der Entstehung maligner Tumore ein in embryonalen Mausstammzellen exprimiertes Fusionsprotein, welches aus einem Effektorabschnitt, hier dem als Ras-Aktivator fungierenden Sos1-Protein, sowie der als Adaptorprotein fungierenden SH2-Domäne von Grb2 besteht.

Ferner offenbaren Aronheim et al. (Cell (1994), 78, 949-961) im Rahmen von Studien zur Aktivierung des Ras-Signaltransduktionswegs mittels des Nukleotidaustauschfaktors Sos Fusionsproteine aus einem Sos-Effektorabschnitt und einem Adaptorprotein, welche unter Verwendung geeigneter Vektoren in HeLa-Zellen exprimiert werden. Als Adaptorprotein finden das Myristylierungssignal von v-Src bzw. das Farnesylierungs- und Palmitylierungssignal von c-Ha-Ras Anwendung.

Membranrezeptor-vermittelte Signaltransduktionen regeln die entscheidenden intrazellulären Prozesse zum Überleben der Zellen, zu Wachstum und Zellteilung, zur Zelldifferenzierung und zum Zelltod (der Apoptose). Fehler in diesen wichtigen Kontrollwegen, ausgelöst durch Mutationen, tragen häufig zur Krankheits- und insbesondere zur Krebsentstehung bei. Es ist verständlich, daß die Liganden-Rezeptor-Interaktion von großem wissenschaftlichen, pharmazeutischen und kommerziellem Interesse ist, da sie die prinzipielle Nahtstelle zwischen extrazellulärem Umfeld und intrazellulärem Geschehen aller lebenden Zellen darstellt. Mit Hilfe der Ligandenmoleküle kann von außen auf natürlichem Wege in fast alle wichtigen intrazellulären Prozesse eingegriffen werden bzw. bei Fehlentwicklungen gegengesteuert werden. Die Wirkung vieler Pharmazeutika basiert auf ihrer Funktion, Agonisten oder Antagonisten in der Natur vorkommender Liganden zu sein. Es ist verständlich, daß großes Interesse daran besteht, solche Rezeptor-Liganden-Interaktionen auf molekularer Ebene zu detektieren und studieren zu können.

Es existieren bereits eine Reihe von Methoden zur Detektion der Membranrezeptor-Liganden-Interaktion. Eine der Methoden nutzt die Tatsache aus, daß viele Rezeptoren aufgrund ihrer evolutionären Verwandtschaft einen sehr ähnlichen strukturellen Aufbau haben und daß funktionelle Proteinteile, sogenannte Domänen, leicht gegeneinander austauschbar sind bzw. in neuer Zusammensetzung aneinander fusioniert werden können. So können die verschiedensten extrazellulären Ligandenbindungsdomänen mit spezifischen zytosolischen Reporterdomänen verknüpft werden, um in Zellen gewisse Vereinheitlichungen im Detektionssystem zu schaffen (siehe z.B. US-Patent 4859609). Der Nachteil dieses Verfahrens beruht auf der Notwendigkeit zur Konstruktion von funktionsfähigen, rekombinanten Rezeptorfusionsproteinen, die in Zellen selektiv auf die zu testende Rezeptor-Liganden-Interaktion reagieren.

Ein anderer Ansatz zum Studium von Membranrezeptor-Liganden-Interaktionen geht von unveränderten Wildtyprezeptoren aus, macht sich aber gegen die spezifischen Konfigurationszustände der zytosolischen Rezeptordomäne gerichtete Antikörper zunutze um die Liganden-Interaktion zu messen.

Ein weiterer Ansatz detektiert die Rezeptor-Liganden-Interaktion *ex vivo,* also außerhalb der lebenden Zelle, indem entweder extrazelluläre Rezeptordomänen oder das Ligandenmolekül auf eine Matrix aufgebracht werden, die dann mit einer Lösung, die den Liganden bzw. den Rezeptor enthält, umspült werden. Hier ist der Aufwand zur Gewinnung und Aufbringung jedes einzelnen Rezeptors oder Liganden auf die entsprechende Matrixoberfläche enorm. Ein weiteres Problem ist hierbei auch die Übertragung der gewonnenen Resultate auf die Verhältnisse in der Membran der lebenden Zellen, da die zellulären Bedingungen von den *ex vivo-*Bedingungen erheblich abweichen können. Das gravierendste Problem ist dabei aber die fehlende Zugriffsmöglichkeit auf die genetische Information der in Screens oder High-Throughput-Assays detektierten, neuen Rezeptorvarianten.

Die der Anmeldung zugrundeliegende Aufgabe besteht darin, alternative Assays, die dazu geeignet sind, spezifische Interaktionen zwischen einem Ligand und einem insbesondere membranständigen Rezeptor in vivo zu detektieren, bereitzustellen, die u.a. die Vorteile bieten, Liganden-Rezeptor-Wechselwirkungen schneller als im Stand der Technik möglich detektieren zu können und auch mittels einfacher handzuhabender Zellen, wie prokaryotischen Zellen oder Hefezellen, oder auch mittels zellwandlosen "ghosts"-Formen von insbesondere Hefen ausgeführt werden zu können. Darüber hinaus besteht aufgrund der Assaygestaltung stets eine unmittelbare Zugriffsmöglichkeit auf die einer Rezeptorvariante zugrundeliegende genetische Information.

Weitere Aufgaben der Anmeldung bestehen darin, transformierte Hefezellen, Kits, heterologe Membranrezeptoren, insbesondere Fusionsproteine, Nukleinsäuren und Vektoren bereitzustellen, die sämtlich für die Assayverfahren oder in Zusammenhang mit diesen eingesetzt werden können.

Die vorliegende Publikation beruht auf den folgenden Erkenntnissen:
1. Bei jedem Membranrezeptor löst die Bindung des Liganden an die extrazelluläre Domäne eine Konfigurationszustandsänderung und/oder eine kovalente Modifikation im zytosolischen Teil aus.
2. Für jeden Membranrezeptor gibt es zytosolische, membrannahe bzw. membranständige Adaptorproteine, die die Ligandenspezifischen Konfigurationsänderungen erkennen können oder die infolgedessen aktiviert werden.
3. Zur Aktivierung verschiedener ras- und ras-ähnlicher Signaltransduktionswege ist die Membranlokalisierung bestimmter Komponenten der Signalwege notwendig (Schlessinger, TIBS, 18: 273-275, 1993).
4.a) Wird nun ein Protein aus der Ras-Familie oder ein Guaninnukleotid-Austauschfaktor ("guanine nucleotide exchange factor"; GEF) als Effektorprotein oder -polypeptid dergestalt vorgesehen, daß es bzw. er infolge einer Ligandenbindung an die extrazelluläre Domäne eines Membranrezeptors an eine Komponente der Membran, beispielsweise an den intrazellulären Abschnitt (= Mediatorabschnitt) des Membranrezeptors oder an membrannah oder membranständig lokalisierte Adaptorproteine, binden kann, wozu es bzw. er gegebenenfalls als Fusionsprotein vorgesehen wird, dessen zusätzlicher Proteinanteil, der auch als Adaptorabschnitt bezeichnet werden kann, die Bindung an eine derartige Komponente der Membran ermöglicht, wird bei einer solchen Bindung eine Membranlokalisierung des Ras-Proteins bzw. des GEF erreicht, die für eine Aktivierung eines ras- oder rasähnlichen Signaltransduktionswegs erforderlich ist.
4.b) Alternativ kann ein Protein aus der Ras-Familie oder ein Guaninnukleotid-Austauschfaktor ("guanine nucleotide exchange factor"; GEF) als Effektorprotein oder -polypeptid dergestalt vorgesehen werden, daß es bzw. er nur infolge einer fehlenden Ligandenbindung an die extrazelluläre Domäne eines Membranrezeptors an eine Komponente der Membran, beispielsweise an den intrazellulären Abschnitt (= Mediatorabschnitt) des Membranrezeptors oder an membrannah oder membranständig lokalisierte Adaptorproteine, binden kann, wozu es bzw. er gegebenenfalls als Fusionsprotein vorgesehen wird, dessen zusätzlicher Proteinanteil, der auch als Adaptorabschnitt bezeichnet werden kann, die Bindung an eine derartige Komponente der Membran ermöglicht. Aufgrund einer solchen Bindung, die in dieser Variante nur bei fehlender Ligandenbindung an die extrazelluläre Domäne des Membranrezeptors ermöglicht wird, wird dann eine Membranlokalisierung des Ras-Proteins bzw. des GEF erreicht, die für eine Aktivierung eines ras- oder ras-ähnlichen Signaltransduktionswegs erforderlich ist.
5. Eine Detektion eines Ergebnisses einer derartigen Membranrezeptor-Liganden-Interaktion kann in Hefezellen erfolgen. Im Stand der Technik sind Zellen bekannt, in denen ein ras- oder ras-ähnlicher Signaltransduktionsweg auf Ebene des dafür spezifischen Ras-Proteins oder eines für das Ras-Protein spezifischen Guaninnukleotid-Austauschfaktors zumindest unter bestimmten Bedingungen inaktiviert werden kann. Vermag in einer derartigen Zelle das unter 4. erläuterte Effektorprotein oder -polypeptid eben diesen inaktivierten Signalweg zu aktivieren, so erhält man eine Zelle, bei der der zumindest unter bestimmten Bedingungen inaktive zelleigene ras- oder ras-ähnliche Signaltransduktionsweg durch dieses Ras-Protein aktiviert werden kann - allerdings im Falle der Variante 4.a) nur in Gegenwart eines Liganden für den extrazellulären Ligandenbindungsabschnitt des Membranrezeptors, im Falle der Variante 4.b) nur in Abwesenheit eines Liganden für den extrazellulären Ligandenbindungsabschnitt des Membranrezeptors.

Man erhält auf diese Weise eine Zelle, in der der oder ein bestimmter ras-Signaltransduktionsweg nur in Abhängigkeit von einer Ligandenbindung an den Ligandenbindungsabschnitt eines membranständigen Rezeptors (Variante 4.a)) bzw. nur bei fehlender Ligandenbindung an den Ligandenbindungsabschnitt eines membranständigen Rezeptors (Variante 4.b)) aktiviert werden kann. Diese Zelle ermöglicht die Etablierung eines *in vivo-*Assayverfahrens, welches anhand des Nachweises einer gegebenenfalls erfolgten Aktivierung des speziellen ras-Signaltransduktionsweges, ggf. indirekt über an oder in der Zelle nachweisbare spezifische Auswirkungen, wie Zellwachstum, die Detektion von Interaktionen zwischen einem solchen Rezeptor und einem dafür spezifischen Liganden ermöglicht.

Somit ist ein erster Gegenstand dieser Publikation eine transformierte Hefezelle, umfassend einen heterologen Membranrezeptor, der einen Ligandenbindungsabschnitt, ein Membranlokalisierungssignal und einen Mediatorabschnitt umfasst, wobei nur bei Bindung oder, alternativ, nur bei fehlender Bindung eines Liganden an den Ligandenbindungsabschnitt eine Strukturänderung mit Auswirkungen auf den Mediatorabschnitt bewirkt wird, so dass in Folge ein Effektorprotein oder -polypeptid, das in der Lage ist, einen Ras- oder Ras-ähnlichen Signalweg in der Zelle zu aktivieren, an eine Komponente der Membran, gegebenenfalls über weitere Proteine oder Polypeptide (Adaptoren), bindet, dadurch gekennzeichnet, dass das Effektorprotein oder -polypeptid, das in der Lage ist, einen Ras- oder Ras-ähnlichen Signalweg in der Zelle zu aktivieren, in Form eines heterologen Fusionsproteins eines Effektorabschnitts mit einem Adaptorprotein oder -polypeptid, das die Bindung an die Komponente der Membran, gegebenenfalls über weitere Proteine oder Polypeptide (Adaptoren), ermöglicht, vorliegt, wobei der Effektorabschnitt des Fusionsproteins die Sequenz eines konstitutiv aktiven Ras-Proteins umfasst.

Der Membranrezeptor kann ein natürlich vorkommender Rezeptor sein, wie beispielsweise ein Transmembranrezeptor, ein Enzymgekoppelter Rezeptor, ein G-Protein-gekoppelter Rezeptor, ein 7-Transmembranrezeptor oder ein Geruchsrezeptor ("Odour Receptor" oder "Olfactorial Receptor"). Der Membranrezeptor kann in der Zelle natürlicherweise vorkommen oder kann aus einem andersartigen Zellsystem oder auch einem Virus stammen und in die Zelle durch Transformation oder Transfektion eingeschleust worden sein.

Alternativ kann der Membranrezeptor auch ein in der Natur nicht vorkommender, synthetischer Rezeptor sein. Ein solcher umfaßt bevorzugt Abschnitte oder Domänen, die ihrerseits jeweils in der Natur vorkommen, wie z.B. die Aminosäuresequenz des Ligandenbindungsabschnitts eines Transmembranrezeptors, eines Enzym-gekoppelten Rezeptors, eines G-Protein-gekoppelten Rezeptors, eines 7-Transmembranzezeptors oder eines Geruchsrezeptors ("Odour Receptor" oder "Olfactorial Receptor"), aber auch die eines in der Natur vorkommenden nukleären Rezeptors, wie eines Steroid-Rezeptors, Orphan-Rezeptors, Vitamin-Rezeptors, beispielsweise Vitamin D-Rezeptors, Thyroxin-Rezeptors oder Retinsäurerezeptors, oder jene eines in der Natur vorkommenden viralen Rezeptors, insbesondere Membranrezeptors, oder davon durch Aminosäureanfügung, -austausch, -modifizierung, -insertion oder -deletion abgeleitete Sequenzen, kann aber auch beispielsweise mittels "molecular modelling" erzeugte Abschnitte umfassen. Beispielsweise kann ein solcher Membranrezeptor einen Ligandenbindungsabschnitt und ein Membranlokalisierungssignal, die beide von einem bestimmten Protein aus einem bestimmten Organismus abstammen, und einen Mediatorabschnitt, der von einem unterschiedlichen Protein und ggf. auch Organismus abstammt, umfassen. Insbesondere stammen der Ligandenbindungsabschnitt und der Mediatorabschnitt von unterschiedlichen Proteinen und ggf. auch Organismen ab; auch das Membranlokalisierungssignal kann dementsprechend von einem unterschiedlichen Protein und/oder Organismus abstammen oder auch synthetisch erzeugt sein. Außerdem kann der Ligandenbindungsabschnitt einen in der Natur nicht vorkommenden, beispielsweise durch "molecular modelling" erzeugten synthetischen Ligandenbindungsabschnitt mit insbesondere zunächst nur vermuteter Ligandenbindungsfunktion umfassen.

In bevorzugten Ausführungsformen dieser Anmeldung umfaßt das Membranlokalisierungssignal des Membranrezeptors die Aminosäuresequenz einer Transmembrandomäne, wie sie insbesondere bei Transmembranrezeptoren gefunden wird, eines Farnesylierungssignals, Myristylierungssignals oder Prenylierungssignals oder ist davon, z.B. durch Aminosäureaustausch, -modifizierung, -insertion oder -deletion, abgeleitet.

Im Bereich der Membranlokalisierungsdomäne oder als ein zusätzlicher, insbesondere N-terminal angeordneter Sequenzabschnitt kann ferner eine Signalsequenz vorgesehen werden, die zwar nicht der Membranverankerung des Membranrezeptors an sich dient, jedoch bewirkt, daß der Membranrezeptor nach seiner Expression mit höherer Effizienz an die Zellmembran transportiert wird. Die daraus resultierende höhere Konzentration von Membranrezeptor in unmittelbarer Nähe der Membran führt in der Folge zu einer höheren Einbaurate des Membranrezeptors in die Zellmembran aufgrund der Membranlokalisierungsdomäne. Solche Signalsequenzen werden bevorzugt speziell auf den Zelltyp, in dem der Membranrezeptor exprimiert werden soll, abgestimmt verwendet, da beispielsweise in Hefe wirksame Signalsequenzen in Säugetierzellen nur mit geringerer Effizienz wirksam sind und umgekehrt. Beispiele für solche Signalsequenzen sind Signalsequenzen von hefeeigenen GPCRs oder von hefeeigener Invertase (SUC2) zur bevorzugten Verwendung in Membranrezeptoren, die in Hefezellen exprimiert werden sollen.

Dementsprechend kann es je nach gewünschtem Zelltyp auch sinnvoll sein, eine rezeptoreigene Signalsequenz durch eine in dem gewünschten Zelltyp effektivere Signalsequenz zu ersetzen, beispielsweise bei Arbeit insbesondere in Hefezellen durch Austausch einer rezeptoreigenen Signalsequenz am N-Terminus eines Membranrezeptorproteins gegen die Signalsequenz eines der hefeeigenen G-Protein gekoppelten Rezeptoren (GPCRs) oder einer ähnlichen Signalsequenz, die dafür sorgt, daß der Rezeptor in aktiver Form in der Zellmembran verankert wird. Bei Rezeptor-Tyrosinkinasen (RTKs) oder Rezeptor-Phosphatasen kann insbesondere für eine Arbeit in Hefen ein Austausch der rezeptoreigenen Signalsequenz, sofern vorhanden, gegen die signalsequenz der hefeeigenen Invertase (SUC2) oder einer anderen Signalsequenz, die die Verankerung des Rezeptors in aktiver Form in der Zellmembran optimiert, beispielhaft genannt werden.

Wie bereits angesprochen, ist das Effektorprotein oder -polypeptid, das in der Lage ist, einen Ras- oder Ras-ähnlichen Signalweg zu aktivieren, ein Guanine Nucleotide Exchange Factor (GEF), z.B. das CDC25-Protein aus *Saccharomyces cerevisiae* oder ein SOS-Protein aus einem Säugetier oder ein SOS-ähnliches Protein aus einem beliebigen Organismus, oder ein aktives Protein aus der Ras-Familie, ist von derartigen Faktoren oder Proteinen abgeleitet oder weist eine derartige Funktion in einem Abschnitt davon auf.

In einer besonderen Ausführungsform, die nachfolgend detaillierter erläutert wird, liegt das Effektorprotein oder -polypeptid, das in der Lage ist, einen Ras- oder Ras-ähnlichen Signalweg zu aktivieren, in Form eines Fusionsproteins eines Effektorabschnitts mit einem Adaptorprotein oder -polypeptid vor, das die Bindung an eine Komponente der Membran im Falle einer Ligandenbindung an den Ligandenbindungsabschnitt des Membranrezeptors oder, alternativ, nur im Falle einer fehlenden Ligandenbindung an den Ligandenbindungsabschnitt des Membranrezeptors, gegebenenfalls über weitere Proteine oder Polypeptide, ermöglicht.

In einer weiteren speziellen Ausführungsform bedarf das Fusionsprotein einer enzymatischen Modifizierung, bevor es an die Komponente der Membran, gegebenenfalls über weitere Proteine oder Polypeptide, gebunden werden kann. In diesem Falle wird in dem erfindungsgemäßen Zellsystem eine für die enzymatische Modifizierung erforderliche enzymatische Aktivität nur aufgrund einer Ligandenbindung an den Ligandenbindungsabschnitt oder, alternativ, nur aufgrund fehlender Ligandenbindung an den Ligandenbindungsabschnitt aktiviert.

In allen Fällen erfolgt in den Hefezellen infolge der Ligandenbindung an den Ligandenbindungsabschnitt des Membranrezeptors oder, alternativ, infolge der fehlenden Ligandenbindung an den Ligandenbindungsabschnitt des Membranrezeptors eine Translokation des Effektorproteins oder -polypeptids an die Zellmembran, wodurch die Aktivierung des Ras- oder Ras-ähnlichen Signalwegs ermöglicht wird. Die Translokation wird aufgrund der Bindung des Effektorproteins oder -polypeptids an eine Komponente der Membran, und in einer bevorzugten der möglichen Ausführungsformen, an den Mediatorabschnitt des Membranrezeptors, gegebenenfalls über weitere Proteine oder Polypeptide, bewirkt. Die Bindung des Effektorproteins oder -polypeptids an die Komponente der Membran ist nur bei Bindung eines Liganden an den Ligandenbindungsabschnitt dieses Membranrezeptors oder, alternativ, nur bei fehlender Bindung eines Liganden an den Ligandenbindungsabschnitt dieses Membranrezeptors möglich, da dadurch eine Strukturänderung, insbesondere Konformationsänderung mit Auswirkungen auf den Mediatorabschnitt bewirkt wird, so daß in der Folge das Effektorprotein oder -polypeptid, gegebenenfalls über weitere Proteine oder Polypeptide, an die Komponente der Membran, und in einer bevorzugten Ausführungsform an eben diesen Mediatorabschnitt bindet.

Diese Bindung des Effektorproteins oder -polypeptids an eine Komponente der Membran, gegebenenfalls über weitere Proteine oder Polypeptide, kann abhängig von dem jeweiligen Membranrezeptor und insbesondere von dem jeweiligen Mediatorabschnitt des Membranrezeptors auf unterschiedliche Weise erfolgen:

1) In einer ersten Variante entspricht der Mediatorabschnitt dem zytoplasmatischen Teil eines G-Protein gekoppelten Rezeptors. Alternativ umfaßt er für die nachfolgend erläuterten Eigenschaften dieses Teils G-Protein gekoppelter Rezeptoren wesentliche Abschnitte solcher Rezeptoren oder auch von den erwähnten Teilen oder Abschnitten beispielsweise durch Aminosäureanfügung, -substitution, -deletion, -insertion und/oder - modifizierung abgeleitete Sequenzen, die jedoch noch die zu erläuternden Eigenschaften der Ausgangssequenzen aufweisen.

Der den genannten Mediatorabschnitt umfassende Membranrezeptor kann beispielsweise ein natürlich vorkommender 7-Transmembranrezeptor sein. Diese Rezeptoren umfassen z. B. die Toxinrezeptoren, die Geruchsrezeptoren, Neurotransmitterrezeptoren und viele andere mehr (Dohlman et al.; 1991, Leurs et al., 1998).

Darüber hinaus kann der Membranrezeptor auch ein synthetischer Rezeptor sein, z.B. mit dem zytoplasmatischen Teil eines bestimmten G-Protein-gekoppelten Rezeptors als Mediatorabschnitt und der Ligandenbindungsdomäne eines anderen G-Protein-gekoppelten Rezeptors oder auch eines andersartigen Rezeptors. In einer speziellen Ausführungsform der Anmeldung bewirkt bei synthetischen Rezeptoren dieser Art der Ligandenbindungsabschnitt bei Bindung von Ligand die natürlicherweise bei G-Protein-gekoppelten Rezeptoren in diesem Falle auftretende Strukturänderung, insbesondere Konformationsänderung mit Auswirkung auf den Mediatorabschnitt, den zytoplasmatischen Teil des Rezeptors. Alternativ sind jedoch bei einer Bindung von Ligand auch andersartige Strukturänderungen mit erfindungsgemäß nachweisbaren Auswirkungen auf den Mediatorabschnitt des Rezeptors denkbar.

Wie bereits erläutert, wird bei den G-Protein-gekoppelten Rezeptoren die zytoplasmatische Rezeptorkonfiguration abhängig von einer Ligandenbindung durch einen G-Proteinkomplex abgegriffen, der aus drei Untereinheiten besteht und der das Signal durch Dissoziation in einen aktivierten membrangebunden βγ-Teil und in eine aktivierte, ebenfalls membrangebundene Gα-Proteinuntereinheit weiterleitet. Die Aktivierung der Gα-Proteinuntereinheit geht einher mit einem Austausch des für den inaktiven Zustand charakteristischen Kofaktors GDP durch den für den aktivierten Zustand charakteristischen Kofaktor GTP, der nach GTP zu GDP-Hydrolyse die Gα-Proteinuntereinheit wieder inaktiviert.

Die Weiterleitung des Signals aus der G-Protein-Aktivierung, d.h. -Dissoziierung, bzw. -Inaktivierung, d.h. -Reassoziierung, zu den G-Protein-Mediatoren, diversen Signaltransduktionswegen, die ihrerseits auf den Stoffwechsel einwirken, vermitteln sogenannte Adaptorproteine. Adaptorproteine für die G-Protein-Mediatoren sind z. B. Adenylatzyklasen, die Phospholipase C und andere G-Protein-gekoppelte Rezeptorkinasen, sowie bestimmte Ionenkanäle. Einige von diesen binden insbesondere an den nach wie vor mit dem Mediatorabschnitt assoziierten und aufgrunddessen membranständigen aktivierten βγ-Teil des G-Proteins, andere wiederum an die aktivierte, abdissoziierte, jedoch ebenfalls nach wie vor membrangebundene Gα-Proteinuntereinheit.

Eine Effektorprotein- bzw. -polypeptid-Translokation an die Membran kann nun
- über Vermittlung des membranständigen aktivierten βγ-Teils des G-Proteins (Variante (a)),
- über Vermittlung der von dem βγ-Teil des G-Proteins abdissoziierten, jedoch nach wie vor membrangebundenen aktivierten Gα-Proteinuntereinheit (Variante (b)) oder
- über den Mediatorabschnitt des Membranrezeptors in seiner speziellen Struktur, wie sie nach Bindung eines Liganden an den Ligandenbindungsabschnitt des Membranrezeptors oder, alternativ, bei fehlender Bindung eines Liganden an den Ligandenbindungsabschnitt des Membranrezeptors vorliegt, (Variante (c)) erfolgen.

### Varianten (a) und (b):

Eine Effektorprotein- bzw. -polypeptid-Translokation an die Membran über Vermittlung des membranständigen aktivierten βγ-Teils des G-Proteins (Variante (a)) bzw. über Vermittlung der von dem βγ-Teil des G-Proteins abdissoziierten, jedoch nach wie vor membrangebundenen aktivierten Gα-Proteinuntereinheit (Variante (b)) kann
-- durch "Bindung" an den membrangebundenen aktivierten βγ-Teil (Variante (a)) bzw. an die abdissoziierte membrangebundene aktivierte Gα-Proteinuntereinheit des G-Proteins (Variante (b)) direkt (Alternative (i)),
-- über eines der genannten Adaptorproteine, die mit dem membranständigen aktivierten βγ-Teil des G-Proteins (Variante (a)) oder mit der abdissoziierten membrangebundenen aktivierten Gα-Proteinuntereinheit des G-Proteins (Variante (b)) assoziiert sind, (Alternative (ii)) oder
-- über eine "Bindung" an ein membranständiges Molekül, das durch Vermittlung des aktivierten βγ-Teils (Variante (a)) oder der aktivierten Gα-Proteinuntereinheit (Variante (b)) des G-Proteins in einem speziellen modifizierten Zustand vorliegt, (Alternative (iii)) erfolgen.

Für Alternative (i) wird das Effektorprotein oder -polypeptid in der Zelle z.B. in Form eines Fusionsproteins eines Effektorabschnitts, der die Aktivierung des Ras- oder Ras-ähnlichen Signalwegs bewirken kann, mit einem mit dem βγ-Teil des G-Proteins nach Abdissoziierung der α-Untereinheit (Variante (a)) oder mit der aktivierten Gα-Proteinuntereinheit nach Abdissoziierung von dem βγ-Teil des G-Proteins (Variante (b)) wechselwirkenden Adaptorprotein, insbesondere einer Kinase oder einer Phospholipase C, z.B. einer GRK2- oder GRK3-Kinase (GRK = "G-protein coupled receptor kinase") oder einer Phosphatidylcholin-Phospholipase C, vorgesehen.

Alternativ kann für Alternative (i) das Effektorprotein oder -polypeptid auch in Form eines Fusionsproteins des Effektorabschnitts mit einem Antikörper, der spezifisch den βγ-Teil des G-Proteins nur nach Abdissoziierung der α-Untereinheit (Variante (a)) bzw. spezifisch die aktivierte Gα-Proteinuntereinheit nach Abdissoziierung von dem βγ-Teil des G-Proteins (Variante (b)) erkennt, bereitgestellt werden.

Für Alternative (ii) kann das Effektorprotein oder -polypeptid u.a. als Fusionsprotein bereitgestellt werden, dessen Adaptorabschnitt an ein Adaptorprotein mit in der Regel enzymatischer Aktivität, wie sie vorstehend speziell für eine Wechselwirkung mit G-Proteinen beispielhaft angeführt wurden, wobei das Adaptorprotein spezifisch mit dem βγ-Teil des G-Proteins nur nach Abdissoziierung der α-Untereinheit (Variante (a)) bzw. mit der aktivierten Gα-Proteinuntereinheit nur nach Abdissoziierung von dem βγ-Teil des G-Proteins (Variante (b)) assoziieren oder wechselwirken kann, bindet. Den Adaptorabschnitt des Fusionsproteins kann dabei insbesondere ein Antikörper oder Bindungsproteinbilden, der das Adaptorprotein spezifisch erkennt und bindet.

In einem weiteren Falle weist auch das Adaptorprotein Antikörper- oder Bindungsproteineigenschaft auf, wobei das Adaptorprotein entweder spezifisch den βγ-Teil des G-Proteins nur nach Abdissoziierung der α-Untereinheit (Variante (a)) oder die aktivierte Gα-Protein-untereinheit nur nach Abdissoziierung von dem βγ-Teil des G-Proteins (Variante (b)) erkennt. Bei dieser Konstellation weist der Adaptorabschnitt des Fusionsproteins in der Regel insbesondere die Funktion eines sogenannten "sekundären Antikörpers" oder anti-Antikörpers, welcher den mit dem Mediatorabschnitt assoziierten, sogenannten "primären" Antikörper erkennt, auf.

Für Alternative (iii) wird die Tatsache ausgenutzt, daß ein G-Protein in aktiviertem Zustand eine Phosphatidylinositol-3-kinase (PI3K) aktivieren kann, die ihrerseits infolge der Aktivierung sogenannte "second messenger"-Verbindungen durch Phosphorylierung der D-3-Position des Inositolrings von Phosphoinositiden (z.B. PtdIns(3,4)P₂ (AKT) oder PtdIns(3,4,5)P₃ (BTK)) erzeugt. Diese Phosphoinositide sind membranständig und binden in ihrem phosphorylierten Zustand sogenannte "Src homology (2)" (SH2)- und "pleckstrin homology" (PH)-Domänen. Für Alternative (iii) stellt man dementsprechend das Effektorprotein oder -polypeptid in einer besonderen Ausführungsform in Form eines Fusionsproteins des Effektorabschnitts, der die Aktivierung des Ras-oder Ras-ähnlichen Signalwegs bewirken kann, mit einer "Src homology (2)" (SH2)-Domäne oder mit einer "Pleckstrin homology" (PH)-Domäne bereit. Die Translokation des Effektorproteins oder -polypeptids in Form des Effektorabschnitts erfolgt dementsprechend nur, wenn eine Phosphorylierung zu den speziellerläuterten Phosphoinositiden infolge der G-Protein-Aktivierung erfolgt, infolgedessen die (SH2)- oder (PH)-Domänen des Fusionsproteins mit diesen Phosphoinositiden assoziieren.

### Variante c:

Wie erläutert, kann eine Effektorprotein- bzw. -polypeptid-Translokation an die Membran alternativ auch über den Mediatorabschnitt des Membranrezeptors in seiner speziellen Struktur, wie sie nach Bindung eines Liganden an den Ligandenbindungsabschnitt des Membranrezeptors oder, alternativ, bei fehlender Bindung eines Liganden an den Ligandenbindungsabschnitt des Membranrezeptors vorliegt, erfolgen, und zwar
-- durch direkte "Bindung" an den Mediatorabschnitt in seiner speziellen Struktur, wie sie nach Bindung bzw. nur bei fehlender Bindung eines Liganden an den Ligandenbindungsabschnitt des Membranrezeptors vorliegt, (Alternative (i)) oder
-- über ein oder mehrere Adaptorprotein(e), die mit dem Mediatorabschnitt in seiner speziellen Struktur, wie sie nach Bindung bzw. nur bei fehlender Bindung eines Liganden an den Ligandenbindungsabschnitt des Membranrezeptors vorliegt, assoziiert sind, (Alternative (ii)).

Für Alternative (i) wird das Effektorprotein oder -polypeptid in der Zelle z.B. in Form eines Fusionsproteins eines Effektorabschnitts, der die Aktivierung des Ras- oder Ras-ähnlichen Signalwegs bewirken kann, mit einem mit dem Mediatorabschnitt des Membranrezeptors nur in seiner speziellen Struktur, wie sie nach Bindung eines Liganden an den Ligandenbindungsabschnitt des Membranrezeptors oder, alternativ, nur bei fehlender Bindung eines Liganden an den Ligandenbindungsabschnitt des Membranrezeptors vorliegt, wechselwirkenden Adaptorprotein, insbesondere einem Antikörper oder Bindungsprotein entsprechender Spezifität, vorgesehen.

Für Alternative (ii) dieser Variante kann das Effektorprotein oder -polypeptid insbesondere als Fusionsprotein bereitgestellt werden, dessen Adaptorabschnitt an ein Adaptorprotein, welches spezifisch mit dem Mediatorabschnitt des Membranrezeptors nur in seiner speziellen Struktur, wie sie nach Bindung eines Liganden an den Ligandenbindungsabschnitt des Membranrezeptors oder, alternativ, nur bei fehlender Bindung eines Liganden an den Ligandenbindungsabschnitt des Membranrezeptors vorliegt, assoziieren oder wechselwirken kann, bindet. Den Adaptorabschnitt des Fusionsproteins kann dabei insbesondere ein Antikörper oder Bindungsprotein bilden, der bzw. das das Adaptorprotein spezifisch erkennt und bindet. In dem speziellen Falle, wo auch das Adaptorprotein Antikörpereigenschaft aufweist, wobei das Adaptorprotein den Mediatorabschnitt des Membranrezeptors spezifisch nur in seiner speziellen Struktur, wie sie nach Bindung bzw. nur bei fehlender Bindung eines Liganden an den Ligandenbindungsabschnitt des Membranrezeptors vorliegt, erkennt, weist der Adaptorabschnitt des Fusionsproteins bei dieser Konstellation die Funktion eines sogenannten "sekundären Antikörpers" oder anti-Antikörpers, welcher den mit dem Mediatorabschnitt assoziierten, sogenannten "primären" Antikörper erkennt, auf.

2) Bei einer zweiten Variante werden für den Membranrezeptor Rezeptoren mit Mediatorabschnitten ausgewählt, welche bei Bindung von Ligand an den Ligandenbindungsabschnitt des Membranrezeptors oder, alternativ, nur bei fehlender Bindung von Ligand an den Ligandenbindungsabschnitt des Membranrezeptors Adaptorproteine binden können, welche die Bindung des Effektorproteins oder -polypeptids vermitteln.

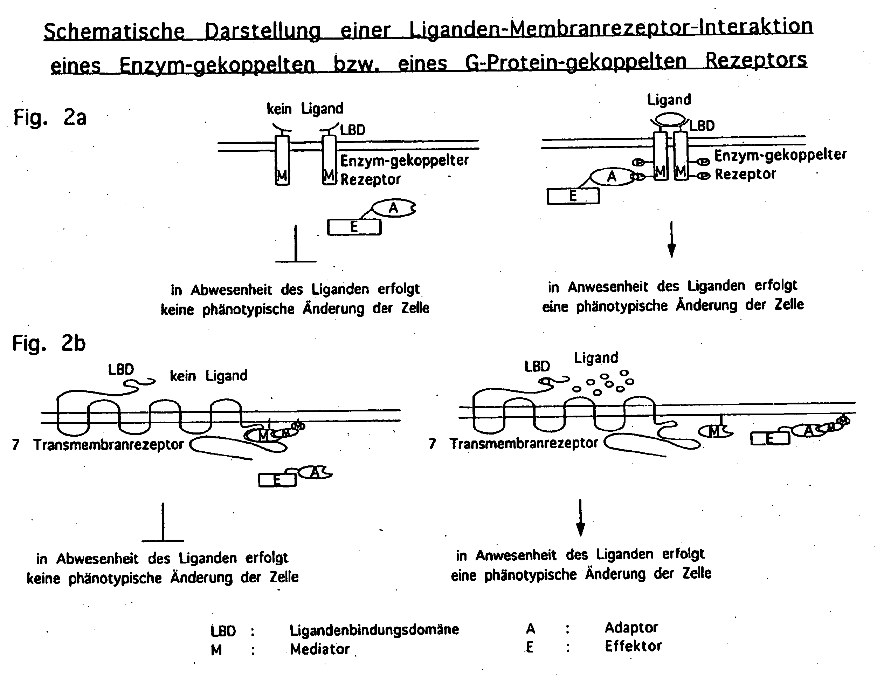

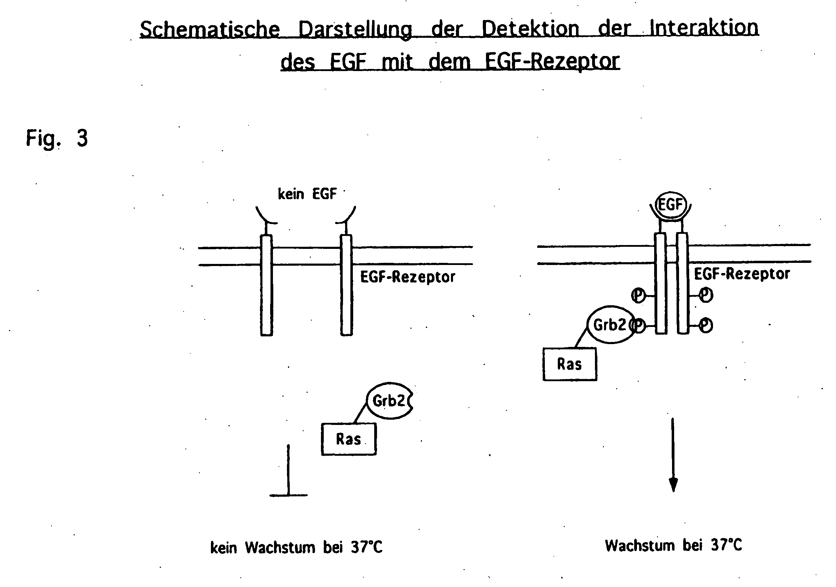

Die hierfür geeigneten Membranrezeptoren, die ggf. auch nur den Mediatorabschnitt bereitstellen, sind insbesondere Enzym-gekoppelte Rezeptoren mit eigener enzymatischer Aktivität und insbesondere mit Kinaseaktivität, beispielsweise Tyrosinkinaseaktivität, Serin/Threoninkinaseaktivität oder Phosphataseaktivität, welche im zytoplasmatischen Abschnitt des Rezeptors lokalisiert ist. Die enzymatische Aktivität des Mediatorabschnitts wird dabei stets erst bei Bindung oder, alternativ, nur bei fehlender Bindung von Ligand an die Ligandenbindungsdomäne des Membranrezeptors infolge der durch die (fehlende) Ligandenbindung bewirkten Struktur- und insbesondere Konformationsänderung aktiv. Die enzymatische Aktivität ist dabei bei bestimmten Ausführungsformen für die bei Ligandenbindung oder, alternativ, bei fehlender Ligandenbindung mögliche Bindung des oder der Adaptorprotein(e) an den Mediatorabschnitt mit erforderlich, z.B. über eine z.T. mehrfache Phosphorylierung des Mediatorabschnitts (vgl. Fig. 2a und 3) und/oder auch über eine Phosphorylierung von Adaptorprotein(en). Ein Beispiel für einen entsprechenden natürlich vorkommenden Enzym-gekoppelten Rezeptor ist der epidermaler Wachstumsfaktor-Rezeptor (EGFR), der eine Tyrosinkinaseaktivität aufweist (vgl. Fig. 3).

Die hier einsetzbaren Membranrezeptoren können wie bei 1) natürlich vorkommende Rezeptoren, von solchen Rezeptoren abgeleitete Rezeptoren oder auch synthetische Rezeptoren sein, die insbesondere Abschnitte verschiedenen Ursprungs mit den vorstehend erwähnten Funktionen, wie Membranlokalisierung, Ligandenbindung und Mediatorfunktion, umfassen. Diese Abschnitte können ihrerseits von natürlich vorkommenden Rezeptoren oder andersartigen Proteinen abstammen oder von solchen nach den bereits erläuterten Maßgaben abgeleitet sein. In einer speziellen Ausführungsform der Erfindung bewirkt auch bei den synthetischen Rezeptoren dieser Art der Ligandenbindungsabschnitt bei Bindung von Ligand die natürlicherweise bei Enzym-gekoppelten Rezeptoren in diesem Falle auftretenden Struktur- und insbesondere Konformationsänderungen mit Auswirkung auf den Mediatorabschnitt und ins-besondere auf die in diesem Abschnitt lokalisierte enzymatische Aktivität. Alternativ sind jedoch auch hier bei einer Bindung von Ligand auch andersartige Strukturänderungen mit nachweisbaren Auswirkungen auf den Mediatorabschnitt des Rezeptors und insbesondere auf die in diesem Abschnitt lokalisierte enzymatische Aktivität denkbar.

Eine Effektorprotein- bzw. -polypeptid-Translokation an die Membran kann nun durch Bindung des Effektorproteins oder -polypeptids an das aufgrund einer Bindung an den Mediatorabschnitt membranständige Adaptorprotein erfolgen (Alternative (a)) oder durch Verwendung eines Fusionsproteins, das das Effektorprotein oder -polypeptid in Fusion mit dem Adaptorprotein enthält (Alternative (b)).

Für Alternative (a) können in einer besonderen Ausführungsform Mediatorabschnitte von Membranrezeptoren gewählt werden, die im Falle einer Bindung von Ligand an ihren Ligandenbindungsabschnitt oder, alternativ, bei fehlender Bindung von Ligand an ihren Ligandenbindungsabschnitt Adaptorproteine binden, an die natürlicherweise eine Bindung von Guaninnukleotid-Austauschfaktor (GEF) erfolgt. Ein Beispiel für einen derartigen Membranrezeptor stellen der epidermaler Wachstumsfaktor-Rezeptor (EGFR) und die für dessen zytoplasmatischen oder Mediatorabschnitt spezifischen Adaptorproteine Grb2 und Shc dar. An diese Adaptorproteine Gbr2 und Shc, die an den Mediatorabschnitt des EGFR nur im Falle einer Ligandenbindung an den Ligandenbindungsabschnitt von diesem binden, bindet wiederum ein Guaninnukleotidaustauschfaktor, der in der Lage ist, einen Ras- oder Ras-ähnlichen Signalweg in einer Zelle zu aktivieren.

In einer weiteren Ausführungsform der Alternative (a) kann das Effektorprotein oder -polypeptid jedoch auch in Form eines Fusionsproteins aus einem Effektorabschnitt, der in der Lage ist, einen Ras- oder Ras-ähnlichen Signalweg in einer Zelle zu aktivieren, und einem Abschnitt mit Antikörper- oder Bindungsproteineigenschaft bereitgestellt werden, wobei der Abschnitt mit Antikörper- oder Bindungsproteineigenschaft spezifisch das oder die Adaptorprotein(e) erkennt und bindet, die infolge einer Ligandenbindung oder, alternativ, aufgrund *fehlender* Ligandenbindung an den Ligandenbindungsabschnitt des Membranrezeptors an den Mediatorabschnitt von diesem gebunden werden.

Bei einer speziellen Variante dieser Ausführungsform stellt das Adaptorprotein kein natürlicherweise vorkommendes Adaptorprotein, wie Grb2 oder Shc, oder ein davon abgeleitetes Adaptorprotein dar, sondern weist ebenfalls Antikörpereigenschaft auf, wobei das Antikörper-Adaptorprotein, wie soeben erläutert, den Mediatorabschnitt des Membranrezeptors nur infolge einer Ligandenbindung an den Ligandenbindungsabschnitt oder, alternativ, infolge *fehlender* Ligandenbindung an den Ligandenbindungsabschnitt desselben erkennt und bindet. In diesem Falle weist der Adaptorabschnitt des Fusionsproteins dann die Funktion eines sogenannten "sekundären Antikörpers" oder anti-Antikörpers, welcher den mit dem Mediatorabschnitt assoziierten, sogenannten "primären" Antikörper erkennt, auf.

In Alternative (b) wird das Effektorprotein in Form eines Fusionsproteins, das den Effektorabschnitt in Fusion mit dem Adaptorprotein enthält, vorgesehen. Durch die Bindung des Adaptorproteinabschnitts des Fusionsproteins an den Mediatorabschnitt eines Membranrezeptors infolge der Ligandenbindung oder, alternativ, der *fehlenden* Ligandenbindung an den Ligandenbindungsabschnitt von diesem wird über die kovalente Verknüpfung innerhalb des Fusionsproteins auch der Effektorabschnitt an die Membran geführt und kann dort seine Wirkung zur Aktivierung eines Ras- oder RAS-ähnlichen Signalwegs ausüben.

Ein Beispiel für diese Variante (b) ist ein Fusionsprotein, das als Adaptorabschnitt ein Gbr2- oder Shc-Protein, fusioniert mit einem bevorzugt konstitutiv aktiven ras-Protein, beispielsweise dem humanen konstitutiv aktiven ras-Protein (Ha-ras, L61), oder einem funktionalen GEF als Effektorabschnitt, umfaßt. Ein solches Fusionsprotein wird z.B. wieder in Verbindung mit einem EGFR-Membranrezeptor oder mit einem Rezeptor, der den zytoplasmatischen Teil von diesem umfaßt, eingesetzt, wobei der zytoplasmatische oder Mediatorabschnitt des EGFR mit dem Gbr2- oder Shc-Anteil des Fusionsproteins bei Bindung von Ligand wechselwirkt und darüber den Effektorabschnitt an die Membran führt (vgl. auch Fig. 2a, 3).

Ein weiteres Beispiel für diese Variante (b) ist ein Fusionsprotein, das als Adaptorabschnitt einen Antikörper oder ein Bindungsprotein aufweist, der bzw. das spezifisch den Mediatorabschnitt des Membranrezeptors nur in seiner Struktur/Konformation nach Bindung eines Liganden an den Ligandenbindungsabschnitt oder, alternativ, bei *fehlender* Bindung eines Liganden an den Ligandenbindungsabschnitt erkennt und bindet.

3) Eine dritte Variante ist der zweiten Variante ähnlich, wobei jedoch der als Membranrezeptor eingesetzte bzw. für die Gestaltung des Mediatorabschnitts des Membranrezeptors herangezogene Enzym-gekoppelte Rezeptor keine eigene enzymatische Aktivität aufweist, die bei Ligandenbindung oder, alternativ, *fehlender* Ligandenbindung aktiviert wird, sondern vielmehr in strikter Abhängigkeit von einer eigenen Aktivierung durch Ligandenbindung bzw. *fehlende* Ligandenbindung ein separates rezeptorspezifisches Enzym aktiviert, das sich z.B. auf einer separaten Untereinheit befindet. Als Beispiele für einen derartigen Rezeptortyp können diverse Zytokinrezeptoren genannt werden.

Dieses separate rezeptorspezifische Enzym kann in einer besonderen Ausführungsform auch zu der Zelle heterolog sein. Bevorzugt ist das separate rezeptorspezifische Enzym ebenfalls eine Kinase und insbesondere eine Tyrosinkinase. Wieder führt die Kinaseaktivität bevorzugt zur Phosphorylierung des Mediatorabschnitts, d.h. des zytoplasmatischen Abschnitts des Membranrezeptors und wirkt damit zusätzlich auf die Bindung eines für den Mediatorabschnitt spezifischen Adaptorproteins ein.

Ansonsten gilt gleiches wie für den Membranrezeptor von Variante 2), d.h. dieser kann ein natürlich vorkommender Membranrezeptor, ein von einem solchen Membranrezeptor abgeleiteter Membranrezeptor oder ein synthetischer Membranrezeptor sein, dessen verschiedene Domänen oder Abschnitte aus unterschiedlichen Quellen stammen, die ggf. auch von natürlich vorkommenden Sequenzen abgeleitet sein können.

We bei Variante 2) kann auch hier die Effektorprotein- bzw. -polypeptid-Translokation an die Membran durch Bindung des Effektorproteins oder -polypeptids an ein aufgrund einer Bindung an den Mediatorabschnitt membranständiges Adaptorprotein erfolgen (Alternative (a)) oder durch Verwendung eines Fusionsproteins, das das Effektorprotein oder -polypeptid in Fusion mit dem Adaptorprotein enthält (Alternative (b)).

Auch in diesem Falle können als Beispiele für mögliche Adaptorproteine Grb2 oder Shc aufgeführt werden, die gegebenenfalls auch als Fusionsproteine mit einem Effektorabschnitt, der in der Lage ist, den Ras- oder Ras-ähnlichen Signalweg zu aktivieren, eingesetzt werden können, aber auch Antikörper oder andersartige Bindungsproteine, die spezifisch den Mediatorabschnitt des Membranrezeptors nur in seiner Struktur/Konformation nach Bindung eines Liganden an den Ligandenbindungsabschnitt oder, alternativ, nur bei *fehlender* Bindung eines Liganden an den Ligandenbindungsabschnitt erkennen und binden und in Form von Fusionsproteinen mit einem Effektorabschnitt eingesetzt werden können. Alternativ umfaßt das Antikörper-Adaptorprotein bzw. Bindungsprotein-Adaptorprotein keine Effektorfunktion, sondern wird seinerseits von einem Fusionsprotein mit einem Effektorabschnitt und einem Adaptorabschnitt mit der Funktion insbesondere eines sogenannten "sekundären Antikörpers" oder anti-Antikörpers, welcher den mit dem Mediatorabschnitt assoziierten, sogenannten "primären" Antikörper (Antikörper-Adaptorprotein) erkennt, über diesen Adaptorabschnitt erkannt und gebunden.

4) In einer weiteren, vierten Variante wird als Membranrezeptor ein Fusionsprotein eingesetzt, das innerhalb seiner Sequenz, insbesondere am oder im Bereich des C-Terminus, ein sogenanntes "Tag" oder Epitop aufweist, das für eine Erkennung durch einen dafür spezifischen Antikörper oder ein dafür spezifisches Bindungsprotein nur bei Bindung von Ligand an den Ligandenbindungsabschnitt oder, alternativ, nur bei fehlender Bindung von Ligand an den Ligandenbindungsabschnitt zugänglich wird. Als Beispiele für geeignete "Tags" oder Epitope können ein Myc-Tag, His-Tag, Hämagglutinin-Epitop und ähnliche genannt werden.

Der "Tag" oder das Epitop kann ein Teil des Mediatorabschnitts sein, kann jedoch auch zu diesem benachbart oder von diesem durch einen Aminosäuresequenzabschnitt getrennt sein. Jedoch wird dessen Zugänglichkeit für einen spezifischen Antikörper oder ein spezifisches Bindungsprotein erst aufgrund von Konformationsänderungen, ggf. in Kombination mit enzymatischer Aktivität, infolge einer Bindung von Ligand an den Ligandenbindungsabschnitt des Membranrezeptors oder, alternativ, aufgrund einer Abdissoziierung von Ligand von dem Ligandenbindungsabschnitt des Membranrezeptors, d.h. bei der letzteren Variante bei fehlender Ligandenbindung ermöglicht. Ein "Tag" oder Epitop kann somit an sämtliche erwähnten Rezeptortypen, also einen G-Protein-gekoppelten Rezeptor (GPCR) wie auch an Rezeptor-Tyrosinkinasen oder Rezeptor-Phosphatasen, angefügt sein, vorausgesetzt, daß die vorstehend erläuterten Maßgaben hinsichtlich der Zugänglichkeit des "Tags" oder Epitops für einen Antikörper oder ein spezifisches Bindungsprotein erfüllt sind.

Das Effektorprotein wird in diesem Falle als Fusionsprotein, das den Effektorabschnitt und einen für den "Tag" oder das Epitop spezifischen Antikörper bzw. ein für den "Tag" oder das Epitop spezifisches Bindungsprotein umfaßt, vorgesehen. Wird der "Tag" oder das Epitop infolge einer Ligandenbindung an den Ligandenbindungsabschnitt oder, alternativ, infolge fehlender Ligandenbindung und daraus resultierenden Auswirkungen auf den Mediatorabschnitt für den in dem Fusionsprotein enthaltenen Antikörper bzw. das in dem Fusionsprotein enthaltene Bindungsprotein zugänglich, erfolgt die Effektorprotein- bzw. -polypeptidtranslokation an die Membran im Wege der "Tag"/Epitop-Antikörper/Bindungsprotein-Reaktion.

Bei Vorliegen der erläuterten konformationellen Voraussetzungen ist somit diese vierte Variante am umfassendsten einsetzbar, da sie ansonsten die speziellen Erfordernisse natürlich vorkommender Mediatorabschnitte, wie jenen von G-Proteingekoppelten Rezeptoren, Rezeptor-Tyrosinkinasen oder Rezeptor-Phosphatasen, oder davon abgeleiteter synthetischer Mediatorabschnitte nicht berücksichtigen muß. Zudem bewirkt die Notwendigkeit, als Effektorprotein lediglich ein Fusionsprotein mit Effektorabschnitt und einem für den "Tag" oder das Epitop spezifischen Antikörper- bzw. Bindungsproteinabschnitt bereitzustellen, eine wesentliche Vereinfachung des Verfahrens zur Herstellung erfindungsgemäßer Zellen wie auch der nachfolgend detaillierter beschriebenen Assayverfahren, die sich dieser Zellen bedienen.

Sollen mit Hilfe der beschriebenen Hefezellen die Assays ausgeführt werden, die, wie nachfolgend detaillierter erläutert, spezifisch die Wechselwirkung von Liganden mit dem Ligandenbindungsabschnitt von Membranrezeptoren nachweisen, versteht es sich, daß das Translokationsereignis des Effektorproteins oder -polypeptids an die Zellmembran nur bei einer Ligand-Ligandenbindungsabschnitt-Wechselwirkung oder, alternativ, bei *fehlender* Ligand-Ligandenbindungsabschnitt-Wechselwirkung an dem speziell zu untersuchenden Membranrezeptor auftreten darf. D.h. eine Assoziierung des Effektorproteins oder -polypeptids mit membranständigen Zellkomponenten, die (in Abwesenheit des Membranrezeptors und der mit diesem bei der Signaltransduktion spezifisch und ausschließlich zusammenwirkenden Zellkomponenten) natürlicherweise in diesen Zellen vorkommen, muß, gleich welchen Aktivierungs- oder Modifizierungszustands der Zellkomponenten, generell oder bei Wahl gewisser besonderer Kultivierungsbedingungen für die Zellen ausgeschlossen sein. Dies setzt voraus, daß alle Komponenten, die an der Translokation des Effektorproteins oder -polypeptids an die Zellmembran direkt und indirekt beteiligt sind, generell oder bei Wahl dieser besonderen Kultivierungsbedingungen nur in Folge der Aktivierung des speziell zu testenden Membranrezeptors aufgrund der Bindung eines Liganden an den Ligandenbindungsabschnitt oder, alternativ, aufgrund der fehlenden Ligandenbindung an den Ligandenbindungsabschnitt, und genauer nur in Folge der durch diese Aktivierung bedingten Strukturänderung des Mediatorabschnitts in die Lage versetzt werden können, die Bindung des Effektorproteins oder -polypeptids an eine Komponente der Zellmembran zu vermitteln oder zu bewirken.

Soll in einem alternativen Test ein in einer Zelle natürlicherweise vorkommender Membranrezeptor getestet werden, muß sichergestellt werden, daß in der Zelle der Mediatorabschnitt dieses Membranrezeptors nur in Zusammenhang mit diesem vorkommt und daß die damit zusammenwirkenden Adaptor- und Vermittlerproteine nur mit diesem Mediatorabschnitt oder nur aufgrund einer Aktivierung dieses Mediatorabschnitts in einer Weise interagieren können, daß es schließlich zu einer Translokation des Effektorproteins oder -polypeptids an die Zellmembran kommt.

Alternativ kann eine Zelle als Assayzelle ausgewählt werden, in der alle diese Komponenten vor der Einschleusung der genetischen Information für den Membranrezeptor und die gegebenenfalls benötigten Adapter-, Vermittler- und/oder Effektorproteine bzw. -polypeptide natürlicherweise nicht vorkommen, also zu dieser Ausgangszelle heterolog sind und auch keine zelleigenen Komponenten gleicher Spezifität und ggf. Aktivität, die diese ersetzten könnten, vorliegen; bezüglich des Membranrezeptors kann dies auch nur für den Mediatorabschnitt gelten. Oder es muß dafür Sorge getragen werden, daß eine Ausgangszelle, die diese Komponenten, bezüglich des Membranrezeptors ggf. auch nur den speziellen Mediatorabschnitt, in einem ursprünglichen Zustand einmal enthielt, diese Komponenten aufgrund von genetischer oder sonstiger Modifizierung vor der Einschleusung der genetischen Information für den Membranrezeptor und die gegebenenfalls benötigten Adaptor-, Vermittler- und/oder Effektorproteine bzw. -polypeptide nicht mehr enthält. Zusammenfassend erläutert, betrifft dies zumindest die folgenden Komponenten:
- den Mediatorabschnitt,
- ggf. die damit zusammenwirkenden Adaptorproteine oder Adaptorproteinabschnitte, sofern eine Bindung des Effektorproteins oder -polypeptids an diese oder über diese erfolgt, sowie,
- z.B. im Falle der vorliegend erläuterten Alternative 1)c), ggf. eine Vermittlerkomponente, welche mit dem Mediatorabschnitt des Membranrezeptor räumlich nicht unmittelbar assoziiert sein muß, jedoch im Falle einer Ligandenbindung oder, alternativ, fehlenden Ligandenbindung an den Ligandenbindungsabschnitt von diesem aktiviert oder modifiziert wird und aufgrunddessen ein sekundäres Ereignis an einer weiteren Komponente der Zellmembran vermittelt, infolgedessen sich das Effektorprotein oder -polypeptid spezifisch an diese Komponente der Zellmembran, die mit dem Mediatorabschnitt des Membranrezeptors nicht assoziiert ist, bindet; alternativ darf das genannte sekundäre Ereignis in der Zelle nur bei Ligandenbindung oder, alternativ, *fehlender* Ligandenbindung des Ligandenbindungsabschnitts des Membranrezeptors vermittelt werden. Es dürfen im letzteren Falle in der Zelle also keine Wechselwirkungspartner für diese Vermittler vorliegen, die eine entsprechende Aktivierungswirkung wie der Membranrezeptor auf den oder die Vermittler ausüben können.

Dementsprechend ist bei einigen Ausführungsformen der Anmeldung die Expression bestimmter Typen von Proteinen und Fusionsproteinen in einem geeigneten Zellsystem, das diese Proteine und Fusionsproteine natürlicherweise nicht enthält, ein wesentlicher Aspekt. Je nach eingesetztem Membranrezeptor und insbesondere je nach dem durch den Mediatorabschnitt des Membranrezeptors bedingten Mechanismus, der schließlich zu einer Translokation des Effektorproteins oder -polypeptids an die Membran führt, kann es erforderlich werden, eines oder mehrere der vorstehend genannten Proteine in einer Zelle, die diese(s) natürlicherweise nicht enthält, zu exprimieren. Diese Proteine können in ihrer natürlich vorkommenden Form, sofern es eine solche gibt, oder in Form von Fusionsproteinen, exprimiert werden.

Dabei können die Genkonstrukte, die diese Proteine oder Fusionsproteine kodieren, chromosomal, d.h. im Chromosom integriert, oder extrachromosomal, als Bestandteil eines Episoms, insbesondere Plasmids, in der Zelle vorliegen.

Ein erstes derartiges Fusionsprotein kann ein Membranrezeptor, wie er vorstehend erläutert wurde, sein, bei dem insbesondere und gegebenenfalls auch ausschließlich der Mediatorabschnitt zu der Zelle heterolog ist.

Ein weiteres dieser Fusionsproteine stellt das Effektorprotein oder -polypeptid in einer besonderen Form bereit und besteht aus Domänen bzw. Teilen, die folgende Funktionen vereinen:
i) die membranständige spezifische Interaktion eines Adaptorabschnitts des Fusionsproteins mit einer Komponente der Membran und in einer besonderen Ausführungsform dem Mediatorabschnitt des Rezeptors in Abhängigkeit von der extrazellulären Ligandenbindung oder, alternativ, von *fehlender* extrazellulärer Ligandenbindung (Adaptorfunktion);
ii) die Aktivierungsmöglichkeit eines ras- oder ras-ähnlichen Signaltransduktionsweges in Abhängigkeit von der durch den Adaptorabschnitt bedingten Membranlokalisierung aufgrund eines mit dem Adaptorabschnitt in dem Fusionsprotein fusionierten Effektorabschnitts (Effektorfunktion).
Die erstgenannte Funktion bewirkt, daß das Adaptor-Effektor-Fusionsprotein nur dann an die Membran und damit an den Wirkort desjenigen Teils des Fusionsproteins, welcher für die zweite Funktion verantwortlich ist, gelangt, wenn ein Ligand an den in der Regel extrazellulären Teil, d.h. den Ligandenbindungsabschnitt des Rezeptors gebunden hat oder, alternativ, wenn keine solche Ligandenbindung vorliegt.

Im ersteren Falle liegt der Rezeptor ohne Ligandenbindung in einer Konfiguration vor, die den Adaptorabschnitt nicht direkt binden kann oder einen membranständigen Folgeprozess über einen Mediator nicht auslösen kann, den der Adaptorabschnitt erkennen konnte. Bei Ligandenbindung nimmt der zytosolische Teil des Rezeptors bei dieser Variante dann eine Konfiguration ein, die der Adaptorteil direkt erkennen kann oder er löst einen Folgevorgang aus, der zu einer membranständigen Proteinkonfiguration anderer Mediatorproteine führt, die der Adaptor erkennen kann. In Abwesenheit der Ligandenbindung liegt also das Adaptor-Effektor-Fusionsprotein im Zytosol nicht am wirkort des Effektorabschnitts vor. Nur wenn der Effektorabschnitt membranständig orientiert wird, kann er als Signaltransduktionskomponente im ras-oder ras-ähnlichen Signaltransduktionsweg aktiv werden. (Fig. 2a und b).

Im letzteren Falle liegt der Rezeptor *bei Bindung von Ligand* in einer Konfiguration vor, die den Adaptorabschnitt nicht direkt binden kann oder einen membranständigen Folgeprozess über einen Mediator nicht auslösen kann, den der Adaptorabschnitt erkennen könnte. Bei *Abdissoziierung des Liganden* bzw. fehlender Ligandenbindung nimmt der zytosolische Teil des Rezeptors bei dieser Variante dann eine Konfiguration ein, die der Adaptorteil direkt erkennen kann, oder er löst einen Folgevorgang aus, der zu einer membranständigen Proteinkonfiguration anderer Mediatorproteine führt, die der Adaptor erkennen kann. Bei dieser Variante liegt also das Adaptor-Effektor-Fusionsprotein bei Ligandenbindung im Zytosol nicht am Wirkort des Effektorabschnitts vor. Nur wenn der Effektorabschnitt membranständig orientiert wird, kann er als Signaltransduktionskomponente im ras- oder ras-ähnlichen Signaltransduktionsweg aktiv werden

Eine solche Aktivierung kann, wie weiter unten ausführlicher erläutert wird, detektiert werden über phänotypische Änderungen (z.B. Wachstum oder Gen- bzw. Reportergenaktivität) in der Zelle, wobei die Zellen bei der ersten Variante unter den Assaybedingungen in Abwesenheit des Liganden inaktiv bezüglich des ras-oder ras-ähnlichen Signaltransduktionsweges sein müssen, bei der letzteren Variante unter den Assaybedingungen in Anwesenheit des Liganden inaktiv bezüglich des ras- oder ras-ähnlichen Signaltransduktionsweges sein müssen.

In einem bevorzugt verwendeten experimentellen System der Erfindung umfaßt das Fusionsprotein als Effektorabschnitt ein mutiertes humanes Ras-Protein (Ha-Ras, L61), dem die Farnesylierungssequenz, welche für eine Membranlokalisierung des Proteins sorgt, fehlt.

Auch weitere Protein-Komponenten, die neben dem Membranrezeptor und insbesondere dessen Mediatorabschnitt sowie dem Effektorprotein selbst an der Translokation des Effektorproteins oder -polypeptids unmittelbar oder mittelbar beteiligt sind, können gegebenenfalls als in der Natur nicht vorkommende Fusionsproteine in der Zelle exprimiert werden.

Zum klareren Verständnis der Lehre dieser Unterlagen sei angefügt, daß im vorliegenden Zusammenhang unter dem Begriff "Ligand" nur solche Bindungspartner für Rezeptoren und insbesondere Membranrezeptoren verstanden werden sollen, die bei einer Bindung an den Ligandenbindungsabschnitt eines solchen Rezeptors eine Strukturänderung, insbesondere eine Konformationsänderung und/oder eine enzymatische Modifizierung, z.B. durch Phosphorylierung oder Dephosphorylierung, hervorrufen, deren Auswirkungen auf den Mediatorabschnitt bewirken, daß in der Folge ein Effektorprotein oder -polypeptid, das in der Lage ist, einen Ras- oder Ras-ähnlichen Signalweg inder Zelle zu aktivieren, an eine Komponente der Membran, gegebenenfalls über weitere Proteine oder Polypeptide (Adaptoren) bindet. Dies kann insbesondere Struktur- und speziell Konformationsänderungen umfassen, wie sie *in vivo* bei der Bindung eines natürlichen Liganden erfolgen und wofür Beispiele vorstehend erläutert wurden. Es werden jedoch auch Fälle in Betracht gezogen, bei denen die Struktur- und speziell Konformationsänderung nicht oder nur teilweise der bei Bindung eines *in vivo* in der Zelle vorkommenden Liganden erfolgenden Struktur- bzw. Konformationsänderung entspricht. Bindungspartner, die eine solche Strukturänderung nicht hervorrufen, werden von dem Begriff "Ligand" nicht umfaßt.

Von den hier synonym verwendeten Begriffen "Ras- und Ras-ähnlicher Signalweg" oder "sich an ein Ras-Protein anschließender Signalweg" werden auch die sogenannten ras-ähnlichen Signalwege mitumfaßt, die von diversen weiteren Mitgliedern der Ras-Familie gesteuert werden. Unter den Mitgliedern der Ras-Familie gibt es solche, die trotz Ursprungs aus unterschiedlichen Organismen ein und denselben Signaltransduktionsweg in einer gewählten Zielzelle aktivieren können. Ein Beispiel hierfür ist das humane Ha-Ras (L61), das in der Lage ist, auch einen ras-Signalweg in *Saccharomyces cerevisiae* zu aktivieren, der auf den Zellzyklus einwirkt und dessen Aktivierung für eine Vermehrung der Hefezellen essenziell ist. Andere Mitglieder der Ras-Familie sind nur in der Lage, einen einzigen, für sie spezifischen Signalweg zu aktivieren.

Eine Reihe von Mitgliedern der Ras-Familie, wie das vorstehend erwähnte Ha-Ras (L61), aktiviert Signalwege, die auf den Zellzyklus einwirken und deren Aktivierung über die Aktivierung spezieller Transkriptionsfaktoren für die Zellvermehrung essenziell sind. Andere derartige Ras-Proteine aktivieren Signalwege, die spezifisch zur Aktivierung jeweils eines einer Vielzahl von Transkriptionsfaktoren, die für andere Gene als jene des Zellzyklus spezifisch sind, führen. Im vorliegenden Kontext ist allen ras-Signalwegen gemeinsam, daß sie für ihre Aktivierung ein an der Zellmembran vorliegendes aktives Ras-Protein erfordern, wobei das Ras-Protein gegebenenfalls für seine Aktivität die gleichzeitige Anwesenheit eines Guaninnukleotid-Austauschfaktors an der Zellmembran benötigt.

Wird in diesen Unterlagen auf eine Inaktivierung eines ras-Signalwegs oder eines ras-ähnlichen Signalwegs Bezug genommen, so wird darunter stets eine Inaktivierung auf Ebene des Ras-Proteins und/oder eines dafür spezifischen Guaninnukleotid-Austauschfaktors verstanden. Die genannte Signalwegsinaktivierung tritt in einer Zelle im vorliegenden Zusammenhang bevorzugt nur unter bestimmten Umweltbedingungen, wie Temperatur, auf, kann also durch gezielte Einstellung von Umweltbedingungen induziert und wieder aufgehoben werden.

Im zellulären Kontext wird nun bei jeder der erläuterten Ausführungsformen durch Einwirkung der aktiven Signaltransduktionskomponente ein ras- oder ras-ähnlicher Signaltransduktionsweg aktiviert. Verwendet man eine Hefezelle, bei der dieser ras-oder ras-ähnliche Signalweg in Abwesenheit des erfindungsgemäß untersuchten Membranrezeptors aufgrund von Mutationen zumindest unter bestimmten Bedingungen nicht aktiviert ist, kann eine solche allein durch die infolge der Ligandenbindung oder, alternativ, infolge fehlender Ligandenbindung an eben diesen Membranrezeptor erfolgte Translokation des Effektorproteins oder - polypeptids an die Zellmembran vermittelte Aktivierung über phänotypische Veränderungen, z.B. Wachstum oder Gen- bzw. Reportergenaktivität, in der Zelle detektiert werden.

Das Effektorprotein oder -polypeptid vermag bevorzugt, ras-Signaltransduktionswege zu aktivieren, die auf den Zellzyklus einwirken und deren Aktivierung für die Zellvermehrung essenziell ist. Alternativ und ebenso bevorzugt wirkt es auf einen der Ras-Signalwege ein, die der Aktivierung von Transkriptionsfaktoren für Gene dienen, die für die Zellvermehrung nicht essenziell sein müssen.

Das Effektorprotein oder -polypeptid kann die Aktivität eines aktiven und insbesondere eines konstitutiv aktiven Ras-Proteins aufweisen. Konstitutiv aktive Ras-Proteine zeigen Aktivität unabhängig von der Anwesenheit von Guaninnukleotid-Austauschfaktor-Molekülen, die diverse andere Ras-Proteine für ihre Aktivität benötigen. Zu diesem Zweck kann das Effektorprotein oder -polypeptid beispielsweise die Aminosäuresequenz eines aktiven oder konstitutiv aktiven Ras-Proteins, das in der Natur vorkommt, z.B. dem humanen Ha-Ras (L61), oder von Teilen davon umfassen. Oder es kann Aminosäuresequenzen umfassen, die von derartigen Sequenzen, z.B. durch Aminosäureanfügung, -austausch, -modifizierung, -insertion oder -deletion, abgeleitet sind.

Alternativ kann das Effektorprotein oder -polypeptid die Aktivität eines funktionellen Guaninnukleotid-Austauschfaktors ("guanine nucleotide exchange factor") aufweisen. In dieser Hinsicht kann die Aminosäuresequenz des Effektorproteins oder -polypeptids ebenfalls beispielsweise Sequenzen von in der Natur vorkommenden Guaninnukleotid-Austauschfaktoren oder Teilsequenzen davon umfassen oder sie kann davon, z.B. durch Aminosäureanfügung, -austausch, -modifizierung, -insertion oder -deletion, abgeleitet sein. In einer bevorzugten Ausführungsform ist die Aminosäuresequenz des Effektorproteins oder -polypeptids abgeleitet von der Aminosäuresequenz des CDC25-Proteins aus *Saccharomyces cerevisiae,* eines SOS-Proteins aus einem Säugetier oder eines aus einem beliebigen Organismus abgeleiteten SOS-ähnlichen Proteins.

Darüber hinaus umfaßt die Anmeldung DNA-Moleküle, die neue Fusionsproteine, die ebenfalls Gegenstand der Anmeldung sind, kodieren, sowie Vektoren, insbesondere Plasmide, Cosmide, Viren- oder Phagengenome, die mindestens eines dieser DNA-Moleküle umfassen. Besondere Vektoren sind zur Transformation oder Transfektion von Wirtszellen oder zur Expression mindestens eines heterologen Fusionsproteins geeignet. Zu dem letztgenannten Zweck steht ein DNA-Molekül in dem Vektor unter Kontrolle eines in einer Wirtszelle funktionsfähigen Promotors, der die Expression ermöglicht und steuert.

Die Herstellung der heterologen Fusionsproteine, DNA-Moleküle und Vektoren kann nach im Stand der Technik bekannten Protokollen erfolgen (siehe z.B. Sambrook, J., Fritsch, E.F., Maniatis, T. (1989), Molecular Cloning. A Laboratory Handbook, Cold Spring Harbor Laboratory, New York; Current Protocols in Molecular Biology (1991)). Auch wenn die Fusionsproteine grundsätzlich vollsynthetisch aus einzelnen Aminosäure-Derivaten hergestellt werden können, wozu im Stand der Technik diverse Verfahren zur Verfügung stehen, werden sie üblicherweise über die Expression der entsprechenden Gene in Zellen produziert. Dabei kann das Gen für das Fusionsprotein extrachromosomal oder in das Genom der Wirtszelle integriert vorliegen. Die Klonierung der Gene für die Fusionsproteine ausgehend von bekannten Genabschnitten, die Proteinabschnitte mit den erforderlichen oder im Falle des Ligandenbindungs- oder Rezeptorabschnitts auch vorerst nur vermuteten Funktionen kodieren, gehört ebenso zu den Standardfähigkeiten eines Fachmanns, wie die Konstruktion von Vektoren, wie Transkriptions- oder Transfektionsvektoren oder auch Expressionsvektoren, in denen das Gen in funktionaler Verknüpfung mit einem in der Produktionszelle wirksamen Promotor vorliegt, die Transformation oder Transfektion von Wirtszellen wie auch die Züchtung der transformierten bzw. transfizierten Wirtszellen zur Produktion des Proteins. Die Isolierung und Reinigung der Fusionsproteine kann durch Einsatz herkömmlicher Verfahren, wie Fällung, Einsatz diverser Chromatographieverfahren, wie Gelfiltration, Affinitätschromatographie u.s.w. erfolgen. Insbesondere erlaubt die Affinitätschromatographie beispielsweise bei Verwendung von an die Matrix gebundenen spezifischen Antikörpern oder Bindungsproteinen, die gegen eine Determinante eines bezüglich der Wirtszelle heterologen Abschnitts des Fusionsproteins gerichtet sind, eine selektive Bindung nur des Fusionsproteins. Alternativ kann das Fusionsprotein beispielsweise auch als Vorläuferprotein exprimiert werden, das eine zusätzliche Domäne mit einer spezifischen Bindungseigenschaft an eine bestimmte Affinitätssäule aufweist. Nach Bindung und darauffolgender Elution von der Affinitätssäule kann die zusätzliche Domäne dann selektiv von dem nun bereits im wesentlichen rein vorliegenden Vorläuferprotein unter Erzeugung des Fusionsprotein abgespalten werden. Sofern die zusätzliche Domäne die Eignung des Fusionsproteins für die erfindungsgemäßen Assays nicht beeinflußt, besteht jedoch alternativ auch die Möglichkeit, auf den Abspaltungsschritt zu verzichten. Ein Beispiel für eine derartige Domäne besteht aus mehreren, z.B. 10, zusätzlich N-terminal angefügten Histidinresten ("His-tag"), die spezifisch an eine MetallChelat-Affinitätschromatographiesäule binden. Bezüglich aller genannten Techniken und den dafür erforderlichen Reagenzien, einschließlich Vektormolekülen, kann auf Standardliteratur (z.B. Sambrook, J., Fritsch, E.F., Maniatis, T. (1989), a.a.O.; Current Protocols in Molecular Biology (1991)) und eine unübersehbare Vielzahl von Einzelprotokollen verwiesen werden.

Ein weiterer zentraler Gegenstand der Anmeldung sind neben den vorstehend erläuterten Zellen auch Zellen, die einen oder mehrere der Membranrezeptoren, wie sie in Anspruch 1 beschrieben wurden, insbesondere eines oder mehrere der Membranrezeptor-Fusionsproteine enthalten. Im Rahmen dieser Anmeldung sind dabei einzelne, mehrere oder auch alle Domänen des Membranrezeptors/Fusionsproteins und/oder gegebenenfalls auch Teile eines oder mehrerer Abschnitte oder Domänen von diesen bezüglich der Wirts- oder Ausgangszelle heterolog.

Zur Herstellung der Zellen kann beispielsweise eine Transformation oder Transfektion von Ausgangszellen mit einem Expressionsvektor, der ein Gen für den Membranrezeptor mit den in Anspruch 1 erläuterten Merkmalen, insbesondere ein Fusionsprotein unter Kontrolle eines in der Ausgangszelle funktionsfähigen Promotors enthält, erfolgen. Als Ausgangszellen werden Hefezellen, wie bestimmte Stämme von *Saccharomyces cerevisiae*, seswendet.

Die Zellen können auch in Form von zellwandlosen "ghost"-Formen der Hefen, die im vorliegenden Kontext von dem Begriff "Zellen" ebenfalls umfaßt werden, bereitgestellt werden.

Ein wesentliches Merkmal der Zellen besteht darin, daß bei fehlender Bindung von Ligand an den Ligandenbindungsabschnitt des Membranrezeptors das Effektorprotein oder -polypeptid nicht in der Lage ist, die Aktivierung des speziellen Ras- oder Ras-ähnlichen Signalwegs in den Zellen zu bewirken, oder, alternativ, daß das Effektorprotein oder -polypeptid speziell bei Ligandenbindung an den Ligandenbindungsabschnitt des Membranrezeptors nicht in der Lage ist, die Aktivierung des speziellen Ras- oder Ras-ähnlichen Signalwegs in den Zellen zu bewirken.

In einer bevorzugten Ausführungsform ist die Hefezelle **dadurch gekennzeichnet, daß** in Abwesenheit des in Anspruch 1 erläuterten Membranrezeptors zumindest unter bestimmten Bedingungen ein Ras- oder Ras-ähnlicher Signalweg in der Zelle nicht aktiviert werden kann, und insbesondere der Signalweg, zu dessen Aktivierung das Effektorprotein oder - polypeptid in der Lage ist. So sind im Stand der Technik Zellen bekannt, bei denen ein bestimmter ras-Signaltransduktionsweg temperaturabhängig aktiv bzw. inaktiv ist. Derartige Zellen können als Ausgangszellen für eine Expression der heterologen Fusionsproteins oder Membranrezeptors eingesetzt werden.

Die zumindest unter bestimmten Bedingungen vorliegende Inaktivierung eines ras-Signaltransduktionswegs resultiert aus einem zumindest unter den bestimmten Bedingungen nicht funktionsfähigen Ras-Protein und/oder Guaninnukleotid-Austauschfaktor. Die Inaktivierung kann auf Genmutation oder vollständiger oder teilweiser Gendeletion beruhen. Beispielsweise kann ein zelleigenes Ras-Protein inaktiviert werden, wenn dessen Membranlokalisierungssignal, in der Regel ein Farnesylierungssignal, deletiert wird. Gleiche Wirkung hätte eine Mutation in diesem Membranlokalisierungssignal, welche bewirkt, daß keine Bindung des Ras-Proteins an zelluläre Membranen mehr erfolgen kann. Ein Beispiel für eine Zelle mit einem temperaturabhängig defekten Guaninnukleotid-Austauschfaktor ist der *Saccharomyces cerevisiae-*Hefestamm cdc25-2 (Broder et al., 1998). Bei diesem Stamm ist der Guaninnukleotid-Austauschfaktor bei einer restriktiven Temperatur von 33 bis 37°C, typischerweise 36°C, nicht mehr aktiv, jedoch bei einer Temperatur von beispielsweise 25°C voll funktionsfähig. Da der Guaninnukleotid-Austauschfaktor in diesem Hefestamm mit einem Ras-Protein zusammenwirkt, das einen auf den Zellzyklus.einwirkenden und daher für das Zellwachstum essenziellen ras-Signal-transduktionsweg steuert, ist bei einer restriktiven Temperatur keine Vermehrung der Zellen des Hefestamms mehr festzustellen.

In analoger Weise zu Hefestämmen mit einer temperatursensitiven Mutation eines hefeeigenen SOS-Proteins (CDC25-2) kann auch ein Hefestamm mit einer temperatursenstiven Mutation eines hefeeigenen Ras-Proteins eingesetzt werden.

Alternativ kann für die Herstellung der erfindungsgemäßen Zellen aber auch eine Hefezelle verwendet werden, die zwar in der Lage ist, ein wildtypisches oder mutiertes, aber aktives CDC25/SOS-Protein oder Ras-Protein zu exprimieren, in der jedoch das dieses aktive CDC25/SOS-Protein oder Ras-Protein kodierende Gen unter der Kontrolle eines induzierbaren Promoters steht, durch welchen die Expression des Gens über die Wahl bestimmter Kulturbedingungen gezielt an- oder abgeschaltet werden kann. Beispiele für in diesem Zusammenhang einsetzbare induzierbare Promotoren sind der Galactose-Promotor oder Teile davon aus Hefe oder anderen Organismen. Dem Fachmann ist eine Vielzahl von hierzu geeigneten, induzierbaren Promotoren aus den unterschiedlichsten Organismen bekannt. Es können auch hybride Promotoren mit geeigneter Induzierbarkeit eingesetzt werden.

Exprimiert die Zelle ein aktives CDC25/SOS-Protein oder ein aktives Ras-Protein, so kann in einer weiteren bevorzugen Ausführungsform der Anmeldung das CDC25/SOS- oder Ras-Protein zusätzlich eine Modifizierung enthalten, durch die das Protein in der Zelle beschleunigt degradiert wird. Diese Modifizierung kann beispielsweise ein Ubiquitin-Signal oder ein sonstiges Signal sein, welches für den bevorzugten Abbau eines derartig modifizierten Proteins in der Zelle sorgt. Der Vorteil der Expression eines derartig modifizierten Proteins während einer Induktion des Promotors liegt darin, daß nach "Abschalten" des Promotors, d.h. nach Vorsehen von Kulturbedingungen, bei denen der Promotor nicht induziert ist und dementsprechend keine Transkription des CDC25/SOS- oder Ras-Gens mehr erfolgt, das noch zu Zeiten der Induktion des Promotors produzierte CDC25/SOS- oder Ras-Protein beschleunigt abgebaut wird. Dementsprechend wird bereits kurze Zeit nach "Abschalten" des Promotors kein derartiges aktives CDC25/SOS- oder Ras-Protein in der Zelle mehr nachzuweisen sein. In der bevorzugten Situation, wo der Mediatorabschnitt des untersuchten Membranrezeptors in der Lage ist, über seine Einwirkung auf das Effektorprotein oder -polypeptid eben den durch das vorstehend angesprochene aktive CDC25/SOS- oder Ras-Protein aktivierten Signalweg zu aktivieren, ist es somit möglich, bereits kurze Zeit nach Wechsel der Kulturbedingungen zur Abschaltung des Promotors die Aktivierung dieses Signalwegs ausschließlich aufgrund der Wirkungen des untersuchten Membranrzeptors zu messen, was einen beträchtlichen Zeitvorteil bedeuten kann. Zudem kann auf diese Weise das Hintergrundsignal aufgrund einer möglicherweise noch oder nach wie vor in geringem Umfang vorliegenden Aktivierung des Signalwegs, die nicht auf den erfindungsgemäß untersuchten Membranrezeptor zurückzuführen ist, signifikant verringert werden.

Beruht die Inaktivierung oder Inaktivierbarkeit des zelleigenen ras-Signaltransduktionswegs auf einem Defekt oder Fehlen eines Guaninnukleotid-Austauschfaktors, so kann in dem bevorzugten Falle, wo das Effektorprotein oder -polypeptid eben diesen ras-Signaltransduktionsweg aktivieren kann, dieses Effektorprotein oder -polypeptid die Aktivität eines funktionellen Guaninnukleotid-Austauschfaktors oder eines aktiven, insbesondere konstitutiv aktiven Ras-Proteins aufweisen, welcher bzw. welches den inaktiven ras-Signaltransduktionsweg aktivieren kann. Wird ein Effektorprotein oder -polypeptid mit einer Aktivität eines nicht konstitutiv aktiven Ras-Proteins eingesetzt, so wird es sinnvollerweise die folgenden Eigenschaften aufweisen:
- es benötigt eine Aktivierung durch einen andersartigen Guaninnukleotid-Austauschfaktor, der mit dem zelleigenen Ras-Protein des inaktiven ras-Signaltransduktionsweg nicht funktional wechselwirken kann. Gegebenenfalls kann dieser spezifisch geeignete Guaninnukleotid-Austauschfaktor als heterologer Faktor in der Zelle oder Assay-Zelle koexprimiert werden.
   Ist die Inaktivierung oder Inaktivierbarkeit des zelleigenen ras-Signalwegs auf einen Defekt oder ein Fehlen eines zelleigenen Ras-Proteins zurückzuführen, so wird in dem bevorzugten Falle, wo das Effektorprotein oder -polypeptid eben diesen ras-Signalweg aktivieren kann, dieses die Aktivität eines aktiven, insbesondere konstitutiv aktiven Ras-Proteins aufweisen. Weist das Effektorprotein oder -polypeptid die Aktivität eines nichtkonstitutiv aktiven Ras-Proteins auf, so erfolgt deren Aktivierung bevorzugt durch einen zelleigenen Guaninnukleotid-Austauschfaktor, kann jedoch alternativ hierzu auch einen in der Zelle kozuexprimierenden heterologen Guaninnukleotid-Austauschfaktor benötigen.
   Die für die Herstellung der Zellen erforderlichen molekularbiologischen Techniken, z.B. Klonierung, Vektorkonstruktion, Transformation oder Transfektion, Selektionierung transformierter bzw. transfizierter Zellen und Züchtung der transformierten bzw. transfizierten Zellen u.s.w., sind dem Fachmann wohlbekannt und es existieren viele allgemeine Protokolle dafür, die gegebenenfalls allenfalls geringfügig angepaßt werden müssen, siehe z.B. Sambrook, J., Fritsch, E.F., Maniatis, T. (1989), a.a.O.; Current Protocols in Molecular Biology (1991) sowie zahlreiche Protokolle, die speziell für einen bestimmten Zelltyp erstellt wurden. Die Expression von beispielsweise heterologen Proteinen und Fusionsproteinen kann dabei, wie erwähnt, ausgehend von einem innerhalb eines Episoms, insbesondere Plasmides enthaltenen, extrachromosomal vorliegenden Gen wie auch ausgehend von einem in das Genom der Ausgangszelle integrierten Gen erfolgen. Für die Erzeugung von Zellen, bei denen ein bestimmter ras-Signaltransduktionsweg auf Ebene des Ras-Proteins und/oder eines Guaninnukleotid-Austauschfaktors inaktiviert ist, und insbesondere zur gezielten Geninaktivierung, welche auch in anderem Zusammenhang bei bestimmten Assay-Konstellationen erforderlich werden kann, stehen dem Fachmann diverse Techniken, beispielsweise durch "antisense"-Strategien, oder zur gezielten Einführung von Mutationen oder Deletionen in die jeweiligen Gene bzw. zugehörigen Genomabschnitte zur Verfügung. Insbesondere gibt es diverse bekannte Möglichkeiten zur Herstellung von Zellmutanten, in denen die Transkription der Gene z.B. für das Ras-Protein oder einen Guaninnukleotid-Austauschfaktor gezielt unter bestimmten Bedingungen, z.B. temperaturabhängig, inaktiviert werden kann. Hierzu enthalten diese Zellen diese Gene insbesondere in Verknüpfung mit Promotoren, die unter bestimmten Bedingungen, wie ab einer bestimmten Temperatur, inaktiv werden.
   In einer besonderen Ausführungsform sind die erfindungsgemäßen Zellen und Assayzellen auf einem festen Träger aufgebracht. Als geeignete Trägersubstanzen sind im Stand der Technik insbesondere Polysaccharide, z.B. Agarose, spezielle Kunststoffe, wie Polyacrylamide, Polystyrol, Polyvinylalkohol, Silikone, oder auch bestimmte Glasqualitäten bekannt. Der Träger kann hier in Form diskreter Teilchen, z.B. Kügelchen, oder als im wesentlichen plattenförmiges Substrat, z.B. in Form einer Mikrotiterplatte, vorliegen. Der Bedeckung des Trägers mit den Zellen kann vollständig sein, wie dies in der Regel z.B. bei Trägerkügelchen der Fall ist, oder auch nur auf Teilen oder Abschnitten desselben vorliegen, wie z.B. nur in den Vertiefungen einer Mikrotiterplatte. In einer bevorzugten Ausführungsform sind die Zellen auf sogenannten Biochips immobilisiert (Wolf et al. 1997 und 1998). Auf festen Träger und insbesondere Biochips immobilisiert, werden die Zellen in dieser Form vor allem in High-Throughput-Verfahren zum Nachweisen und Messen von Membranrezeptor-Liganden-Interaktionen eingesetzt. Methoden zur Immobilisierung der Zellen auf diesen Trägern sind dem Fachmann bekannt. In Abhängigkeit von dem gewählten Trägertyp ist es möglich, daß die Zellen ohne weitere Maßnahmen an den Träger binden. In diesem Falle wird die feste Trägerphase mit einer im wesentlichen homogenen Population von Zellen inkubiert,
wobei diese sich in der Folge an die feste Phase anheften. Alternativ kann die Immobilisierung beispielsweise auch mittels chemischer Reagenzien, wie Glutaraldehyd, Formalin u.s.w., erfolgen. Derartige Maßnahmen sind dem Fachmann bekannt.

Die transformierten Hefezellen und heterologen Fusionsproteine sind die Grundlage einer Reihe von in vivo-Assayverfahren, die ebenfalls Gegenstand dieser Anmeldung sind.

Die unter Nutzung der ersten Variante, d.h. nur bei Ligandenbindung an den Ligandenbindungsabschnitt des untersuchten Membranrezeptors möglicher Effektorprotein- bzw. - polypeptidtranslokation an die Zellmembran, realisierbaren, im folgenden näher erläuterten Assayverfahren können u.a. dazu genutzt werden,
1. die Eignung einer Testsubstanz als Ligand für einen Rezeptor zu bestimmen, und in diesem Falle insbesondere Massenscreens mit Ligandenderivaten durchzuführen, um zu testen, welche Derivate in der Lage sind, an den wildtypischen Ligandenbindungsabschnitt des Rezeptors zu binden,
2. die Anwesenheit eines bestimmten Liganden in einer Probe zu detektieren,
3. die Konzentration eines solchen Liganden in einer Probe zu bestimmen,
4. nachzuweisen, ob eine Verbindung in der Lage ist, eine Bindungsaktivität eines Ligandenbindungsabschnitts eines Rezeptors gegenüber einem Liganden zu verändern, also als Agonist, Antagonist oder Inhibitor zu wirken, und in diesem Falle insbesondere Massenscreens zum Auffinden solcher Agonisten- oder Antagonistenverbindungen auszuführen; und
5. die Ligandenbindungsfunktion eines Polypeptids oder Proteins, bei denen eine solche Funktion vermutet wird, für Liganden bestimmter Rezeptoren nachzuweisen; bei den Polypeptiden oder Proteinen kann es sich insbesondere um durch Mutation von natürlichen Rezeptoren abgeleitete neuartige Ligandenbindungsabschnitte von Rezeptoren handeln, deren Ligandenbindungsfunktion noch bestätigt werden muß; in diesem Zusammenhang können insbesondere Massenscreens mit derartigen neuartigen, mutierten Ligandenbindungsabschnitten, die beispielsweise in Form einer Rezeptormutantenbibliothek, die insbesondere Rezeptormutanten mit zufallsbedingt lokalisierten Mutationen im Ligandenbindungsabschnitt enthält, durchgeführt werden, um neue künstliche, funktionelle Liganden-Rezeptor-Partner zu finden.

Ein erster Assay dient der Bestimmung der Eignung einer Testsubstanz als Ligand für einen Rezeptor- oder, synonym, Ligandenbindungsabschnitt eines Rezeptors und umfaßt die folgenden Schritte:
(a) Inkontaktbringen der Testsubstanz mit Zellen unter Bedingungen, bei denen bei Fehlen des Membranrezeptors ein Ras- oder Ras-ähnlicher Signalweg in den Zellen nicht aktiviert werden kann, wobei der in den Zellen enthaltene Membranrezeptor den genannten Ligandenbindungsabschnitt enthält und das Effektorprotein oder -polypeptid, dessen Bindung an eine Membrankomponente von der Bindung eines Liganden an den Ligandenbindungsabschnitt des Membranrezeptors abhängig ist, in der Lage ist, den inaktiven Ras- oder Ras-ähnlichen signalweg zu aktivieren,
(b) Untersuchen, ob eine Aktivierung des Ras- oder Ras-ähnlichen Signalwegs erfolgt ist.
Ein Nachweis der Aktivierung des Ras- oder Ras-ähnlichen Signalwegs zeigt dabei die Bindungsfähigkeit der Testsubstanz an den Ligandenbindungsabschnitt an.

Ein weiterer *in vivo*-Assay ermöglicht den Nachweis der Anwesenheit eines Liganden für einen Ligandenbindungsabschnitt eines Rezeptors in einer auf den Liganden hin zu untersuchenden Probe, und ist durch die folgenden Schritte gekennzeichnet:
(a) Inkontaktbringen der Probe mit transformierten Hefezellen nach Anspruch 9 unter Bedingungen, bei denen bei Fehlen des heterologen Membranrezeptors der Ras- oder Ras-ähnliche Signalweg in der Zelle nicht aktiviert werden kann, wobei der heterologe Membranrezeptor den genannten Ligandenbindungsabschnitt enthält und das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors, wie in Anspruch 1 definiert, abhängig ist, in der Lage ist, diesen Ras- oder Ras-ähnlichen Signalweg zu aktivieren,
(b) Untersuchen, ob eine Aktivierung des Ras- oder Ras-ähnlichen Signalwegs erfolgt ist,
(c) wobei für den Fall, dass die Bindung des Efektorproteins oder -polypeptids von der Bindung eines Liganden an den Ligandenbindungsabschnitt des heterologen Membranrezeptors abhängig ist, ein Nachweis der Aktivierung des Ras- oder Ras-ähnlichen Signalwegs die Abwesenheit eines Liganden oder Ligandenbindungsabschnitt eines Rezeptors in der Probe anzeigt,
   und wobei für den Fall, dass die Bindung des Effektorproteins oder -polypeptids von der fehlenden Bindung eines Liganden an den Ligandenbindungsabschnitt des heterologen Membranrezeptors abhängig ist,
   Zellen, wie sie in Schritt (a) eingesetzt wurden, unter Bedingungen, bei denen bei Fehlen des heterologen Membranrezeptors der Ras- oder Ras-ähnliche Signalweg in der transformierten Hefezelle nicht aktiviert werden kann, in Abwesenheit der Probe auf Aktivierung des Ras- oder Ras-ähnlichen Signalwegs untersucht werden,
wobei ein Nachweis der Aktivierung des Ras- oder Ras-ähnlichen Signalwegs in Abwesenheit der Probe und die Inaktivität des Ras- oder Ras-ähnlichen Signalwegs in Anwesenheit der Probe die Anwesenheit eines Liganden für den Ligandenbindungsabschnitt eines Rezeptors in der Probe anzeigt.
Analog zu dem erstgenannten Assay zeigt ein Nachweis der Aktivierung des Ras- oder Ras-ähnlichen Signalwegs die Anwesenheit eines Liganden für den Ligandenbindungsabschnitt eines Rezeptors in der Probe an.

Als bevorzugte Ras- oder Ras-ähnliche Signalwege, im Folgenden alternativ auch nur als Ras-Signalwege bezeichnet, werden in diesem Zusammenhang, wie erläutert, Signalwege angesehen, die auf den Zellzyklus einwirken und deren Aktivierung für die Zellvermehrung essenziell ist. Alternative und ebenso bevorzugte ras-Signalwege dienen der Aktivierung von Transkriptionsfaktoren für Gene, die für die Zellvermehrung nicht essenziell sein müssen.

Der Nachweis der Ras-Signalwegsaktivierung erfolgt bei den Assays somit bevorzugt indirekt, d.h. über phänotypische Änderungen, hier insbesondere Zellvermehrung oder Gen- bzw. Reportergenaktivität, in den Zellen.

Werden dementsprechend für die Assays Zellen eingesetzt, bei denen der inaktive Ras- oder Ras-ähnliche ein Signalweg ist, der auf den Zellzyklus einwirkt und dessen Aktivierung für die Zellvermehrung essenziell ist, umfassen die vorstehend erläuterten Schritte (b), zu untersuchen, ob die Zellen unter den genannten Bedingungen vermehrungsfähig sind, wobei ein Nachweis der Vermehrungsfähigkeit der Zellen die Bindungsfähigkeit der Testsubstanz an bzw, die Anwesenheit eines Liganden für den Ligandenbindungsabschnitt des Membranrezeptors in der Probe anzeigt. Setzt man für die Assays alternativ Zellen ein, bei denen der inaktive Ras- oder Ras-ähnliche Signalweg ein Signalweg ist, der auf die Aktivität eines Transkriptionsfaktors für ein Gen, das für die Zellvermehrung nicht notwendigerweise essenziell ist, einwirkt, so kann bei gleichzeitigem Vorliegen eines Konstrukts, umfassend eine Bindungsstelle für den Transkriptionsfaktor, einen damit zusammenwirkenden Minimalpromotor und ein unter Kontrolle des Minimalpromotors stehendes Gen für ein Reporterprotein, der Nachweis einer Expression des Reportergens durch Detektion des Transkriptions- oder Translationsprodukts von diesem für die Feststellung der Aktivierung des ras-Signaltransduktionswegs und damit einer erfolgten Ligandenbindung an den Ligandenbindungsabschnitt des Membranrezeptors herangezogen werden. Denn nur bei Aktivierung des ras-Signaltransduktionsweges kann es zu einer Aktivierung des genannten Transkriptionsfaktors kommen, welcher in der Folge über die Bindung an seine Bindungsstelle den Minimalpromotor aktivieren kann und damit die Expression des Reportergens ermöglicht.

In einer bevorzugten Ausführungsform handelt es sich bei dem Reportergen bzw. -protein um ein bezüglich der Assayzelle heterologes Gen bzw. Protein, dessen Anwesenheit spezifisch nur dann nachgewiesen werden kann, wenn die Expression des synthetischen Promotor-Reportergen-Konstrukts aufgrund der Aktivierung des spezifischen ras-Signalweges und der infolge auftretenden Aktivierung des spezifischen Transkriptionsfaktors erfolgt. Erfolgt der Nachweis nicht als direkter Nachweis des Transkriptions- oder Translationsprodukts mittels dafür spezifischer Nukleinsäuresonden oder Antikörper, sondern z.B. über die enzymatische Aktivität des Translationsprodukts, muß bei einer Verwendung von Enzyme kodierenden Genen vorab sichergestellt werden, daß die verwendete Assayzelle vor der Transformation oder Transfektion mit dem synthetischen Promotor-Reportergen-Konstrukt eine enzymatische Aktivität, wie sie das bei Ligandenbindung exprimierte heterologe Enzym ausübt, nicht enthält. Entsprechendes gilt für die anderen Reporterproteintypen.

Alternativ kann als Reportergen auch ein bezüglich der Assayzelle homologes Gen eingesetzt werden. Eine Reportergenexpression infolge der Aktivierung eines Transkriptionsfaktors, die nur aufgrund der im Assay nachzuweisenden Aktivierung eines Ras- oder Ras-ähnlichen Signalweges in den Zellen erfolgt, wird in diesem Falle zu einer Erhöhung der in den Zellen vorliegenden Mengen an Transkriptionsprodukt des Reportergens und gegebenenfalls auch einer erhöhten Menge an Translationsprodukt des Reportergens führen, welche anhand von Vergleichsversuchen ohne Einsatz von Ligand, z.B. mittels Northern-Blotting oder Western-Blotting, ermittelt werden können.

Bei Wahl dieser beiden letztgenannten Alternativen ist die Kenntnis des jeweiligen durch den gewählten ras-Signaltransduktionsweg aktivierten Transkriptionsfaktors sowie des mit diesem Transkriptionsfaktor zusammenwirkenden Promotorabschnitts bzw. von dessen Sequenz erforderlich. Um diese Assayvariante zu ermöglichen, wird die Assayzelle mit einem Konstrukt, umfassend den Promotor in funktionaler Verknüpfung mit dem Reportergen, transformiert bzw. transfiziert, was gegebenenfalls im Wege der Cotransformation oder Cotransfektion zusammen mit einem oder mehreren Konstrukt(en), die Gene für weitere Komponenten des Assaysystems, z.B. den Membranrezeptor, enthalten, erfolgen kann.

Wie erwähnt, können diese Konstrukte nach Transformation oder Transfektion einer Ausgangszelle chromosomal oder extrachromosomal, d.h. als Bestandteil eines Episoms, z.B. Plasmids, in der Assayzelle vorliegen.

Gegenwärtig sind bereits eine Reihe von ras-Signaltransduktionswegen z.B. in unterschiedlichen eukaryotischen Organismen vollständig auch bezüglich der dadurch aktivierten Transkriptionsfaktoren und den damit zusammenwirkenden Promotorbereichen erforscht. Somit steht dem Fachmann für eine diesbezügliche Auswahl eine Reihe von Möglichkeiten zur Verfügung.

In einer bevorzugten Ausführungsform umfaßt das Reporterprotein eine Modifizierung, durch die das Protein in der Zelle beschleunigt abgebaut oder degradiert wird. Diese Modifizierung kann beispielsweise ein Ubiquitinsignal oder sonstiges Signal sein, welches für den Abbau eines derartig modifizierten Proteins sorgt. Der Vorteil der Verwendung eines Reporterproteins, das in einer Assayzelle beschleunigt abgebaut wird, ist angesichts der Tatsache, daß unter den Assay-Bedingungen auch ohne Aktivierung des Ras- oder Ras-ähnlichen Signalwegs durch den Membranrezeptor in der Assayzelle nahezu stets eine geringe Hintergrund-Expression des Reportergenkonstrukts nachzuweisen sein wird, offensichtlich: durch den beschleunigten Abbau des Reporterproteins wird das aus dieser Hintergrund-Expression resultierende Signal bei Detektion auf Proteinebene, d.h. des Reporterproteins, signifikant verringert, da es zu keiner Akkumulation von Reporterprotein über die Zeit kommt. Wird jedoch die Expression des Reporterproteins durch Ligandenbindung an den Membranrezeptor und daraus resultierende Aktivierung eines Ras-oder Ras-ähnlichen Signalwegs spezifisch aktiviert, kann aufgrund des geringen Hintergrundsignals eine eindeutige Detektion erfolgen. Da die Halbwertszeit des Reporterproteins in der Assayzelle stets ausreichend lang sein wird, wird der Nachweis des infolge der Ligandenbindung an den Membranrezeptor produzierten Reporterproteins durch den beschleunigten Abbau desselben in keiner weise beeinträchtigt werden.

Die für die Herstellung von Transformations- oder Expressionsvektoren, die das Reportergen in funktionaler Verknüpfung mit einem geeigneten spezifischen Promotor enthalten, sowie die Transformation oder Transfektion von Zellen erforderlichen molekularbiologischen Techniken, z.B. Klonierung, Vektorkonstruktion u.s.w., sind dem Fachmann wohlbekannt und es existieren zahlreiche allgemeine Protokolle dafür, die gegebenenfalls allenfalls einer geringfügigen Anpassung bedürfen (siehe z.B. Sambrook, J., Fritsch, E.F., Maniatis, T. (1989), a.a.O.; Current Protocols in Molecular Biology (1991)). Auch die Ergänzung bzw. Fusion eines Reportergens mit einem Sequenzabschnitt, der einen Signalabschnitt kodiert, welcher einen beschleunigten Abbau des exprimierten Reporterproteins bewirkt, z.B. ein Ubiquitin-Signal, liegt ohne weiteres im Vermögen eines Fachmanns.

Als hier einsetzbare Reportergene sind dem Fachmann zahlreiche Gene bekannt, die Proteine kodieren, die einem einfachen und schnellen Nachweis zugänglich sind. Beispiele hierfür sind Gene, die enzymatisch aktive Proteine, z.B. β-Galactosidase, fluoreszierende Proteine, z.B. GFP ("green fluorescence protein") oder chemolumineszierende Proteine kodieren. Bine weitere Möglichkeit besteht in Genen, die Proteine kodieren, die mittels spezifischer Antikörper nachgewiesen werden können. Hierbei trägt dann der Antikörper eine nachweisbare Markierung oder kann seinerseits durch einen sekundären, markierten Antikörper nachgewiesen werden. Derartige Möglichkeiten sind im Stand der Technik wohlbekannt. wie bereits vorstehend erläutert, ist neben dem Brfordernis der Nachweisbarkeit allein essenziell, daß das in der Zelle nachzuweisende Ereignis, z.B, enzymatische Aktivität, Antikörperbindung, Fluoreszenz, Chemolumineszenz, in Abwesenheit des Konstrukts mit dem Gen für das Reporterprotein nicht nachweisbar ist.

Alternativ kann die Transkription des Reportergens anhand der gebildeten mRNA mittels dafür spezifischer Sonden durch Northern-Blotting nachgewiesen werden.

Ein weiterer *in vivo*-Assay, welcher nicht Gegenstand der Erfindung ist, erlaubt die quantitative Bestimmung der Konzentration eines Liganden für einen Ligandenbindungsabschnitt eines Rezeptors in einer Probe, die diesen enthält, gekennzeichnet durch die folgenden Schritte:
(a) Inkontaktbringen eines Aliquots der Probe mit erfindungsgemäßen Zellen unter Bedingungen, bei denen bei Fehlen des Membranrezeptors ein Ras- oder Ras-ähnlicher Signalweg in der Zelle nicht aktiviert werden kann, wobei der Membranrezeptor den genannten. Ligandenbindungsabschnitt enthält und das Effektorprotein oder -polypeptid, dessen Bindung an eine Membrankomponente von der Bindung eines Liganden an den Ligandenbindungsabschnitt des Membranrezeptors, wie in Anspruch 1 definiert, abhängig ist, in der Lage ist, den inaktiven Ras- oder Ras-ähnlichen Signalweg zu aktivieren,
(b) quantitatives Nachweisen des Ausmaßes der Aktivierung des Ras- oder Ras-ähnlichen Signalweges auf direktem oder indirektem Weg,
(c) Ermittlung der Konzentration des Liganden in der Probe durch Vergleich des ermittelten Aktivierungsausmaßes mit entsprechenden Werten, die für bekannte Standardkonzentrationen des Liganden ermittelt wurden.

Verwendet man für den quantitativen Nachweis des Schrittes (b) Zellen, bei denen der zumindest unter bestimmten Bedingungen inaktive ras-Signaltransduktionsweg ein Signalweg ist, der auf den Zellzyklus einwirkt und dessen Aktivierung für die Zellvermehrung essenziell ist, erfolgt dieser auf einfache Weise, indem die Vermehrung der Zellen zu einem festgelegten Zeitpunkt oder die Vermehrungsrate der Zellen unter den genannten Bedingungen bestimmt wird. Die erhaltenen Daten werden dann mit anhand von Standardpräparationen bekannter Konzentration erhaltenen Daten verglichen und die Konzentration der Probe rechnerisch ermittelt.

Alternativ kann auch hier der quantitative Nachweis des Ausmaßes der ras-Signalwegsaktivierung anhand des Ausmaßes der Expression eines bevorzugt zu der Zelle heterologen Reportergens in der Zelle erfolgen. Wie vorstehend erläutert, kann dessen Expression nur aufgrund der infolge der Aktivierung des sich an ein Ras-Protein anschließenden Signalwegs bewirkten Aktivierung eines spezifischen Transkriptionsfaktors erfolgen. Bevorzugt erfolgt der Assay bei dieser Nachweisvariante unter Bedingungen, die eine Zellvermehrung ausschließen, beispielsweise durch Verwendung der cdc25-2-Hefemutante bei restriktiven Temperaturen, so daß die transkribierte Menge an Transkriptionsprodukt des Reportergens oder die exprimierte Menge an Reporterprotein zu einem bestimmten Zeitpunkt oder alternativ die Expressionsrate dieses Reportergens bzw. -proteins bei im wesentlichen gleichbleibender Zellzahl bestimmt werden kann. Jedoch kann die quantitative Bestimmung auch unter Proliferationsbedingungen erfolgen, wenn gleichzeitig fortlaufend oder in bestimmten Zeitabständen die Zellzahl bestimmt wird und die ermittelten werte für die Reportergenexpression in Werte pro Einheitswert der Zellzahl umgerechnet werden.

Alternativ kann auch hier ein quantitativer Nachweis über die Expression eines zu der Wirtszelle homologen Reportergens erfolgen, wobei hier die jeweils beobachtbare Zunahme der Expression des Reportergens gegenüber dem in Zellen ohne Aktivierung des ras-Signalwegs vorliegenden Expressionsniveau für die Ermittlung des Ergebnisses oder Werts aus jedem Einzeltest, der dann zur Ermittlung des Gesamtergebnisses, der Ligandenkonzentration, mit den Werten der übrigen Tests verglichen wird, herangezogen wird.

Ein weiterer alternativer *in vivo*-Assay ermöglicht den Nachweis, ob eine Verbindung in der Lage ist, eine Bindungsaktivität eines Ligandenbindungsabschnitts eines Rezeptors gegenüber einem Liganden zu verändern, also als Agonist, Antagonist oder Inhibitor zu wirken. Dieser Assay ist durch die folgenden Schritte gekennzeichnet:
(a) Inkontaktbringen des Liganden in Anwesenheit der Verbindung mit transformierten Hefezellen nach Anspruch 9 unter Bedingungen, bei denen bei Fehlen des heterologen Membranrezeptors der Ras- oder Ras-ähnliche Signalweg in den Zellen nicht aktiviert werden kann, wobei der heterologe Membranrezeptor den genannten Ligandenbindungsabschnitt enthält und das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors, abhängig ist, in der Lage ist, den inaktiven Ras- oder Ras-ähnlichen Signalweg zu aktivieren,
(b) Untersuchen, ob und gegebenenfalls in welchem Ausmaß eine Aktivierung des Ras- oder Ras-ähnlichen Signalweges erfolgt ist,
(c) Vergleichen des Untersuchungsergebnisses von Schritt (b) mit einem Untersuchungsergebnis, das bei Ausführen des Assay in Abwesenheit der Verbindung erhalten wird.
Dabei zeigt eine verstärkte Aktivierung des Ras-oder Ras-ähnlichen Signaltransduktionsweges in Anwesenheit der Verbindung eine Agonistenfunktion dieser Verbindung, eine verringerte oder gegebenenfalls auch vollständig fehlende Aktivierung dagegen eine Antagonisten- oder Inhibitorfunktion der Verbindung an.

Schritt (a) kann dabei umfassen, die Verbindung vor dem Liganden den Zellen zuzusetzen, wobei gegebenenfalls eine Vorinkubation der Verbindung mit den Zellen vorgenommen werden kann, die Verbindung separat, aber gleichzeitig mit dem Liganden den Assayzellen zuzusetzen oder die Verbindung vorab mit dem Liganden zu mischen und gegebenenfalls eine Vorinkubation der beiden Verbindungen durchzuführen und erst dann die Mischung den Assayzelle zuzusetzen.

Handelt es sich bei der Verbindung um ein Peptid, Polypeptid oder Protein, so kann die Verbindung auch durch Expression eines dafür kodierenden Gens innerhalb der Zelle selbst hergestellt werden. Zu diesem Zweck können die Zellen mit einem Expressionsvektor, der ein solches Gen enthält, transformiert oder transfiziert werden. Die hierfür erforderlichen Mittel und Methoden sind dem Fachmann wohlbekannt.

Wird die auf ihre Agonisten- oder Antagonistenwirkung zu testende Verbindung in der Assayzelle exprimiert, so erfolgt die Expression des dafür kodierenden Gens vorzugsweise unter Kontrolle eines konstitutiv aktiven Promotors sowie unter Verwendung von Zellen, in denen der Ras- oder Ras-ähnliche Signaltransduktionsweg nur unter den speziellen Assaybedingungen inaktiviert wird. Ein solches System ermöglicht es, auszuschließen, daß allein die Expression der Verbindung in der Zelle Veränderungen hervorruft, die das Ergebnis des Assay verfälschen könnten. Für diesen Ausschluß ist erforderlich, die Aktivität des unter restriktiven Bedingungen inaktivierten zelleigenen ras-Signaltransduktionsweges unter nicht-restriktiven Bedingungen und in Abwesenheit von Ligand nachzuweisen. Wird unter nichtrestriktiven Bedingungen und in Abwesenheit von Ligand gearbeitet, liegt der Membranrezeptor in einem inaktiven Zustand vor, so daß die nachweisbare Aktivierung des ras-Signaltransduktionsweges anzeigt, daß das Expressionsprodukt mit keiner Komponente des Ras- oder Ras-ähnlichen Signaltransduktionswegs, und insbesondere nicht mit dem dafür spezifischen Ras-Protein oder Guaninnukleotid-Austauschfaktor, interferiert. Auf diese Weise kann ausgeschlossen oder die Wahrscheinlichkeit minimiert werden, daß das Expressionsprodukt anstatt mit dem Ligandenbindungsabschnitt des Membranrezeptors mit dem Effektorprotein oder einem für die Bindung des Effektorproteins an die Membrankomponente erforderlichen Adaptorprotein wechselwirkt, so daß deren Fähigkeit zur Aktivierung des Ras- oder Ras-ähnlichen Signaltransduktionsweges beseitigt wird.

Ein entsprechender Test auf Aktivierung des jeweiligen Ras-oder Ras-ähnlichen Signaltransduktionswegs unter nicht-restriktiven Bedingungen, in Anwesenheit der Verbindung und in Abwesenheit von Ligand wird analog bei einer Verbindung, die den Assayzellen von außen zugesetzt wird, vorgenommen.

Handelt es sich bei dem unter den Assaybedingungen inaktivierbaren Ras- oder Ras-ähnlichen Signaltransduktionsweg um einen, dessen Aktivierung für die Zellvermehrung essenziell ist, so wird unter nicht-restriktiven Bedingungen einfach die normale Vermehrbarkeit der Zellen sichergestellt. Erfolgt der Nachweis unter Nutzung einer Reportergenaktivität, so ist ein Nachweis dieser Reportergenaktivität unter nicht-restriktiven Bedingungen erforderlich.

Für den Nachweis unter den restriktiven Bedingungen des Assay in Schritt (b) besteht ebenfalls beispielsweise die Möglichkeit, die Aktivierung des sich an ein Ras-Protein anschließenden Signalweges über die gegebenenfalls erfolgende Expression eines zu den Zellen heterologen Reportergens, die nur aufgrund der infolge der Aktivierung des sich an ein Ras-Protein anschließenden Signalweges erfolgenden Aktivierung eines spezifischen Transkriptionsfaktors erfolgt, nachzuweisen. Der bei Nachweis von Aktivierung des Ras- oder Ras-ähnlichen Signalweges erfolgende Nachweis des Ausmaßes dieser Aktivierung, kann eine quantitative Bestimmung umfassen, bei welcher die in den Zellen vorliegende Menge an Transkriptions- oder Translationsprodukt (Reporterprotein) des Reportergens zu einem bestimmten Zeitpunkt oder die Transkriptionsrate des Reportergens oder die Expressionsrate des Reporterproteins unter den genannten Bedingungen bestimmt wird.

Alternativ kann auch nur eine Analyse von Aliquots, d.h. gleichen Volumina der bis auf den Zusatz der Verbindung identisch erzeugten und behandelten Assaylösungen, bevorzugt unter Sicherstellen gleicher oder im wesentlichen gleicher Zellzahlen in diesen Aliquots, vorgenommen werden. In diesem Falle erfolgt keine absolute Quantifizierung des Expressionsniveaus des Reportergens, sondern es wird nur ein relativer Vergleich der Expressionsniveaus in beiden Assays ermöglicht.

In dem Falle, wo der Vergleich in Schritt (c) ergibt, daß in Anwesenheit der Verbindung eine stärkere Expression des Reportergens auftritt, ist eine Agonistenwirkung der Verbindung und in dem Falle, wo der Vergleich in Schritt (c) ergibt, daß in Anwesenheit der Verbindung eine geringere Expression des Reportergens auftritt, eine Antagonistenwirkung der Verbindung anzunehmen. Wie der vorstehend beschriebene nicht erfindungsgemäße quantitative Assay wird auch dieser Assay bevorzugt unter Bedingungen durchgeführt wird, bei denen keine Vermehrung der Zellen auftritt.

Alternativ kann das für den Nachweis eingesetzte Reportergen auch hier zu der Assayzelle homolog sein, wobei hier die jeweils beobachtbare Zunahme der Expression, d.h. Transkription und/oder Translation, des Reportergens gegenüber dem in Zellen ohne Aktivierung des Ras- oder Ras-ähnlichen Signalwegs vorliegenden Expressionsniveau für die Ermittlung des Ergebnisses herangezogen wird.

Werden für den Assay Zellen eingesetzt, bei denen der inaktive, sich an ein Ras-Protein anschließende Signalweg ein Signalweg ist, der auf den Zellzyklus einwirkt und dessen Aktivierung für die Zellvermehrung essenziell ist, umfaßt Schritt (b), zu untersuchen, ob und gegebenenfalls in welchem Ausmaß die Zellen unter den genannten Bedingungen vermehrungsfähig sind. In dem Falle, wo der Vergleich in Schritt (c) ergibt, daß in Anwesenheit der Verbindung eine stärkere Zellvermehrung auftritt, ist auf eine Agonistenwirkung der Verbindung und in dem Falle, wo der Vergleich in Schritt (c) ergibt, daß in Anwesenheit der Verbindung eine geringere Zellvermehrung auftritt, auf eine Antagonistenwirkung der Verbindung zu schließen.

Wie sämtliche Assays im Rahmen dieser Anmeldung eignet sich auch dieser Assay insbesondere für ein Massenscreening, hier auf Agonisten- und Antagonistenverbindungen für Rezeptoren.

Alternativ kann der Assay auch als zusätzlicher Test zur Bestätigung der Ligandenbindungseigenschaft eines neuen, insbesondere synthetischen Ligandenbindungsabschnitts oder zur Bestätigung der Eignung einer Testsubstanz als Ligand für den Ligandenbindungsabschnitt herangezogen werden. Liegt eine Ligandenbindungseigenschaft des Ligandenbindungsabschnitts vor bzw. besteht eine Eignung als Ligand für den Ligandenbindungsabschnitt, so müßte bei Verwendung eines bekannten Agonisten für den zum ersten Nachweis der Ligandeneigenschaft eingesetzten Liganden bzw. für den Ligandenbindungsabschnitt eine verstärkte Aktivierung des Ras- oder Ras-ähnlichen Signalweges beobachtet werden, und bei Verwendung eines bekannten Antagonisten für den Liganden bzw. den Ligandenbindungsabschnitt im Gegenzug eine verringerte Aktivierung des Ras- oder Ras-ähnlichen Signalweges.

Ein weiterer alternativer *in vivo*-Assay ermöglicht den Nachweis, ob ein Polypeptid oder Protein, bei dem eine Ligandenbindungsfunktion eines Rezeptors vermutet wird, diese auch tatsächlich aufweist. Dieser Assay umfaßt die folgenden Schritte:
(a) Inkontaktbringen von transformierten Hefezellen nach Anspruch 9 mit dem Liganden unter Bedingungen, bei denen bei Fehlen des heterologen Membranrezeptors, wie in Anspruch 1 definiert, ein Ras- oder Ras-ähnlicher Signalweg in den Zellen nicht aktiviert werden kann, wobei der Ligandenbindungsabschnitt des heterologen Membranrezeptors das zu untersuchende Polypeptid oder Protein umfasst oder daraus besteht und wobei das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors abhängig ist, in der Lage ist, diesen Ras- oder Ras-ähnlichen Signalweg zu aktivieren,
(b) Untersuchen, ob eine Aktivierung des Ras- oder Ras-ähnlichen Signalwegs erfolgt ist,
wobei für den Fall, dass die Bindung des Effektorproteins oder -polypeptids von der Bindung eines Liganden an den Ligandenbindungsabschnitt des heterologen Membranrezeptors abhängig ist, ein Nachweis der Aktivierung des Ras- oder Ras-ähnlichen Signalweges anzeigt, dass der Ligandenbindungsabschnitt des heterologen Membranrezeptors und dementsprechend das zu untersuchende Polypeptid oder Protein eine Ligandenbindungsfunktion eines Rezeptors aufweist und für den Fall, dass die Bindung des Effektorproteins oder -polypeptids von der fehlenden Bindung eines Liganden an den Ligandenbindungsabschnitt des heterologen Membranrezeptors abhängig ist, Zellen, wie sie in Schritt (a) eingesetzt wurden, unter Bedingungen, bei denen bei Fehlen des heterologen Membranrezeptors der Ras- oder Ras-ähnliche Signalweg in der transformierten Hefezelle nicht aktiviert werden kann, in Abwesenheit von Ligand auf Aktivierung des Ras- oder Ras-ähnlichen Signalwegs untersucht werden, wobei ein Nachweis der Aktivierung des Ras- oder Ras-ähnlichen Signalwegs in Abwesenheit des Liganden und die Inaktivität des Ras- oder Ras-ähnlichen Signalwegs in Anwesenheit des Liganden anzeigt, dass der Ligandenbindungsabschnitt des heterologen Membranrezeptors und dementsprechend das zu untersuchende Polypeptid oder Protein eine Ligandenbindungsfunktion eines Rezeptors aufweist.
Bin Nachweis der Aktivierung des Ras- oder Ras-ähnlichen Signalweges zeigt an, daß der Ligandenbindungsabschnitt des Membranrezeptors und dementsprechend das zu untersuchende Polypeptid oder Protein eine Ligandenbindungsfunktion eines Rezeptors aufweist.

Beispielsweise kann der in den Zellen enthaltene Membranrezeptor einen Ligandenbindungsabschnitt umfassen, der einen von einem Ligandenbindungsabschnitt eines natürlich vorkommenden Rezeptors durch Mutation abgeleiteten Ligandenbindungsabschnitt enthält oder daraus besteht.

Wie zuvor, kann die Aktivierung des Ras- oder Ras-ähnlichen Signaltransduktionsweges bei Verwendung von Zellen, bei denen der zumindest unter bestimmten Bedingungen inaktive Ras- oder Ras-ähnliche Signaltransduktionsweg ein Signalweg ist, der auf den Zellzyklus einwirkt und dessen Aktivierung für die Zellvermehrung essenziell ist, anhand einer gegebenenfalls erfolgenden Zellvermehrung nachgewiesen werden.

Alternativ kann der Nachweis der Aktivierung des Ras- oder Ras-ähnlichen Signaltransduktionsweges auch anhand der gegebenenfalls feststellbaren Expression eines Reportergens in den Zellen erfolgen. Wie erläutert, erfolgt dessen Expression, wenn es sich um ein heterologes Reportergen handelt, nur aufgrund der infolge der Aktivierung des Ras- oder Ras-ähnlichen Signalweges erfolgenden Aktivierung eines spezifischen Transkriptionsfaktors. Im Falle eines homologen Reportergens wird auf den Nachweis der Erhöhung der Reportergenexpression, z.B. aufgrund höherer Mengen an Transkriptions- oder Translationsprodukt, im Vergleich zu dem Expressionsniveau ohne Aktivierung des spezifischen Ras-Signaltransduktionswegs abgestellt.

Die unter Nutzung der zweiten Variante, d.h. nur bei fehlender Ligandenbindung an den Ligandenbindungsabschnitt des erfindungsgemäß untersuchten Membranrezeptors möglicher Effektorprotein- bzw. -polypeptidtranslokation an die Zellmembran, realisierbaren, im folgenden näher erläuterten Assayverfahren können u.a. dazu genutzt werden,
1. die Eignung einer Testsubstanz als Ligand für einen Rezeptor zu bestimmen, und in diesem Falle insbesondere Massenscreens mit Ligandenderivaten durchzuführen, um zu testen, welche Derivate in der Lage sind, an den wildtypischen Ligandenbindungsabschnitt des Rezeptors zu binden,
2. die Anwesenheit eines bestimmten Liganden in einer Probe zu detektieren,
3. nachzuweisen, ob eine Verbindung in der Lage ist, eine Bindungsaktivität eines Ligandenbindungsabschnitts eines Rezeptors gegenüber einem Liganden zu verändern, also als Agonist, Antagonist oder Inhibitor zu wirken, und in diesem Falle insbesondere Massenscreens zum Auffinden solcher Agonisten- oder Antagonistenverbindungen auszuführen; und
4. die Ligandenbindungsfunktion eines Polypeptids oder Proteins, bei denen eine solche Funktion vermutet wird, für Liganden bestimmter Rezeptoren nachzuweisen; auch hier kann es sich bei den Polypeptiden oder Proteinen insbesondere um durch Mutation von natürlichen Rezeptoren abgeleitete neuartige Ligandenbindungsabschnitte von Rezeptoren handeln, deren Ligandenbindungsfunktion noch bestätigt werden muß; in diesem Zusammenhang können insbesondere Massenscreens mit derartigen neuartigen, mutierten Ligandenbindungsabschnitten, die beispielsweise in Form einer Rezeptormutantenbibliothek, die insbesondere Rezeptormutanten mit zufallsbedingt lokalisierten Mutationen im Ligandenbindungsabschnitt enthält, durchgeführt werden, um neue künstliche, funktionelle Liganden-Rezeptor-Partner zu finden.

Die vorstehend genannten Assays können im wesentlichen analog zu den im Vorangehenden erläuterten Assayvarianten unter Nutzung der ersten Variante erfolgen, wobei jedoch in diesem Falle der Nachweis der Aktivierung des Ras- oder Ras-ähnlichen Signalwegs die Abwesenheit eines Liganden für den Ligandenbindungsabschnitt des untersuchten Membranrezeptors in der Assayzelle anzeigt. Die letztgenannten Assays werden demzufolge in der Regel zwei Nachweise umfassen, und zwar einen Nachweis in Anwesenheit von (potentiellem) Ligand, wobei bei Ligandenbindungseigenschaft des (potentiellen) Liganden für den Ligandenbindungsabschnitt des untersuchten Membranrezeptor keine Aktivierung des Ras- oder Ras-ähnlichen Signalwegs nachzuweisen sein wird, sowie einen weiteren Nachweis in Abwesenheit des (potentiellen) Liganden, wobei eine derartige Aktivierung regelmäßig nachgewiesen werden wird.

Bei dem Nachweis, ob eine Verbindung in der Lage ist, eine Bindungsaktivität eines Ligandenbindungsabschnitts eines Rezeptors gegenüber einem Liganden zu verändern, also als Agonist, Antagonist oder Inhibitor zu wirken, erfolgt der Nachweis bei gleichzeitiger Anwesenheit von Ligand und Verbindung insbesondere über
a) die bei Antagonisten- oder Inhibitorwirkung der Verbindung möglicherweise doch nachweisbare Aktivierung des Ras- oder Ras-ähnlichen Signalwegs, beispielsweise mit anschließender Bestimmung der für eine vollständige Inaktivität des Ras- oder Ras-ähnlichen Signalwegs erforderlichen Ligandenkonzentration bei einer bestimmten Konzentration an Verbindung oder einer Bestimmung der Abhängigkeit der Signalwegs-Aktivierung von der Konzentration an Verbindung bei einer bestimmten Ligandenkonzentration; oder
b) die bei Agonistenwirkung der Verbindung bereits bei einer geringeren Ligandenkonzentration vollständigen Inaktivität des Ras- oder Ras-ähnlichen Signalwegs; auch hier kann die Abhängigkeit der vollständigen Inaktivität des Ras- oder Ras-ähnlichen Signalwegs von der Konzentration der Verbindung und/oder des Liganden im Einzelnen bestimmt werden.

Bei dem Nachweis einer Ligandenbindungsfunktion eines Polypeptids oder Proteins, bei denen eine solche Funktion vermutet wird, für Liganden bestimmter Rezeptoren erfolgt der Nachweis der Ligandenbindungsfunktion analog über die Inaktivität des Ras- oder Ras-ähnlichen Signalwegs in Anwesenheit von Ligand bei Aktivität dieses Signalwegs in Abwesenheit von Ligand.

Die Detektion von Rezeptor-Liganden-Interaktionen mittels der Assayverfahren, Zellen und heterologen Fusionsproteine erfolgt in Hefezellen wie z.b. in zellwandlosen Hefen, sogenannten "ghost"-Formen von Hefen.

Je nach eingesetztem Membranrezeptor und insbesondere je nach dem durch den Mediatorabschnitt des Membranrezeptors bedingten Mechanismus, der schließlich zu einer Translokation des Effektorproteins oder -polypeptids an die Membran führt, kann es bei einigen Ausführungsformen der Anmeldung, wie bereits weiter oben angesprochen, zusätzlich erforderlich werden, in der Assayzelle weitere Voraussetzungen vorzusehen, die sicherstellen, daß das nachweisbare Translokationsereignis ausschließlich bei Wechselwirkung eines Liganden oder, alternativ, bei fehlender Wechselwirkung eines Liganden mit dem Ligandenbindungsabschnitt dieses Membranrezeptors auftritt. Eine Assoziierung des Effektorproteins oder -polypeptids mit membranständigen Zellkomponenten, die (in Abwesenheit des Membranrezeptors und der mit diesem bei der Signaltransduktion spezifisch und ausschließlich zusammenwirkenden Zellkomponenten) natürlicherweise in diesen Zellen vorkommen und zwar gleich welchen Aktivierungs- und Modifizierungszustandes, muß ausgeschlossen sein. Dies setzt voraus, daß alle Komponenten, die an der Translokation des Effektorproteins oder -polypeptids an die Zellmembran direkt und indirekt beteiligt sind, nur in Folge der Aktivierung des speziell zu testenden Membranrezeptors aufgrund der Bindung oder, alternativ, fehlenden Bindung eines Liganden an den Ligandenbindungsabschnitt, und genauer nur in Folge der durch diese Aktivierung bedingten Strukturänderung des Mediatorabschnitts in die Lage versetzt werden können, die Bindung des Effektorproteins oder -polypeptids an eine Komponente der Zellmembran zu vermitteln oder zu bewirken.

Soll in einem Test ein in einer Zelle natürlicherweise vorkommender Membranrezeptor getestet werden, muß sichergestellt werden, daß in der Zelle der Mediatorabschnitt dieses Membranrezeptors nur in Zusammenhang mit diesem vorkommt und daß die damit zusammenwirkenden Adaptor- und Vermittlerproteine nur mit diesem Mediatorabschnitt oder nur aufgrund einer Aktivierung dieses Mediatorabschnitts in einer Weise interagieren können, daß es schließlich zu einer Translokation des Effektorproteins oder -polypeptids an die Zellmembran kommt.

Erfindungsgemäß wird eine Hefezelle als Assayzelle ausgewählt, in der alle diese Komponenten vor der Einschleusung der genetischen Information für den Membranrezeptor und die gegebenenfalls benötigten Adaptor-, Vermittler- und/oder Effektorproteine bzw. -polypeptide natürlicherweise nicht vorkommen, also zu dieser Ausgangszelle heterolog sind und auch keine zelleigenen Komponenten gleicher Spezifität und ggf. Aktivität, die diese ersetzten könnten, vorliegen; bezüglich des Membranrezeptors kann dies auch nur für den Mediatorabschnitt gelten. Zusammenfassend erläutert, betrifft dies zumindest die folgenden Komponenten:
- den Mediatorabschnitt,
- ggf. die damit zusammenwirkenden Adaptorproteine oder Adaptorproteinabschnitte, sofern eine Bindung des Effektorproteins oder -polypeptids an diese oder über diese erfolgt, sowie,
- z.B. im Falle der vorliegend erläuterten Alternative 1)c), ggf. eine Vermittlerkomponente, welche mit dem Mediatorabschnitt des Membranrezeptor räumlich nicht unmittelbar assoziiert sein muß, jedoch im Falle einer Ligandenbindung oder, alternativ, *fehlenden* Ligandenbindung an den Ligandenbindungsabschnitt von diesem aktiviert oder modifiziert wird und aufgrunddessen ein sekundäres Ereignis an einer weiteren Komponente der Zellmembran vermittelt, infolgedessen sich das Effektorprotein oder -polypeptid spezifisch an diese Komponente der Zellmembran, die mit dem Mediatorabschnitt des Membranrezeptors nicht assoziiert ist, bindet; alternativ darf das genannte sekundäre Ereignis in der Zelle nur bei Ligandenbindung oder, alternativ, *fehlender* Ligandenbindung des Ligandenbindungsabschnitts des Membranrezeptors vermittelt werden. Es dürfen im letzteren Falle in der Zelle also keine Wechselwirkungspartner für diese Vermittler vorliegen, die eine entsprechende Aktivierungswirkung wie der Membranrezeptor auf den oder die Vermittler ausüben können.
Verwendet man folglich ein Zellsystem, in dem der zu testende Membranrezeptor natürlicherweise nicht vorkommt, kann es erforderlich werden, neben dem Membranrezeptor weitere Proteine in diesem Zellsystem zu exprimieren, und zwar gegebenenfalls insbesondere Adaptor- und/oder Vermittlerproteine oder -polypeptide.

Bezüglich der Assaybedingungen sind keine bestimmten allgemeinen Vorgaben erforderlich. Im Falle eines Nachweises der gegebenenfalls erfolgenden Zellvermehrung ist jedoch darauf zu achten, daß das verwendete Medium eine solche grundsätzlich ermöglicht. Sofern in den Zellen Gene und/oder Promotoren, wie erläutert, durch Wahl bestimmter Assaybedingungen inaktiviert werden können und im Zuge des Assays auch inaktiviert werden sollen, sind diese Bedingungen, wie z.B. eine bestimmte restriktive Assaytemperatur, bei cdc25-2-Zellen z.B. 33 - 37°C, während des Assay einzuhalten. Das gewählte Reaktionsmedium sollte ferner nicht mit der Testverbindung oder dem Liganden, die diesem zugesetzt werden, auf eine Weise wechselwirken, daß der Assay beeinträchtigt wird.

Als Liganden können in sämtlichen vorstehend erläuterten Assayverfahren natürliche vorkommende Substanzen, wie Geruchsstoffe, Geschmacksstoffe, Peptide, Peptidhormone und Proteine, insbesondere Zytokine, Neurotransmitter, nicht-protein- oder -peptidartige Hormone, insbesondere Steroidhormone, Vitamine, z.B. Vitamin D, Thyroxin oder Retinsäure, wie auch in der Natur nicht vorkommende Substanzen, z.B. synthetische Derivate natürlicher Liganden oder Toxine/Giftstoffe, wie Dioxin, untersucht bzw. bestimmt werden.

In der Mehrzahl der Fälle wird der Ligandenbindungsabschnitt des Membranrezeptors extrazellulär lokalisiert sein. Bei Einsatz von Ligandenbindungsabschnitten insbesondere nukleärer Rezeptoren besteht jedoch auch die Möglichkeit, daß diese intrazellulär lokalisiert sind. Da die Liganden nukleärer Rezeptoren überwiegend kleine Moleküle mit geringer relativer Molekülmasse und von überwiegend hydrophober Natur darstellen, diffundieren diese ohne weitere Maßnahmen in die Assayzellen, um dort mit einem intrazellulär und unmittelbar an der Zellmembran lokalisierten Ligandenbindungsabschnitt des Membranrezeptors eine Bindung einzugehen. Sofern gewünscht und/oder erforderlich, können die Zellen jedoch vor dem Assay in geeigneter Weise vorbehandelt werden, um die äußere Zellmembran für den Durchtritt der Testverbindung oder des Liganden durchlässiger zu machen. Ein Beispiel hierfür ist die Herstellung von Zell-"ghosts" durch z.B. enzymatische Behandlung der Zellen. Derartige wie auch auf andere Weise hergestellte Zell-"ghosts" sowie Zellen mit zur Erhöhung der Permeabilität anderweitig modifizierter Zellwand werden im vorliegenden Zusammenhang von dem Begriff "Zellen" mit umfaßt.

Handelt es sich bei den zu testenden Liganden um Peptide, Polypeptide oder Proteine, so können diese auch in der Assayzelle ausgehend von in die Assayzelle eingeschleusten Nukleinsäurekonstrukten, die diese kodieren, exprimiert werden, in einer speziellen Variante auch nicht konstitutiv, sondern unter Kontrolle eines induzierbaren Promotors; dabei können die Konstrukte nach Einschleusen in die Assayzelle chromosomal oder extrachromosomal, d.h. als Bestandteil eines Episoms, z.B. Plasmids, vorliegen. Das Inkontaktbringen der Assayzelle mit dem Liganden erfolgt dementsprechend im Falle einer nicht-konstitutiven Expression unter Bedingungen, bei denen die Expression des zu testenden Liganden in der Zelle induziert wird. Dem Fachmann sind zu diesem Zweck eine Vielzahl von induzierbaren Promotoren, die beispielsweise durch bestimmte Temperaturen oder chemische Verbindungen induzierbar sind, bekannt. Bei Einsatz von Membranrezeptoren mit extrazellulär lokalisierter Ligandenbindungsdomäne ist zusätzlich dafür Sorge zu tragen, daß in der Assayzelle exprimierte Liganden auch aus den Assayzellen in den Extrazellulärraum sezerniert werden. Auch hierfür sind dem Fachmann diverse Möglichkeiten bekannt.

Allgemein ist festzuhalten, daß bei Hinzusetzen des zu testenden Liganden zu der Assayzelle von außen alle in dem Assaysystem zusammenwirkenden Komponenten, d.h. insbesondere der zu untersuchende heterologe Membranrezeptor, wie in Anspruch 1 definiert, das Effektorprotein- oder -polypeptid, etwaige Adaptor- und gegebenenfalls Vermittlerproteine oder -polypeptide sowie jegliche Komponenten des spezifisch durch das Effektorprotein oder -polypeptid bei dessen Bindung an eine Komponente der Membran aktivierten, sich an ein Ras-Protein anschließenden Signalwegs, die nur an diesem spezifischen Signalweg beteiligt sind, in der Assayzelle konstitutiv exprimiert werden können. Bei einer Expression des zu testenden Liganden in der Assayzelle können bei der ersten Variante, in welcher eine Effektorprotein- oder -polypeptidtranslokation an die Membran nur bei Bindung von Ligand an den Ligandenbindungsabschnitt des untersuchten Membranrezeptors nachweisbar ist, alle Komponenten des Assaysystems, nun einschließlich des Liganden, bis auf eine in der Assayzelle konstitutiv exprimiert werden. Die Assaysystem-Komponente(n), deren Gen(e) unter Kontrolle eines induzierbaren Promotors vorgesehen wird bzw. werden, wird bzw. werden dann insbesondere nur unter Assaybedingungen, d.h. bei Untersuchung der möglicherweise erfolgten Aktivierung des ras-Signaltransduktionsweges, aufgrund der gezielten Induktion des/der jeweils eingesetzten induzierbaren Promotors bzw. Promotoren exprimiert. Bei der zweiten Variante, bei welcher die Effektorprotein- oder -polypeptidtranslokation an die Membran nur bei fehlender Ligandenbindung an den Membranrezeptor auftritt, wird bei Expression des Liganden in der Assayzelle dessen Gen stets unter Kontrolle eines induzierbaren Promotors vorgesehen werden, um einen Nachweis auch in Abwesenheit des Liganden zu ermöglichen.

Der Nachweis der Aktivierung des Ras- oder Ras-ähnlichen Signaltransduktionswegs erfolgt je nach Nachweisstrategie auf dem Fachmann geläufige Weise. Befinden sich die Zellen während des Assay immobilisiert an einem festen Träger, so kann es insbesondere bei Nachweis einer Reportergenaktivität oder eines Reporterproteins erforderlich oder hilfreich sein, die Zellen vor der Nachweisreaktion zu solubilisieren, d.h. sie von dem Träger abzulösen und ggf. auch aufzubrechen. Auch die hierfür erforderlichen Maßnahmen und Reagenzien sind dem Fachmann wohlbekannt.

Die Anmeldung stellt darüber hinaus Kits zur Verwendung in den verstehend beschriebenen Assays bereit, welche es z.B. ermöglichen, schnell und effizient zu bestimmen, ob ein spezifischer Ligand in der Lage ist, an einen bestimmten Rezeptor und genauer an dessen Ligandenbindungsabschnitt zu binden.

Ein erster Kit zur Verwendung in den Assayverfahren zur Bestimmung der Eignung einer Testsubstanz als Ligand für einen Ligandenbindungsabschnitt eines Rezeptors, zur Bestimmung der Anwesenheit eines Liganden für einen Ligandenbindungsabschnitt eines Rezeptors in einer Probe, zur Konzentrationsbestimmung eines solchen Liganden sowie zur Charakterisierung von Verbindungen als mögliche Agonisten oder Antagonisten bezüglich Rezeptor-Liganden-Wechselwirkungen, welcher nicht Gegenstand der Erfindung ist, umfaßt jeweils Zellen mit den vorstehend bei den Assayverfahren im Einzelnen erläuterten Eigenschaften. So enthalten die Hefezellen des Kits beispielsweise bei einem vorgesehenen Nachweis einer Reportergenaktivität zusätzlich ein Konstrukt mit einer Bindungsstelle für den Transkriptionsfaktor, der spezifisch durch den Ras- oder Ras-ähnlichen Signalweg, dessen Aktivierung mittels der Assays nachgewiesen werden soll, aktiviert wird, einem Minimalpromotor und dem Reportergen. Alternativ kann der Kit wie auch alle folgenden einen Transformations- bzw. Transfektionsvektor, der das Konstrukt enthält, umfassen. Auf diese Weise kann der Verwender des Kits bei Wahl dieses Nachweisweges die im Kit enthaltenen Assayzellen durch Transformation oder Transfektion mit diesem Konstrukt ausstatten. In einer weiteren Ausführungsform dieses und aller folgenden Kits enthält der separat im Kit bereitgestellte Transformations-bzw. Transfektionsvektor nur die Bindungsstelle für den Transkriptionsfaktor und den damit funktional verknüpften Minimalpromotor sowie eine geeignet vorgesehene Insertionsstelle für die Insertion eines durch den Verwender frei wählbaren Reportergens.

Darüber hinaus kann dieser Kit wie auch alle folgenden unter anderem gegebenenfalls auch Assaypuffer, Reagenzien zur Detektion der phänotypischen Aktivierung des Ras- oder Ras-ähnlichen Signaltransduktionsweges in diesen Zellen und/oder eine Gebrauchsanweisung enthalten.

Ein alternativer Kit für die vorstehend genannten Assayverfahren, welcher nicht Gegenstand der Erfindung ist, umfaßt mindestens zwei der folgenden Komponenten:
(a) Zellen, in denen zumindest unter bestimmten Bedingungen ein Ras- oder Ras-ähnlicher Signalweg nicht aktiviert werden kann;
(b) einen Nukleinsäurevektor, in den eine DNA-Sequenz exprimierbar eingefügt ist, die einen Membranrezeptor, wie vorstehend definiert, kodiert, wobei das Effektorprotein oder -polypeptid, dessen Bindung an eine Membrankomponente von der Bindung oder, alternativ, *fehlenden* Bindung eines Liganden an den Ligandenbindungsabschnitt des Membranrezeptors abhängig ist, in der Lage ist, den inaktiven Ras- oder Ras-ähnlichen Signalweg in den unter (a) genannten Zellen zu aktivieren;
(c) einen Nukleinsäurevektor, in den eine DNA-Sequenz exprimierbar eingefügt ist, die das Effektorprotein oder -polypeptid, das im Falle einer Ligandenbindung oder, alternativ, *fehlenden* Ligandenbindung an den Ligandenbindungsabschnitt des Membranrezeptors an eine Komponente der Membran, gegebenenfalls über weitere Proteine oder Polypeptide, binden kann, kodiert;
(d) einen Nukleinsäurevektor, in den eine DNA-Sequenz exprimierbar eingefügt ist, die mindestens ein Adaptorprotein, über welches das Effektorprotein oder -polypeptid bei Bindung oder, alternativ, *fehlender* Bindung eines Liganden an den Ligandenbindungaabschnitt des Membranrezeptors an eine Komponente der Membran binden kann, kodiert.

Ein weiterer Kit für die vorstehend genannten Assays ermöglicht bei bestimmter Auswahl der nachfolgend aufgeführten Komponenten insbesondere die Herstellung der Assayzelle mit einem Membranrezeptor, der einen individuell gewünschte Ligandenbindungsabschnitt enthält. Der Kit umfaßt die folgenden Komponenten: (a), (b) und (c) :
(a) Hefezellen in denen zumindest unter bestimmten Bedingungen ein Ras- oder Ras-ähnlicher Signalweg nicht aktiviert werden kann;
(b) einen Nukleinsäurevektor, der in geeigneter Anordnung umfaßt:
   - einen DNA-Abschnitt, der ein Membranlokalisierungssignal eines heterologen Membranrezeptors, wie in Anspruch 1 definiert, kodiert;
   - einen DNA-Abschnitt, der einen Mediatorabschnitt eines heterologen Membranrezeptors, wie in Anspruch 1 definiert, kodiert; und
   - eine geeignet angeordnete Insertionsstelle zur funktionalen Insertion einer DNA-Sequenz, die einen Ligandenbindungsabschnitt, wie in Anspruch 1 definiert, kodiert,
   wobei nach Insertion einer DNA-Sequenz für den Ligandenbindungsabschnitt der Nukleinsäurevektor ein vollständiges exprimierbares Gen für einen heterologen Membranrezeptor, wie in Anspruch 1 definiert, umfaßt, wobei das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors abhängig ist, in der Lage ist, den inaktiven Ras- oder Ras-ähnlichen Signalweg in den unter (a) genannten Hefezellen zu aktivieren;
(c) einen Nukleinsäurevektor, in den eine DNA-Sequenz exprimierbar eingefügt ist, die das Effektorprotein oder -polypeptid, das im Falle einer Ligandenbindung oder, alternativ, *fehlenden* Ligandenbindung an den Ligandenbindungsabschnitt des heterologen Membranrezeptors an eine Komponente der Membran, gegebenenfalls über weitere Proteine oder Polypeptide, binden kann, und das in Form eines heterologen Fusionproteins eines Effectorabschritts mit einem Adaptorprotein oder -polypeptid, das die Bindung and die Komponente der Membran, gegebenenfalls über weitere Proteine oder Polypeptide (Adaptoren), ermöglicht, vorliegt kodiert.

In einer bevorzugten Ausführungsform umfasst der Kit als komponent (d) zusätzlich einen Nukleinsäurevektor, in den eine DNA-Sequenz exprimierbar eingefügt ist, die mindestens ein Adaptorprotein, über welches das Effektorprotein oder -polypeptid bei Bindung oder, alternativ, fehlender Bindung eines Liganden an den Ligandenbindungsabschnitt des heterologen Membrahrezeptors an eine Komponente der Membran binden kann, kodiert.

In einer weiteren speziellen Ausführungsform der Komponente (c) der beiden letztgenannten Kits umfaßt der darin enthaltene Nukleinsäurevektor eine DNA-Sequenz, die ein Effektorprotein oder -polypeptid in Form eines Fusionsproteins aus einem Effek- , torabschnitt und einem Antikörper- oder Bindungsproteinabschnitt mit spezifischer Bindungsaffinität für eine an dem Membranrezeptor befindliche "Tag"- oder Epitopstruktur kodiert, die nur aufgrund von Konformationsänderungen, ggf. in Kombination mit enzymatischer Aktivität, infolge einer Bindung von Ligand oder, alternativ, fehlender Bindung von Ligand an den Ligandenbindungsabschnitt des Membranrezeptors für den Antikörper bzw. das Bindungsprotein zugänglich wird.

Außerdem gilt auch für die beiden letztgenannten Assays, daß die in dem Kit enthaltenen Zellen bei einem vorgesehenen Nachweis einer Reportergenaktivität u.a. zusätzlich auch ein Konstrukt, umfassend eine Bindungsstelle für einen Transkriptionsfaktor, dessen Aktivierung infolge der Aktivierung des speziellen Ras- oder Ras-ähnlichen Signalwegs, dessen Aktivierung durch den Assay nachgewiesen werden soll, erfolgt, einen Minimalpromotor und das Reportergen, wie vorstehend erläutert, enthalten können, oder es kann alternativ getrennt von den Zellen ein Transformations- oder Transfektionsvektor mit dem Transkriptionsfaktorbindungsstelle-Minimalpromotor-Reportergen-Konstrukt oder mit einem andersartigen Konstrukt, umfassend die Transkriptionsfaktorbindungsstelle und den Minimalpromotor und darüber hinaus eine geeignet angeordneten Insertionsstelle für ein frei wählbares Reportergen, vorgesehen werden.

Durch die Anmeldung werden auch Kits für Assayverfahren zum Nachweis, ob ein Polypeptid oder Protein eine Ligandenbindungsfunktion eines Rezeptors aufweist, bereitgestellt.

Ein hierfür geeigneter Kit umfaßt Hefezellen, wobei der darin enthaltene heterologe Membranrezeptor einen Ligandenbindungsabschnitt, umfassend ein oder bestehend aus einem Polypeptid oder Protein, bei dem eine Ligandenbindungsfunktion eines Rezeptors vermutet wird, umfaßt.

Ein alternativer Kit, welcher nicht Gegenstand der Erfindung ist, umfaßt mindestens zwei der folgenden Komponenten:
(a) Zellen, in denen zumindest unter bestimmten Bedingungen ein Ras- oder Ras-ähnlicher Signalweg nicht aktiviert werden kann;
(b) einen Nukleinsäurevektor, in den eine DNA-Sequenz exprimierbar eingefügt ist, die einen Membranrezeptor, wie vorstehend definiert, kodiert, wobei der Ligandenbindungsabschnitt des Membranrezeptors ein Polypeptid oder Protein, bei dem eine Ligandenbindungsfunktion eines Rezeptors vermutet wird, umfaßt oder daraus gebildet wird und das Effektorprotein oder -polypeptid, dessen Bindung an eine Membrankomponente von der Bindung oder, alternativ, *fehlenden* Bindung eines Liganden an den Ligandenbindungsabschnitt des Membranrezeptors abhängig ist, in der Lage ist, den inaktiven Ras- oder Ras-ähnlichen Signalweg in den unter (a) genannten Zellen zu aktivieren;
(c) einen Nukleinsäurevektor, in den eine DNA-Sequenz exprimierbar eingefügt ist, die das Effektorprotein oder -polypeptid, das im Falle einer Ligandenbindung oder, alternativ *fehlenden* Ligandenbindung an den Ligandenbindungsabschnitt des Membranrezeptors an eine Komponente der Membran, gegebenenfalls über weitere Proteine oder Polypeptide, binden kann, kodiert;
(d) einen Nukleinsäurevektor, in den eine DNA-Sequenz exprimierbar eingefügt ist, die mindestens ein Adaptorprotein, über welches das Effektorprotein oder -polypeptid bei Bindung oder, alternativ, *fehlender* Bindung eines Liganden an den Ligandenbindungsabschnitt des Membranrezeptors an eine Komponente der Membran binden kann, kodiert.

Ein alternativer Kit zur Verwendung in dem genannten Assay ermöglicht bei spezieller Auswahl der nachfolgend angegebenen Komponenten u.a. das gezielte Ausstatten einer Assayzelle mit einem Membranrezeptor, der ein vom Verwender des Kits gewünschtes, auf seine Ligandenbindungsfunktion zu untersuchendes Polypeptid oder Protein als Ligandenbindungsabschnitt umfaßt. Ein solcher Kit umfaßt die folgenden Komponenten: (a), (b) und (c) :
(a) Hefezellen, in denen zumindest unter bestimmten Bedingungen ein Ras- oder Ras-ähnlicher Signalweg nicht aktiviert werden kann;
(b) einen Nukleinsäurevektor, der in geeigneter Anordnung umfaßt:
- einen DNA-Abschnitt, der ein Membranlokalisierungssignal eines heterologen Membranrezeptors, wie in Anspruch 1 definiert, kodiert;
- einen DNA-Abschnitt, der einen Mediatorabschnitt eines heterologen Membranrezeptors, wie in Anspruch 1 definiert, kodiert; und
- eine geeignet angeordnete Insertionsstelle zur funktionalen Insertion einer DNA-Sequenz, die ein auf eine Ligandenbindungsfunktion eines Rezeptors hin zu untersuchendes Polypeptid oder Protein kodiert,
wobei nach Insertion einer DNA-Sequenz für den Ligandenbindungsabschnitt der Nukleinsäurevektor ein vollständiges exprimierbares Gen für einen heterologen Membranrezeptor umfaßt, wobei das Effektorprotein oder -polypeptid, dessen Bindung an eine Membrankomponente von der Bindung oder, alternativ, *fehlenden* Bindung eines Liganden an den aus dem auf eine Ligandenbindungsfunktion eines Rezeptors hin zu untersuchendes Polypetid oder Protein, gebildeten Ligandenbindungsabschnitt des Membranrezeptors abhängig ist, in der Lage ist, den inaktive Ras- oder Ras-ähnlichen Signalweg in den unter (a) genannten Hefezellen zu aktivieren;
(c) einen Nukleinsäurevektor, in den eine DNA-Sequenz exprimierbar eingefügt ist, die das Effektorprotein oder -polypeptid, das im Falle einer Ligandenbindung oder, alternativ, *fehlenden* Ligandenbindung an den Ligandenbindungsabschnitt des heterologen Membranrezeptors an eine Komponente der Membran, gegebenenfalls über weitere Proteine oder Polypeptide (Adaptoren), binden kann und das in Form eines heterologen Fusionsproteins eines Effektorabschnitts mit einem Adaptorprotein oder -polypeptid, das die Bindung an die Komponente der Membran, gegebenenfalls über wei- tere Proteine oder Polypeptide (Adaptoren), ermöglicht, vorliegt, Kodiert.
In einer bevorzugten Ausführungsform umfasst der Kit als Komponente (d) zusätzlich einen Nukleinsäurevektor, in den eine DNA-Sequenz exprimierbar eingefügt ist, die mindestens ein Adaptorprotein, über welches das Effektorprotein oder -polypeptid bei Bindung oder, alternativ, *fehlender* Bindung eines Liganden an den Ligandenbindungsabschnitt des heterologen Membranrezeptors an eine Komponente der Membran binden kann, kodiert.

Ebenso umfaßt in einer weiteren speziellen Ausführungsform der Komponente (c) der beiden letztgenannten Kits der darin enthaltene Nukleinsäurevektor eine DNA-Sequenz, die ein Effektorprotein oder -polypeptid in Form eines Fusionsproteins aus einem Effektorabschnitt und einem Antikörper- bzw. Bindungsproteinabschnitt mit spezifischer Bindungsaffinität für eine an dem Membranrezeptor befindliche "Tag"- oder Epitopstruktur kodiert, die nur aufgrund von Konformationsänderungen, ggf. in Kombination mit enzymatischer Aktivität, infolge einer Bindung von Ligand an den Ligandenbindungsabschnitt des Membranrezeptors oder, alternativ, Abdissoziierung des Liganden von dem Ligandenbindungsabschnitt, d.h. *fehlender* Bindung von Ligand an den Ligandenbindungsabschnitt des Membranrezeptors, für den Antikörper bzw. das Bindungsprotein zugänglich wird.

Bei einem vorgesehenen Nachweis einer Reportergenaktivität enthalten in einer Ausführungsform die Zellen in den vorstehend genannten Kits zusätzlich ein Konstrukt, umfassend eine Bindungsstelle für einen Tranakriptionsfaktor, dessen Aktivierung infolge einer Aktivierung des speziellen ras-Signalwegs, dessen Aktivierung durch den Assay nachgewiesen werden soll, erfolgt, einen Minimalpromotor und das Reportergen, wie vorstehend erläutert, oder es kann alternativ getrennt von den Zellen ein Transformations- oder Transfektionsvektor mit dem Transkriptionsfaktorbindungsstelle-Minimalpromotor-Reportergen-Konstrukt oder mit einem andersartigen Konstrukt, umfassend die Transkriptionsfaktorbindungsstelle und den Minimalpromotor und darüber hinaus eine geeignet angeordneten Insertionsstelle für ein frei wählbares Reportergen, vorgesehen werden.

Darüber hinaus können sämtliche Kits dieser Anmeldung in möglichen Ausführungsformen u.a. zusätzlich noch mindestens eine der folgenden Komponenten umfassen:
(e) gegebenenfalls einen Nukleinsäurevektor, in den eine DNA-Sequenz exprimierbar eingefügt ist, die mindestens ein Vermittlerprotein kodiert, welches mit dem Mediatorabschnitt des Membranrezeptor räumlich nicht unmittelbar assoziiert sein muß, jedoch im Falle einer Ligandenbindung oder, alternativ, einer *fehlenden* Ligandenbindung an den Ligandenbindungsabschnitt des Membranrezeptors von diesem Mediatorabschnitt aktiviert oder modifiziert wird und aufgrunddessen ein sekundäres Ereignis an einer weiteren Komponente der Zellmembran vermittelt, infolgedessen sich das Effektorprotein oder -polypeptid spezifisch an diese Komponente der Zellmembran, die mit dem Mediatorabschnitt des Membranrezeptors nicht assoziiert ist, bindet;
(f) gegebenenfalls Reagenzien zur Transformation oder Transfektion der Zellen mit Transformations- oder Transfektionsvektor(en),
(g) gegebenenfalls Reagenzien zur Detektion der phänotypischen Aktivierung des sich an ein Pas-Protein anschließenden Signaltransduktionsweges in diesen Zellen.

In einer bevorzugten Ausführungsform der Erfindung umfassen die Kits die Zellen immobilisiert auf einem festen Träger, wie vorstehend erläutert, insbesondere auf Biochips (vgl. Wolf et al. 1997 und 1998). Für Massenscreens und insbesondere High-Throughput-Verfahren zum Nachweisen und Messen von Membranrezeptor-Liganden-Interaktionen ist insbesondere die Immobilisierung der Zellen auf Biochips sowie in den individuellen Vertiefungen von Mikrotiterplatten geeignet, so daß auf einer solchen Platte eine Vielzahl von separaten Assayverfahren durchgeführt werden können. Es ist hierbei auch möglich, unterschiedliche Hefezellen, d.h. insbesondere Zellen mit unterschiedlichem Ligandenbindungsabschnitt, in Vertiefungen in jeweils bestimmten Abschnitten auf ein und derselben Mikrotiterplatte vorzusehen.

Befinden sich die Zellen in dem Kit immobilisiert an einem festen Träger; so kann es insbesondere bei Nachweis einer Reportergenaktivität oder -transkription oder eines Reporterproteins erforderlich oder hilfreich sein, die Zellen vor der Nachweisreaktion zu solubilisieren, d.h. sie von dem Träger abzulösen und ggf. auch aufzubrechen. Für diesen Fall können die unter g) genannten Reagenzien zur Detektion der phänotypischen Aktivierung des Ras- oder Ras-ähnlichen Signaltransduktionsweges auch geeignete Solubilisierungsreagenzien, die insbesondere ein oder mehrere grenzflächenaktive Mittel oder Tenside enthalten, umfassen.

Die Anmeldung erstreckt sich auch auf
- Liganden für einen Bindungsabschnitt eines Rezeptors,
- Verbindungen, die in der Lage sind, eine Bindungsaktivität eines Ligandenbindungsabschnitts eines Rezeptors gegenüber einem Liganden zu verändern, (im Folgenden als "Modifizierungsverbindungen" bezeichnet) sowie
- Polypeptide oder Proteine, die eine Ligandenbindungsfunktion eines Rezeptors aufweisen,
die mittels eines der Assayverfahren der Anmeldung bestimmt oder ermittelt worden sind, sowie Zusammensetzungen, die diese Liganden, Verbindungen und/oder Polypeptide oder Proteine enthalten.

Hinsichtlich Polypeptiden oder Proteinen, die eine Ligandenbindungsfunktion eines Rezeptors aufweisen und zur Erzeugung des Membranrezeptors, wie in Anspruch 1 definiert, von einem natürlich aufgefundenen oder synthetisch erzeugten Molekül abgeleitet worden sind, umfaßt die Erfindung sowohl das in den erfindungsgesäß eingesetzten Membranrezeptoren enthaltene Fragment mit Ligandenbindungsfunktion als auch das Ausgangsfragment- bzw. - molekül. Nur der Klarheit halber sei diesbezüglich angemerkt, daß die Erzeugung des heterologen Membranrezeptors in der Regel durch Expression einer diesen heterologen Membranrezeptor kodierenden Nukleinsäuresequenz in einer Zelle erfolgt. Das Ableiten eines Polypeptids oder Proteins mit Ligandenbindungsfunktion eines Rezeptors von einem größeren Ausgangsmolekül erfolgt dementsprechend in der Regel in analoger Weise auf Nukleinsäure-Ebene, indem lediglich ein oder mehrere Abschnitte der das Ausgangsmolekül kodierenden Nukleinsäuresequenz, gegebenenfalls unter darauffolgender Klonierung zur Anfügung von Abschnitten, die weitere Membranrezeptorkomponenten oder -abschnitte kodieren, für die Expression des Membranrezeptors genutzt werden. Im Rahmen des Ableitens können auch eine oder mehrere geringfügige Nukleinsäuresequenzmodifizierungen in der Ausgangssequenz oder dem oder den Nukleinsäureabschnitt(en) vorgenommen werden, bevorzugt solcher Art, daß das resultierende Nukleinsäuremolekül unter stringenten Bedingungen noch mit dem jeweiligen Ausgangsnukleinsäuremolekül hybridisiert. Die Anmeldung umfaßt dementsprechend auch ein Verfahren zur Identifizierung von Polypeptiden oder Proteinen, insbesondere Rezeptoren, die eine Ligandenbindungsfunktion eines Rezeptors aufweisen, welches umfaßt:
- eine transformierte Hefezelle nach Anspruch 1 mit einem heterologen Membranrezeptor, der die in Anspruch 1 beschriebenen Merkmale aufweist und die Gesamtheit eines derartigen Polypeptids oder Proteins oder einen Teil eines derartigen Polypeptids oder Proteins, der mutmaßlich die für die Ligandenbindungsfunktion essentiellen Sequenzabschnitte enthält, umfaßt, herzustellen und
- mittels dieser Hefezelle ein *in vivo-*Assayverfahren zum Nachweis, ob ein Polypeptid oder Protein eine Ligandenbindungsfunktion eines Rezeptors aufweist, nach Anspruch 15 auszuführen.
sowie die das durch dieses Verfahren identifizierten Moleküle.

Die Anmeldung offenbart darüber hinaus
- die Verwendung der vorstehend genannten, durch die erfindungsgemäßen Assay-Verfahren identifizierten Liganden, Modifizierungsverbindungen und Polypeptide bzw. Proteine als Arzneimittel, gegebenenfalls nach Formulierung mit in diesem Bereich üblichen Hilfs- und/oder Trägerstoffen, sowie
- die Verwendung der Liganden, Modifizierungsverbindungen und Polypeptide bzw. Proteine als Leitsubstanzen zur Entwicklung von davon - insbesondere durch Derivatisierung - abgeleiteten Liganden, Modifizierungsverbindungen und Polypeptiden bzw. Proteinen, insbesondere solchen mit entsprechender oder verbesserter Aktivität gegenüber der jeweiligen Leitsubstanz.

Somit offenbart die Anmeldung auch ein Verfahren zur Herstellung von Liganden, Modifizierungsverbindungen, Polypeptiden oder Proteinen durch ein- oder mehrfache Derivatisierung ausgehend von mittels der erfindungsgemäßen Assayverfahren identifizierten Liganden, modifizierungsverbindungen, Polypeptiden oder Proteinen. Dieses Verfahren kann gegebenenfalls zusätzlich die Schritte umfassen,
- auch die durch die Derivatisierung erhaltenen Liganden, Modifizierungsverbindungen, Polypeptide oder Proteine mittels der erfindungsgemäßen Assay-Verfahren auf Ligandenfunktion bzw. Ligandenbindungsfunktion zu testen, und/oder
- die durch die Derivatisierung erhaltenen Liganden, Modifizierungsverbindungen, Polypeptide oder Proteine in üblicher weise als Arzneimittel zu formulieren.
Des weiteren offenbart die Anmeldung auch auf die durch dieses Verfahren erhaltenen Liganden, Modifizierungsverbindungen, Polypeptide und Proteine, d.h. die durch dieses Verfahren erhaltenen funktionellen Derivate.

Für eine Verwendung als Gentherapeutika, die die Expression eines Polypeptids oder Proteins, das eine Ligandenbindungsfunktion eines Rezeptors aufweist, bevorzugt eines Rezeptors, insbesondere in memschlichen oder tierischen Zellen bewirken sollen, offenbart die Anmeldung des weiteren Nukleinsäuremoleküle, die ausgehend von einem mittels der beschriebenen Assay-, Identifizierungs-, Screening- oder Herstellungsverfahren identifizierten Polypeptid oder Protein, insbesondere Rezeptor, durch ein Verfahren erhalten werden, das die Bereitstellung des das Polypeptid oder Protein kodierenden Gens oder eines Teils davon, der zumindest die für die Aktivität des kodierten Polypeptids oder Proteins essentiellen Nukleinsäuresequenzabschnitte umfaßt, in im wesentlichen reiner Form, d.h. insbesondere im wesentlichen frei von anderen Nukleinsäuren, die für die Verwendung als Gentherapeutikum nicht erforderlich oder gar abträglich sind, umfaßt. Dieses Verfahren kann, sofern noch nicht bekannt, die vorab erfolgende Identifizierung des Gens, das dieses Polypeptid oder Protein kodiert, erfordern. Insbesondere kann das Verfahren zusätzlich auch die folgenden Schritte umfassen:
- sofern noch nicht bekannt, die Aminosäuresequenz des Polypeptids oder Proteins, insbesondere Rezeptors, zu bestimmen, und/oder
- sofern noch nicht bekannt, das Gen, das dieses Polypeptid oder Protein kodiert, zu identifizieren und zumindest die Sequenz der kodierenden Abschnitte dieses Gens zu bestimmen,
- gegebenenfalls Modifizierungen in der erhaltenen Nukleinsäuresequenz vorzunehmen, beispielsweise zum Anpassen der Codonnutzung an diejenige eines gewünschten Empfängerorganismus, zum Einführen von Mutationen oder zur Entfernung von Intron-Sequenzen, und
- die gegebenenfalls modifizierte Nukleinsäuresequenz in Form eines Gentherapeutikums zu formulieren.

In einem gegenwärtig bevorzugt verwendeten experimentellen System der Anmeldung wird als Zellsystem, welches in einem ras-oder ras-ähnlichen Signaltransduktionsweg inaktiv ist, der Hefestamm cdc25-2 verwendet. Wie erläutert, ist in diesen Zellen das Ras-Protein bei einer restriktiven Temperatur von 33-37°C, typischerweise 36°C, infolge der Abwesenheit eines funktionellen Guaninnukleotid-Austauschfaktors (GEF; "guanyl nucleotide exchange factor") nicht funktionell (Broder et al., 1998). Eine Aktivierung des in diesem System bei restriktiven Temperaturen nicht funktionellen ras-Signaltransduktionswegs, die unter diesen Bedingungen nur über die, wie beschrieben, über verschiedene Maßnahmen bewirkbare Translokation eines Effektorproteins oder -polypeptids, das diesen ras-Signaltransduktionsweg zu aktivieren vermag, an die Zellmembran veranlaßt werden kann, kann dadurch detektiert werden, daß die Hefezellen unabhängig von der Anwesenheit eines funktionellen GEF-Proteins bei restriktiven Temperaturen (33-37°C, typischerweise 36°C) wachsen können. Wie erläutert erfordert die Translokation des Effektorproteins oder -polypeptids, daß ein passender Ligand für den speziell in der Zelle vorgesehenen Membranrezeptor vorhanden ist.

In diesem System wird insbesondere ein Fusionsprotein eingesetzt, das als Effektorabschnitt ein mutiertes humanes Ras-Protein (Ha-Ras, L61) umfaßt, dem die Farnesylierungssequenz, welche für eine Membranlokalisierung des Proteins sorgt, fehlt.

Die nachfolgend exemplarisch angegebenen Beispiele sollen die Erfindung weiter veranschaulichen.

### 1. "Epidermal growth factor" (EGF)-Interaktion mit dem EGF-Rezeptor (Figur 3)

### Material und Methoden

### Folgende zwei Vektoren dienen zur Transformation von Saccharomyces cerevisiae cdc25-2 Zellen:

1. Ein Expressionsvektor für die konstitutive Expression des humanen EGF-Rezeptorproteins.
2. Ein induzierbarer Expressionsvektor mit einem Galaktose-induzierbaren Promotor (Gal 1) für die Expression des Adaptor-Effektor-Fusionsproteins, bestehend aus dem humanen konstitutiv aktiven ras-Proteinteil (Ha-ras, L61), dem die sogenannte CAAX-Box (das Farnesylierungssignal zur Membranlokalisierung) fehlt, (=Effektorabschnitt) und dem murinen Grb2-Proteinteil (=Adaptorabschnitt).

### Hefewachstum und -manipulation

Es wurden konventionelle Hefe-Transformations- und -manipulationsprotokolle (siehe z.B. Hill et al. (1991), NAR 19, 5791) verwendet. Die Zellen wurden entweder auf einem Glucoseminimalmedium, welches die erforderlichen Aminosäuren und Nukleotide (20 mg/l Histidin, 100 mg/l Leucin, 20 mg/l Tryptophan, 20 mg/l Uracil, 10 mg/l Adeninsulfat), 2% Glucose, 0,5% NH₄SO₄, 0,17% Hefeextrakt und 4% Agar enthält, oder auf Galaktosemedium (1,7 g/l Yeast Nitrogen ohne Aminosäuren, 5 g/l Ammoniumsulfat, 30 g/l Galaktose (> 99%) 20 g/l D-Raffinose, 20 g/l Glycerin (100%), 30 g/l Bacto-Agar) ausplattiert.

Replika-Plattierungen werden mit einem Samt-Replikaplattierer durchgeführt. Die Zellen werden nach der Transformation mit den oben beschriebenen zwei Expressionsvektoren auf Glucoseplatten ausplattiert und bei 25°C (nicht-restriktive Temperatur) für 3-4 Tage inkubiert. Anschließend werden verschiedene Testmedien (mit und ohne EGF, bzw. mit synthetischen Proteinfragmenten mit Ligandenaktivität - siehe Komoriya et al., 1984) mit jeweils drei unabhängigen Klonen angeimpft und bei 37°C für 2-3 Tage inkubiert und anschließend das Wachstum der Hefen in den verschiedenen Flüssigmedien detektiert. Hefen, die in Galaktosemedium bei 37°C nur in Anwesenheit von EGF (zellwandlose Hefe-ghost-Zellkultur) oder nur in Anwesenheit von [S-Acetamidomethyl Cys ^{20, 11}]-EGF-(20-31), einem aktiven EGF-Fragment, wachsen, zeigen funktionsfähige Liganden-Rezeptor-Interaktion an.

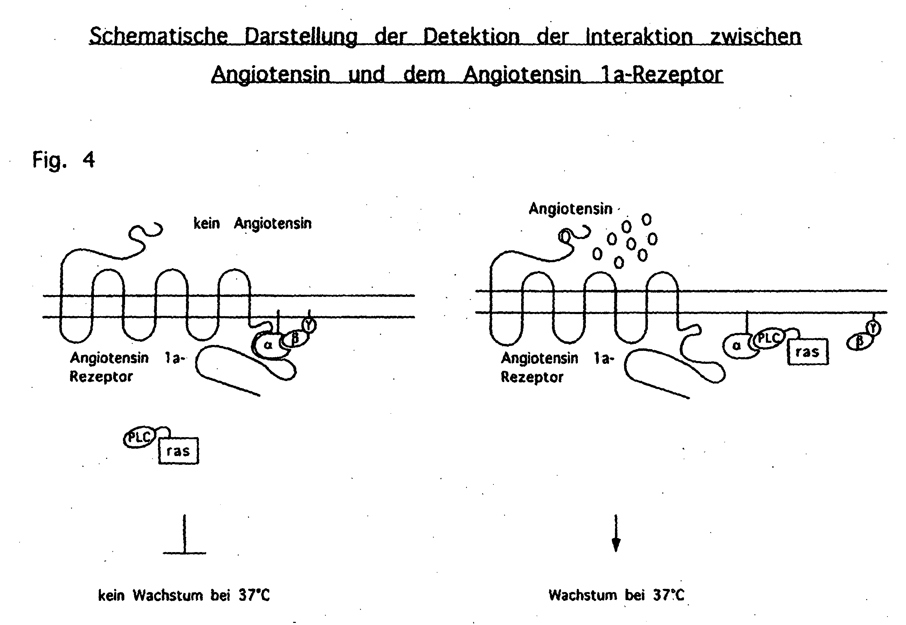

### 2. Angiotensin-Interaktion mit dem Angiotensinrezeptor (Figur 4)

### Material und Methoden

Folgende Vektoren dienen zur Transformation von *Saccharomyces cerevisiae* cdc 25-2 Zellen:
1. Ein Expressionsvektor für die konstitutive Expression des AT1A-Angiotensin-Rezeptors der Ratte (Condon et al., 1997)
2. Ein oder mehrere Expressionsvektoren für die konstitutive Expression der drei G-Proteine Gα₁₃, Gβ₁ und Gγ₃ der Ratte (Macrez-Leprêtre et al., 1997).
3. Ein induzierbarer Expressionsvektor (Selektionsmarker Ura) mit einem Galaktose-induzierbaren Promotor (Gal1) für die Expression des Adaptor-Effektor-Fusionsproteins, bestehend aus der Phosphatidylcholin-Phospholipase C minus der Domäne mit dem Membranlokalisierungssignal (=Adaptorabschnitt) aus vaskulären Myozyten der Ratte (Macrez-Leprêtre et al., 1996) und dem humanen konstitutiv aktiven ras-Proteinanteil (Ha-ras L61), dem die sogenannte CAAX-Box (das Farnesylierungssignal zur Membranlokalisierung) fehlt (=Effektorabschnitt).

### Hefewachstum und Manipulation

Hier gilt entsprechendes wie in Beispiel 1. Die für die Angiotensinrezeptorversuche verwendeten Testmedien wurden plus/minus Angiotensin bzw. plus/minus L-162,313, einem funktionellen, nicht-proteinartigen Ligand des Angiotensinrezeptors (Perlmann et al., 1995), eingesetzt. Hefen, die nach 2 bis 3 Tagen bei 37°C in Galactosemedium nur in Anwesenheit von Angiotensin (zellwandlose Hefe-ghost-Zellkultur) oder nur in Anwesenheit des Nichtproteinliganden L-162,313 wachsen, zeigen funktionsfähige Liganden-Rezeptor-Interaktion an.

### Literatur

- Broder, Y.C., Katz, S. und Aronheim, A. (1998). Curr. Biol. 8, 1121-1124
- Conchon, S., Barrault, M.-B., Miserey, S., Corvol, P. und Clauser, E. (1997). J. Biol. Chem. 272, 25566-25572
- Current Protocols in Molecular Biology, 1991
- Dohlman, H. G., Thomer, J. , Caron, M.G. Leifkowitz, R.J. (1991). Ann. Rev. Biochem. 60, 653-688
- Komoriya, A., Hortsch, M., Meyers, C., Smith, M., Kanety, H. und Schlessinger, J. (1984) Proc. Natl. Acad. Sci. USA 81, 1351-1355
- Leurs, R., Smit, M.J., Alewijnse, A.E. und Timmermann, H. (1998). TIBS 23, 418-422
- Macrez-Leprêtre, N., Morel, J.-L. und Mironneau, J. (1996) J. Pharmacol. Exp. Ther. 278, 468-475
- Macrez-Lepretre, N., Kalkbrenner, F., Morel, J.-L., Schultz, G. und Mironneau, J. (1997). J. Biol. Chem. 272, 10095-10102
- Nishida, E. und Gotoh, Y (1993). Trends Biochem. Sci 18, 128-130
- Perlmann, S., Shambye, H.T., Rivero, R.A., Greenlee, W.J., Hjorth, S.A. und Schwartz, T.W. (1995). J. Biol. Chem. 270, 1493-1496
- Sambrook, J., Fritsch, E.F., Maniatis, T. (1989), Molecular Cloning. A Laboratory Handbook, Cold Spring Harbor Laboratory, New York
- Schlessinger, J. und Ulrich, A. (1992). Neuron 9, 383-391
- Schlessinger, J. (1993). Trends Biochem. Sciences 18, 273-275
- Stahl, N. und Yancopoulos, G.D. (1993) Cell 74, 587-590
- Wolf, B., Kraus, M., Baumann, W., Brischwein, M., Ehret, R., Henning, T., Lehmann, M. und Schwinde, A., (1997). BioTec 6/97, 26-29
- ibid (1998). BioTec 1/98, 24-27

## Patentansprüche

1. Transformierte Hefezelle, umfassend einen heterologen Membranrezeptor, der einen Ligandenbindungsabschnitt, ein Membranlokalisierungssignal und einen Mediatorabschnitt umfasst, wobei nur bei Bindung oder, alternativ, nur bei fehlender Bindung eines Liganden an den Ligandenbindungsabschnitt eine Strukturänderung mit Auswirkungen auf den Mediatorabschnitt bewirkt wird, so dass in der Folge ein Effektorprotein oder -polypeptid, das in der Lage ist, einen Ras- oder Ras-ähnlichen Signalweg in der Zelle zu aktivieren, an eine Komponente der Membran, gegebenenfalls über weitere Proteine oder Polypeptide (Adaptoren), bindet,
**dadurch gekennzeichnet,**
**dass** das Effektorprotein oder -polypeptid, das in der Lage ist, einen Ras- oder Ras-ähnlichen Signalweg zu aktivieren, in Form eines heterologen Fusionsproteins eines Effektorabschnitts mit einem Adaptorprotein oder -polypeptid, das die Bindung an die Komponente der Membran, gegebenenfalls über weitere Proteine oder Polypeptide (Adaptoren), ermöglicht, vorliegt, wobei der Effektorabschnitt des Fusionsproteins die Sequenz eines konstitutiv aktiven Ras-Proteins umfasst.

2. Transformierte Hefezelle nach Anspruch 1,
**dadurch gekennzeichnet,**
**dass** der heterologe Membranrezeptor ein Transmembranrezeptor, ein Enzymgekoppelter Rezeptor, ein G-Protein-gekoppelter Rezeptor, ein 7-Transmembranrezeptor oder ein Geruchsrezeptor ("Odour Receptor" oder "Olfactorial Receptor") ist.

3. Transformierte Hefezelle nach Anspruch 1,
**dadurch gekennzeichnet,**
**dass** der Ligandenbindungsabschnitt von einem Ligandenbindungsabschnitt eines in der Natur vorkommenden Rezeptors durch Mutation abgeleitet oder ein durch "molecular modelling" erzeugter, synthetischer Ligandenbindungsabschnitt ist.

4. Transformierte Hefezelle nach Anspruch 3,
**dadurch gekennzeichnet,**
**dass** der Ligandenbindungsabschnitt eines in der Natur vorkommenden Rezeptors der Ligandenbindungsabschnitt eines Transmembranrezeptors, eines G-Protein-gekoppelten Rezeptors, eines 7-Transmembranrezeptors, eines Geruchsrezeptors ("Odour Receptor" oder "Olfactorial Receptor") oder eines nukleären Rezeptors ist.

5. Transformierte Hefezelle nach Anspruch 3,
**dadurch gekennzeichnet,**
**dass** die Mutation eine Substitution, Deletion, Insertion und/oder Modifizierung einer oder mehrerer Aminosäuren oder Gruppen von Aminosäuren ist.

6. Transformierte Hefezelle nach einem der Ansprüche 1 bis 5,
**dadurch gekennzeichnet,**
**dass** der Mediatorabschnitt infolge der Ligandenbindung oder, alternativ, fehlender Ligandenbindung an den Ligandenbindungsabschnitt in der Lage ist, ein oder mehrere Adaptorproteine zu binden, über welche das Effektorprotein oder -polypeptid, das in der Lage ist, einen Ras- oder Ras-ähnlichen Signalweg in der Zelle zu aktivieren, in Form eines heterologen Fusionsproteins eines Effektorabschnitts mit einem Adaptorprotein oder -polypeptidabschnitt, der die Bindung an die Komponente der Membran über eines oder mehrere der Adaptorproteine ermöglicht, an den Mediatorabschnitt binden kann.

7. Transformierte Hefezelle nach Anspruch 6,
**dadurch gekennzeichnet,**
**dass** der Mediatorabschnitt den cytoplasmatischen Teil des EGFR ("epidermal growth factor receptor") umfasst oder von diesem abstammt.

8. Transformierte Hefezelle nach einem der Ansprüche 1 bis 7,
**dadurch gekennzeichnet,**
**dass** die Hefezelle eine zellwandlose Hefezelle ist.

9. Transformierte Hefezelle nach einem der Ansprüche 1 bis 8,
**dadurch gekennzeichnet,**
**dass** der heterologe Membranrezeptor zur Aktivierung eines Ras- oder Ras-ähnlichen Signalwegs in der transformierten Hefezelle erforderlich ist.

10. *in vivo*-Assay zum Nachweis der Anwesenheit eines Liganden für einen Ligandenbindungsabschnitt eines Rezeptors in einer auf den Liganden hin zu untersuchenden Probe, **gekennzeichnet durch** die folgenden Schritte:
(a) Inkontaktbringen der Probe mit transformierten Hefezellen nach Anspruch 9 unter Bedingungen, bei denen bei Fehlen des heterologen Membranrezeptors der Ras- oder Ras-ähnliche Signalweg in der Zelle nicht aktiviert werden kann,
wobei der heterologe Membranrezeptor den genannten Ligandenbindungsabschnitt enthält und das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors, wie in Anspruch 1 definiert, abhängig ist, in der Lage ist, diesen Ras- oder Ras-ähnlichen Signalweg zu aktivieren,
(b) Untersuchen, ob eine Aktivierung des Ras- oder Ras-ähnlichen Signalwegs erfolgt ist,
(c) wobei für den Fall, dass die Bindung des Effektorproteins oder -polypeptids von der Bindung eines Liganden an den Ligandenbindungsabschnitt des heterologen Membranrezeptors abhängig ist, ein Nachweis der Aktivierung des Ras- oder Ras-ähnlichen Signalwegs die Abwesenheit eines Liganden oder Ligandenbindungsabschnitt eines Rezeptors in der Probe anzeigt,
und wobei für den Fall, dass die Bindung des Effektorproteins oder -polypeptids von der fehlenden Bindung eines Liganden an den Ligandenbindungsabschnitt des heterologen Membranrezeptors abhängig ist,
Zellen, wie sie in Schritt (a) eingesetzt wurden, unter Bedingungen, bei denen bei Fehlen des heterologen Membranrezeptors der Ras- oder Ras-ähnliche Signalweg in der transformierten Hefezelle nicht aktiviert werden kann, in Abwesenheit der Probe auf Aktivierung des Ras- oder Ras-ähnlichen Signalwegs untersucht werden,
wobei ein Nachweis der Aktivierung des Ras- oder Ras-ähnlichen Signalwegs in Abwesenheit der Probe und die Inaktivität des Ras- oder Ras-ähnlichen Signalwegs in Anwesenheit der Probe die Anwesenheit eines Liganden für den Ligandenbindungsabschnitt eines Rezeptors in der Probe anzeigt.

11. Assay nach Anspruch 10,
**dadurch gekennzeichnet,**
**dass** die Testsubstanz (a) eine natürlich vorkommende Substanz oder (b) eine in der Natur nicht vorkommende Substanz ist.

12. Assay nach Anspruch 11,
**dadurch gekennzeichnet,**
**dass** die natürlich vorkommende Substanz ein Geruchsstoff, Geschmacksstoff, Peptid, Peptidhormon, Protein, Wachstumsfaktor, Neurotransmitter, nicht-protein- oder -peptidartiges Hormon und/oder Vitamin ist und dass die in der Natur nicht vorkommende Substanz ein synthetisches Derivat eines natürlichen Liganden oder ein Giftstoff ist.

13. Screeningverfahren nach unbekannten Liganden eines bestimmten Rezeptors,
**dadurch gekennzeichnet,**
**dass** für das Screening ein Assayverfahren nach Anspruch 10 eingesetzt wird.

14. *in vivo*-Assay zum Nachweis, ob eine Verbindung in der Lage ist, eine Bindungsaktivität eines Ligandenbindungsabschnitts eines Rezeptors gegenüber einem Liganden zu verändern, **gekennzeichnet durch** die folgenden Schritte:
(a) Inkontaktbringen des Liganden in Anwesenheit der Verbindung mit transformierten Hefezellen nach Anspruch 9 unter Bedingungen, bei denen bei Fehlen des heterologen Membranrezeptors der Ras- oder Ras-ähnliche Signalweg in der Zelle nicht aktiviert werden kann, wobei der heterologe Membranrezeptor den genannten Ligandenbindungsabschnitt enthält und das Effektorprotein oder -polypeptid, dessen Bindung an eine Membrankomponente von der Bindung oder, alternativ, der fehlenden Bindung eines Liganden an den Ligandenbindungsabschnitt des heterologen Membranrezeptors, wie in Anspruch 1 definiert, abhängig ist, in der Lage ist, diesen Ras- oder Ras-ähnlichen Signalweg zu aktivieren,
(b) Untersuchen, ob und gegebenenfalls in welchem Ausmaß eine Aktivierung des Ras- oder Ras-ähnlichen Signalwegs erfolgt ist,
(c) Vergleichen des Untersuchungsergebnisses von Schritt (b) mit einem Untersuchungsergebnis, das bei Ausführen des Assay in Abwesenheit der Verbindung erhalten wird.

15. *in vivo*-Assay zum Nachweis, ob ein Polypeptid oder Protein eine Ligandenbindungsfunktion eines Rezeptors aufweist, **gekennzeichnet durch** die folgenden Schritte:
(a) Inkontaktbringen von transformierten Hefezellen nach Anspruch 9 mit dem Liganden unter Bedingungen, bei denen bei Fehlen des heterologen Membranrezeptors, wie in Anspruch 1 definiert, ein Ras- oder Ras-ähnlicher Signalweg in den Zellen nicht aktiviert werden kann, wobei der Ligandenbindungsabschnitt des heterologen Membranrezeptors das zu untersuchende Polypeptid oder Protein umfasst oder daraus besteht und wobei das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors abhängig ist, in der Lage ist, diesen Ras- oder Ras-ähnlichen Signalweg zu aktivieren,
(b) Untersuchen, ob eine Aktivierung des Ras- oder Ras-ähnlichen Signalwegs erfolgt ist,
wobei für den Fall, dass die Bindung des Effektorproteins oder -polypeptids von der Bindung eines Liganden an den Ligandenbindungsabschnitt des heterologen Membranrezeptors abhängig ist, ein Nachweis der Aktivierung des Ras- oder Ras-ähnlichen Signalweges anzeigt, dass der Ligandenbindungsabschnitt des heterologen Membranrezeptors und dementsprechend das zu untersuchende Polypeptid oder Protein eine Ligandenbindungsfunktion eines Rezeptors aufweist und
für den Fall, dass die Bindung des Effektorproteins oder -polypeptids von der fehlenden Bindung eines Liganden an den Ligandenbindungsabschnitt des heterologen Membranrezeptors abhängig ist, Zellen, wie sie in Schritt (a) eingesetzt wurden, unter Bedingungen, bei denen bei Fehlen des heterologen Membranrezeptors der Ras- oder Ras-ähnliche Signalweg in der transformierten Hefezelle nicht aktiviert werden kann, in Abwesenheit von Ligand auf Aktivierung des Ras- oder Ras-ähnlichen Signalwegs untersucht werden, wobei ein Nachweis der Aktivierung des Ras- oder Ras-ähnlichen Signalwegs in Abwesenheit des Liganden und die Inaktivität des Ras- oder Ras-ähnlichen Signalwegs in Anwesenheit des Liganden anzeigt, dass der Ligandenbindungsabschnitt des heterologen Membranrezeptors und dementsprechend das zu untersuchende Polypeptid oder Protein eine Ligandenbindungsfunktion eines Rezeptors aufweist.

16. Kit zur Verwendung in einem Assay nach einem der Ansprüche 10 bis 14, weicher die nachfolgend angegebenen Komponenten (a), (b) und (c) umfasst:
(a) Hefezellen, in denen zumindest unter bestimmten Bedingungen ein Ras- oder Ras-ähnlicher Signalweg nicht aktiviert werden kann,
(b) einen Nukleinsäurevektor, der in geeigneter Anordnung umfasst:
- einen DNA-Abschnitt, der ein Membranlokalisierungssignal eines heterologen Membranrezeptors, wie in Anspruch 1 definiert, kodiert;
- einen DNA-Abschnitt, der einen Mediatorabschnitt eines heterologen Membranrezeptors, wie in Anspruch 1 definiert, kodiert und
- eine geeignete angeordnete Insertionsstelle zur funktionalen Insertion einer DNA-Sequenz, die einen Ligandenbindungsabschnitt, wie in Anspruch 1 definiert, kodiert,
wobei nach Insertion einer DNA-Sequenz für den Ligandenbindungsabschnitt der Nukleinsäurevektor ein vollständiges exprimierbares Gen für einen heterologen Membranrezeptor, wie in Anspruch 1 definiert, umfasst, wobei das Effektorprotein oder -polypeptid, dessen Bindung an eine Membrankomponente von der Bindung oder, alternativ, fehlenden Bindung eines Liganden an den Ligandenbindungsabschnitt des heterologen Membranrezeptors abhängig ist, in der Lage ist, den inaktiven Ras- oder Ras-ähnlichen Signalweg in den unter (a) genannten Hefezellen zu aktivieren,
(c) einen Nukleinsäurevektor, in den eine DNA-Sequenz exprimierbar eingefügt ist, die das Effektorprotein oder -polypeptid, das im Falle einer Ligandenbindung oder, alternativ, fehlenden Ligandenbindung an den Ligandenbindungsabschnitt des heterologen Membranrezeptors an eine Komponente der Membran, gegebenenfalls über weitere Proteine oder Polypeptide (Adaptoren), binden kann und das in Form eines heterologen Fusionsproteins eines Effektorabschnitts mit einem Adaptorprotein oder -polypeptid, das die Bindung an die Komponente der Membran, gegebenenfalls über weitere Proteine oder Polypeptide (Adaptoren), ermöglicht, vorliegt, kodiert.

17. Kit zur Verwendung in einem Assay nach Anspuch 15, welcher die nachfolgend angegebenen Komponenten (a), (b) und (c) umfasst:
(a) Hefezellen, in denen zumindest unter bestimmten Bedingungen ein Ras- oder Ras-ähnlicher Signalweg nicht aktiviert werden kann,
(b) einen Nukleinsäurevektor, der in geeigneter Anordnung umfasst:
- einen DNA-Abschnitt, der ein Membranlokalisierungssignal eines heterologen Membranrezeptors, wie in Anspruch 1 definiert, kodiert;
- einen DNA-Abschnitt, der einen Mediatorabschnitt eines heterologen Membranrezeptors, wie in Anspruch 1 definiert, kodiert und
- eine geeignet angeordnete Insertionsstelle zur funktionalen Insertion einer DNA-Sequenz, die ein auf eine Ligandenbindungsfunktion eines Rezeptors hin zu untersuchendes Polypeptid oder Protein kodiert,
wobei nach Insertion einer DNA-Sequenz für den Ligandenbindungsabschnitt der Nukleinsäurevektor ein vollständiges exprimierbares Gen für einen heterologen Membranrezeptor umfasst, wobei das Effektorprotein oder -polypeptid, dessen Bindung an eine Membrankomponente von der Bindung oder, alternativ, fehlenden Bindung eines Liganden an den aus dem auf eine Ligandenbindungsfunktion eines Rezeptors hin zu untersuchenden Polypeptid oder Protein gebildeten Ligandenbindungsabschnitt des Membranrezeptors abhängig ist, in der Lage ist, den inaktiven Ras- oder Ras-ähnlichen Signalweg in den unter (a) genannten Hefezellen zu aktivieren,
(c) einen Nukleinsäurevektor, in den eine DNA-Sequenz exprimierbar eingefügt ist, die das Effektorprotein oder -polypeptid, das im Falle einer Ligandenbindung oder, alternativ, fehlenden Ligandenbindung an den Ligandenbindungsabschnitt des heterologen Membranrezeptors an eine Komponente der Membran, gegebenenfalls über weitere Proteine oder Polypeptide (Adaptoren), binden kann und das in Form eines heterologen Fusionsproteins eines Effektorabschnitts mit einem Adaptorprotein oder
- polypeptid, das die Bindung an die Komponente der Membran, gegebenenfalls über weitere Proteine oder Polypeptide (Adaptoren), ermöglicht, vorliegt, kodiert.

18. Kit nach einem der Ansprüche 16 oder 17,
**dadurch gekennzeichnet,**
**dass** er zusätzlich einen Nukleinsäurevektor umfasst, in den eine DNA-Sequenz exprimierbar eingefügt ist, die mindestens ein Adaptorprotein, über welches das Effektorprotein oder -polypeptid, bei Bindung oder, alternativ, fehlender Bindung eines Liganden an den Ligandenbindungsabschnitt des heterologen Membranrezeptors an eine Komponente der Membran, binden kann, kodiert.

19. Kit nach einem der Ansprüche 16 bis 18,
**dadurch gekennzeichnet,**
**dass** er zusätzlich einen Transformations- oder Transfektionsvektor mit einem Konstrukt, umfassend eine Bindungsstelle für einen Transkriptionsfaktor, dessen Aktivierung infolge einer Aktivierung eines speziellen Ras- oder Ras-ähnlichen Signalwegs, dessen Aktivierung durch den Assay nachgewiesen werden soll, erfolgt, einen Minimalpromotor und eine für eine durch den Minimalpromotor gesteuerte Expression geeignet angeordnete Insertionsstelle für eine Insertion eines Reportergens, wobei der Minimalpromotor infolge einer Bindung des aktivierten Transkriptionsfaktors an seine Bindungsstelle aktiviert wird, enthält.

20. Verfahren zur Identifizierung von Polypeptiden oder Proteinen, die eine Ligandenbindungsfunktion eines Rezeptors aufweisen, welches umfasst:
- eine transformierte Hefezelle nach Anspruch 1 mit einem heterologen Membranrezeptor, der die in Anspruch 1 beschriebenen Merkmale aufweist und die Gesamtheit eines derartigen Polypeptids oder Proteins oder einen Teil eines derartigen Polypeptids oder Proteins, der mutmaßlich die für die Ligandenbindungsfunktion essenziellen Sequenzabschnitte enthält, umfasst, herzustellen und
- mittels dieser transformierten Hefezelle ein *in vivo*-Assayverfahren zum Nachweis, ob ein Polypeptid oder Protein eine Ligandenbindungsfunktion eines Rezeptors aufweist, nach Anspruch 15 auszuführen.

## Claims

1. A transformed yeast cell, comprising a heterologous membrane receptor which comprises a ligand-binding section, a membrane-localization signal and a mediator section, where only when there is binding or, alternatively, only when there is a lack of binding of a ligand to the ligand-binding section a structural change is brought about with effects on the mediator section to result in binding of an effector protein or polypeptide, which is capable of activating a Ras or Ras-like signal pathway in the cell, to a component of the membrane, where appropriate via other proteins or polypeptides (adaptors),
**characterized in that**
the effector protein or polypeptide which is capable of activating a Ras or Ras-like signal pathway is in the form of a heterologous fusion protein of an effector section with an adaptor protein or polypeptide which makes binding to the component of the membrane possible, where appropriate via other proteins or polypeptides (adaptors), wherein the effector section of the fusion protein comprises the sequence of a constitutively active Ras protein.

2. The transformed yeast cell as claimed in claim 1,
**characterized in that**
the heterologous membrane receptor is a transmembrane receptor, an enzyme-coupled receptor, a G-protein-coupled receptor, a 7-transmembrane receptor or an odor receptor (or olfactorial receptor).

3. The transformed yeast cell as claimed in claim 1,
**characterized in that**
the ligand-binding section is derived from a ligand-binding section of a naturally occurring receptor by mutation or is a synthetic ligand-binding section generated by molecular modeling.

4. The transformed yeast cell as claimed in claim 3,
**characterized in that**
the ligand-binding section of a naturally occurring receptor is the ligand-binding section of a transmembrane receptor, of a G-protein-coupled receptor, of a 7-transmembrane receptor, of an odor receptor (or olfactorial receptor) or of a nuclear receptor.

5. The transformed yeast cell as claimed in claim 3,
**characterized in that**
the mutation is a substitution, deletion, insertion and/or modification of one or more amino acids or groups of amino acids.

6. The transformed yeast cell as claimed in any one of claims 1 to 5,
**characterized in that**
the mediator section is able, as a result of the ligand binding or, alternatively, lack of ligand binding to the ligand-binding section, to bind one or more adaptor proteins via which the effector protein or polypeptide which is capable of activating a Ras or Ras-like signal pathway in the cell, in the form of a heterologous fusion protein of an effector section with an adaptor protein or polypeptide section which makes binding possible to the component of the membrane via one or more of the adaptor proteins, can bind to the mediator section.

7. The transformed yeast cell as claimed in claim 6,
**characterized in that**
the mediator section comprises the cytoplasmic part of the EGFR (epidermal growth factor receptor) or is derived from the latter.

8. The transformed yeast cell as claimed in any one of claims 1 to 7,
**characterized in that**
the yeast cell is a yeast cell lacking cell walls.

9. The transformed yeast cell as claimed in any one of claims 1 to 8,
**characterized in that**
the heterologous membrane receptor is necessary for activation of a Ras or Ras-like signal pathway in the transformed yeast cell.

10. An *in vivo* assay for detecting the presence of a ligand for a ligand-binding section of a receptor in a sample to be investigated for the ligand, **characterized by** the following steps:
(a) contacting the sample with transformed yeast cells as claimed in claim 9 under conditions with which the Ras or Ras-like signal pathway in the cell cannot be activated in the absence of the heterologous membrane receptor, where the heterologous membrane receptor contains said ligand-binding section, and the effector protein or polypeptide whose binding to a membrane component depends on the binding or, alternatively, lack of binding of a ligand to the ligand-binding section of the heterologous membrane receptor, as defined in claim 1, is able to activate this Ras or Ras-like signal pathway,
(b) investigating whether activation of the Ras or Ras-like signal pathway has taken place,
(c) where, in the event of the binding of the effector protein or polypeptide being dependent on the binding of a ligand to the ligand-binding section of the heterologous membrane receptor, a detection of the activation of the Ras or Ras-like signal pathway indicates the absence of a ligand or a ligand-binding section of a receptor in the sample,
and where, in the event of the binding of the effector protein or polypeptide being dependent on the lack of binding of a ligand to the ligand-binding section of the heterologous membrane receptor, cells as employed in step (a) are investigated under conditions with which the Ras or Ras-like signal pathway in the transformed yeast cell cannot be activated in the absence of the heterologous membrane receptor, for activation of the Ras or Ras-like signal pathway in the absence of the sample,
where a detection of the activation of the Ras or Ras-like signal pathway in the absence of the sample and the inactivity of the Ras or Ras-like signal pathway in the presence of the sample indicates the presence of a ligand for the ligand-binding section of a receptor in the sample.

11. The assay as claimed in claim 10,
**characterized in that**
the test substance (a) is a naturally occurring substance or (b) a non-naturally occurring substance.

12. The assay as claimed in claim 11,
**characterized in that**
the naturally occurring substance is an odorant, flavoring, peptide, peptide hormone, protein, growth factor, neurotransmitter, non-protein- or -peptide-like hormone and/or a vitamin and the non-naturally occurring substance is a synthetic derivative of a natural ligand or is a poison.

13. A screening method for unknown ligands of a particular receptor,
**characterized in that**
an assay method as claimed in claim 10 is employed for the screening.

14. An *in vivo* assay for detecting whether a compound is able to alter a binding activity of a ligand-binding section of a receptor in relation to a ligand, **characterized by** the following steps:
(a) contacting the ligand in the presence of the compound with transformed yeast cells as claimed in claim 9 under conditions with which in the absence of the heterologous membrane receptor the Ras or Ras-like signal pathway in the cell cannot be activated, where the heterologous membrane receptor contains said ligand-binding section, and the effector protein or polypeptide whose binding to a membrane component depends on the binding or, alternatively, lack of binding of a ligand to the ligand-binding section of the heterologous membrane receptor, as defined in claim 1, is able to activate this Ras or Ras-like signal pathway,
(b) investigating whether and, where appropriate, to what extent activation of the Ras or Ras-like signal pathway takes place,
(c) comparing the result of the investigation in step (b) with a result of an investigation obtained when the assay is carried out in the absence of the compound.

15. An *in vivo* assay for detecting whether a polypeptide or protein has a ligand-binding function of a receptor, **characterized by** the following steps:
(a) contacting transformed yeast cells as claimed in claim 9 with the ligand under conditions with which in the absence of the heterologous membrane receptor, as defined in claim 1, a Ras or Ras-like signal pathway in the cells cannot be activated, where the ligand-binding section of the heterologous membrane receptor comprises the polypeptide or protein to be investigated or consists thereof, and where the effector protein or polypeptide whose binding to a membrane component depends on the binding or, alternatively, lack of binding of a ligand to the ligand-binding section of the heterologous membrane receptor is able to activate this Ras or Ras-like signal pathway,
(b) investigating whether an activation of the Ras or Ras-like signal pathway has taken place,
where, in the event of the binding of the effector protein or polypeptide being dependent on the binding of a ligand to the ligand-binding section of the heterologous membrane receptor, a detection of the activation of the Ras or Ras-like signal pathway indicates that the ligand-binding section of the heterologous membrane receptor and, accordingly, the polypeptide or protein to be investigated has a ligand-binding function of a receptor, and where, in the event of the binding of the effector protein or polypeptide being dependent on the lack of binding of a ligand to the ligand-binding section of the heterologous membrane receptor, cells as employed in step (a) are investigated under conditions with which the Ras or Ras-like signal pathway in the transformed yeast cell cannot be activated in the absence of the heterologous membrane receptor, for activation of the Ras or Ras-like signal pathway in the absence of the ligand,
where a detection of the activation of the Ras or Ras-like signal pathway in the absence of the ligand and the inactivity of the Ras or Ras-like signal pathway in the presence of the ligand indicates that the ligand-binding section of the heterologous membrane receptor and, accordingly, the polypeptide or protein to be investigated has a ligand-binding function of a receptor.

16. A kit for use in an assay as claimed in any one of claims 10 to 14, which comprises components (a), (b) and (c) indicated below:
(a) yeast cells in which a Ras or Ras-like signal pathway cannot be activated at least under certain conditions;
(b) a nucleic acid vector which comprises, in suitable arrangement:
- a DNA section which encodes a membrane-localization signal of a heterologous membrane receptor, as defined in claim 1;
- a DNA section which encodes a mediator section of a heterologous membrane receptor, as defined in claim 1; and
- a suitably arranged insertion site for functional insertion of a DNA sequence which encodes a ligand-binding section, as defined in claim 1,
where, after insertion of a DNA sequence for the ligand-binding section, the nucleic acid vector comprises a complete expressible gene for a heterologous membrane receptor, as defined in claim 1, where the effector protein or polypeptide whose binding to a membrane component depends on the binding or, alternatively, lack of binding of a ligand to the ligand-binding section of the heterologous membrane receptor is able to activate the inactive Ras or Ras-like signal pathway in the yeast cells mentioned under (a);
(c) a nucleic acid vector into which is expressibly inserted a DNA sequence which encodes the effector protein or polypeptide which, in the event of ligand binding or, alternatively, lack of ligand binding to the ligand-binding section of the heterologous membrane receptor, is able to bind to a component of the membrane, where appropriate via other proteins or polypeptides (adaptors), and which is in the form of a heterologous fusion protein of an effector section with an adaptor protein or polypeptide which makes binding possible to the component of the membrane, where appropriate via other proteins or polypeptides (adaptors).

17. A kit for use in an assay as claimed in claim 15, which comprises components (a), (b) and (c) indicated below:
(a) yeast cells in which a Ras or Ras-like signal pathway cannot be activated at least under certain conditions;
(b) a nucleic acid vector which comprises, in suitable arrangement:
- a DNA section which encodes a membrane-localization signal of a heterologous membrane receptor, as defined in claim 1;
- a DNA section which encodes a mediator section of a heterologous membrane receptor, as defined in claim 1; and
- a suitably arranged insertion site for functional insertion of a DNA sequence which encodes a polypeptide or protein to be investigated for a ligand-binding function of a receptor,
where, after insertion of a DNA sequence for the ligand-binding section, the nucleic acid vector comprises a complete expressible gene for a heterologous membrane receptor, where the effector protein or polypeptide whose binding to a membrane component depends on the binding or, alternatively, lack of binding of a ligand to the ligand-binding section, formed from the polypeptide or protein to be investigated for a ligand-binding function of a receptor, of the membrane receptor is able to activate the inactive Ras or Ras-like signal pathway in the yeast cells mentioned under (a);
(c) a nucleic acid vector into which is expressibly inserted a DNA sequence which encodes the effector protein or polypeptide which, in the event of ligand binding or, alternatively, lack of ligand binding to the ligand-binding section of the heterologous membrane receptor, is able to bind to a component of the membrane, where appropriate via other proteins or polypeptides (adaptors), and which is in the form of a heterologous fusion protein of an effector section with an adaptor protein or polypeptide which makes binding possible to the component of the membrane, where appropriate via other proteins or polypeptides (adaptors).

18. The kit as claimed in claim 16 or 17,
**characterized in that**
it additionally comprises a nucleic acid vector into which is expressibly inserted a DNA sequence which encodes at least one adaptor protein, via which the effector protein or polypeptide is able, when there is binding or, alternatively, lack of binding of a ligand to the ligand-binding section of the heterologous membrane receptor, to bind to a component of the membrane.

19. The kit as claimed in any one of claims 16 to 18,
**characterized in that**
it additionally contains a transformation or transfection vector with a construct comprising a binding site for a transcription factor whose activation results from an activation of a specific Ras or Ras-like signal pathway whose activation is to be detected by the assay, a minimal promoter and an insertion site, suitably arranged for expression controlled by the minimal promoter, for insertion of a reporter gene, where the minimal promoter is activated as a result of a binding of the activated transcription factor to its binding site.

20. A method for identifying polypeptides or proteins which have a ligand-binding function of a receptor, which comprises:
- preparing a transformed yeast cell as claimed in claim 1 with a heterologous membrane receptor having the features described in claim 1 and comprising the whole of such a polypeptide or protein or a part of such a polypeptide or protein which presumably contains the sequence sections essential for the ligand-binding function, and
- using this transformed yeast cell to carry out an *in vivo* assay method for detecting whether a polypeptide or protein has a ligand-binding function of a receptor, as claimed in claim 15.

## Revendications

1. Cellule de levure transformée, comprenant un récepteur membranaire hétérologue qui comprend un tronçon de liaison à un ligand, un signal de localisation à la membrane et un tronçon médiateur, dans laquelle une modification de structure est produite avec des répercussions sur le tronçon médiateur seulement en présence d'une liaison ou, en variante, seulement en l'absence de liaison d'un ligand sur le tronçon de liaison au ligand, de telle sorte que par la suite, une protéine ou un polypeptide effecteur, qui est en mesure d'activer dans la cellule la voie de signalisation Ras ou une voie similaire, se lie à un composant de la membrane, le cas échéant par l'intermédiaire d'autres protéines ou polypeptides (adaptateurs),
**caractérisée en ce que**
la protéine ou le polypeptide effecteur qui est en mesure d'activer la voie de signalisation Ras ou une voie similaire, se présente sous la forme d'une protéine hétérologue de fusion d'un tronçon effecteur avec une protéine ou un polypeptide adaptateur qui permettent la liaison à le composant de la membrane, le cas échéant par l'intermédiaire d'autres protéines ou polypeptides (adaptateurs), le tronçon effecteur de la protéine de fusion comprenant la séquence d'une protéine Ras constitutivement active.

2. Cellule de levure transformée selon la revendication 1,
**caractérisée en ce que**
le récepteur hétérologue membranaire est un récepteur transmembranaire, un récepteur couplé à une enzyme, un récepteur couplé à une protéine G, un récepteur 7-transmembranaire ou un récepteur olfactif (« odour receptor » ou « olfactorial receptor »).

3. Cellule de levure transformée selon la revendication 1,
**caractérisée en ce que**
le tronçon de liaison à un ligand est dérivé par mutation d'un tronçon de liaison à un ligand d'un récepteur existant dans la nature ou est un tronçon de liaison à un ligand synthétique, produit par « molecular modelling ».

4. Cellule de levure transformée selon la revendication 3,
**caractérisée en ce que**
le tronçon de liaison à un ligand d'un récepteur existant dans la nature est le tronçon de liaison à un ligand d'un récepteur transmembranaire, d'un récepteur couplé par protéine G, d'un récepteur 7-transmembranaire, d'un récepteur olfactif (« odour receptor » ou « olfactorial receptor ») ou d'un récepteur nucléaire.

5. Cellule de levure transformée selon la revendication 3,
**caractérisée en ce que**
la mutation est une substitution, une délétion, une insertion et/ou une modification d'un ou de plusieurs acides aminés ou d'un ou de plusieurs groupes d'acides aminés.

6. Cellule de levure transformée selon l'une quelconque des revendications 1 à 5,
**caractérisée en ce que**
par suite de la liaison au ligand ou, en variante par suite de l'absence de liaison au ligand sur le tronçon de liaison au ligand, le tronçon médiateur est en mesure de lier une ou plusieurs protéines adaptatrices, par l'intermédiaire desquelles la protéine ou le polypeptide effecteur, qui est en mesure d'activer dans la cellule la voie de signalisation Ras ou une voie similaire, peut se lier au tronçon médiateur sous la forme d'une protéine hétérologue de fusion d'un tronçon effecteur avec une protéine adaptatrice ou avec un tronçon adaptateur d'un polypeptide, qui permet la liaison au composant de la membrane par l'intermédiaire d'une ou de plusieurs des protéines adaptatrices.

7. Cellule de levure transformée selon la revendication 6,
**caractérisée en ce que**
le tronçon médiateur comprend la partie cytoplasmique du EGFR (« epidermal growth factor receptor ») ou provient de cette dernière.

8. Cellule de levure transformée selon l'une quelconque des revendications 1 à 7,
**caractérisée en ce que**
la cellule de levure est une cellule de levure sans paroi cellulaire.

9. Cellule de levure transformée selon l'une quelconque des revendications 1 à 8,
**caractérisée en ce que**
le récepteur membranaire hétérologue est nécessaire pour l'activation de la voie de signalisation Ras ou une voie similaire dans la cellule de levure transformée.

10. Analyse *in vivo* pour la détection de la présence d'un ligand pour un tronçon de liaison à un ligand d'un récepteur dans un échantillon devant être étudié quant au ligand, **caractérisée par** les étapes suivantes :
(a) mise en contact de l'échantillon avec des cellules de levure transformées selon la revendication 9 sous des conditions sous lesquelles, en l'absence du récepteur membranaire hétérologue, la voie de signalisation Ras ou une voie similaire ne peut pas être activée, le récepteur membranaire hétérologue contenant le tronçon de liaison au ligand susmentionné et la protéine ou le polypeptide effecteur, dont la liaison à un composant de membrane dépend de la liaison ou, en variante, de l'absence de liaison, d'un ligand sur le tronçon de liaison au ligand du récepteur membranaire hétérologue, tels qu'ils sont définis dans la revendication 1, étant en mesure d'activer cette voie de signalisation Ras ou une voie similaire,
(b) étude pour déterminer si une activation de la voie de signalisation Ras ou d'une voie similaire a eu lieu,
(c) dans laquelle, pour le cas où la liaison de la protéine ou du polypeptide effecteur dépend de la liaison d'un ligand sur le tronçon de liaison au ligand du récepteur membranaire hétérologue, une détection de l'activation de la voie de signalisation Ras ou d'une voie similaire indique l'absence d'un ligand ou d'un tronçon de liaison au ligand,
et dans laquelle, pour le cas où la liaison de la protéine ou du polypeptide effecteur dépend de l'absence de liaison d'un ligand sur le tronçon de liaison au ligand du récepteur membranaire hétérologue, des cellules telles qu'elles ont été utilisées à l'étape (a), sous des conditions auxquelles, en cas d'absence du récepteur membranaire hétérologue, la voie de signalisation Ras ou une voie similaire dans la cellule de levure transformée ne peut pas être activée, sont étudiées en présence de l'échantillon quant à l'activation de la voie de signalisation Ras ou d'une voie similaire,
dans laquelle une détection de l'activation de la voie de signalisation Ras ou d'une voie similaire en l'absence de l'échantillon et l'inactivité de la voie de signalisation Ras ou d'une voie similaire en présence de l'échantillon indiquent la présence dans l'échantillon d'un ligand pour le tronçon de liaison au ligand d'un récepteur.

11. Analyse selon la revendication 10,
**caractérisée en ce que**
la substance à tester est (a) une substance d'origine naturelle ou (b) une substance ne se rencontrant pas dans la nature.

12. Analyse selon la revendication 11,
**caractérisée en ce que**
la substance d'origine naturelle est une substance odoriférante, un agent de sapidité, un peptide, une hormone peptidique, un facteur de croissance, un neurotransmetteur, une hormone de nature non protéique ou non peptidique et/ou une vitamine, et **en ce que** la substance ne se rencontrant pas dans la nature est un dérivé synthétique d'un ligand naturel ou un poison.

13. Procédé de dépistage de ligands inconnus d'un récepteur particulier,
**caractérisé en ce que,**
pour le dépistage, on utiliser un procédé d'analyse selon la revendication 10.

14. Analyse *in vivo* pour détecter si un composé est en mesure de modifier une activité de liaison d'un tronçon de liaison à un ligand d'un récepteur par rapport à un ligand, **caractérisé par** les étapes suivantes :
(a) mise en contact du ligand en présence du composé avec des cellules de levure transformées selon la revendication 9 sous des conditions sous lesquelles en l'absence du récepteur membranaire hétérologue, la voie de signalisation Ras ou une voie similaire dans la cellule ne peut pas être activée, le récepteur membranaire hétérologue contenant le tronçon de liaison au ligand susmentionné, et la protéine ou le polypeptide effecteur, dont la liaison à un composant de membrane dépend de la liaison ou, en variante, de l'absence de liaison, d'un ligand sur le tronçon de liaison au ligand du récepteur membranaire hétérologue, tels qu'ils sont définis dans la revendication 1, étant en mesure d'activer cette voie de signalisation Ras ou une voie similaire,
(b) étude pour déterminer si une activation de la voie de signalisation Ras ou d'une voie similaire a eu lieu et, le cas échéant, dans quelle mesure,
(c) comparaison du résultat de l'étude de l'étape (b) à un résultat d'étude qui a été obtenu lors de l'exécution de l'essai en l'absence du composé.

15. Analyse *in vivo* pour détecter si un polypeptide ou une protéine comporte une fonction de liaison à un ligand d'un récepteur, **caractérisé par** les étapes suivantes :
(a) mise en contact des cellules de levure transformées selon la revendication 9 avec le ligand, sous des conditions sous lesquelles, en l'absence du récepteur membranaire hétérologue tel qu'il est défini à la revendication 1, la voie de signalisation Ras ou une voie similaire dans la cellule ne peut pas être activée, le tronçon de liaison au ligand du récepteur membranaire hétérologue comprenant la protéine ou le polypeptide effecteur ou étant constitué de ces derniers, et la protéine ou le polypeptide effecteur, dont la liaison sur un composant de la membrane dépend de la liaison ou, en variante, de l'absence de liaison, d'un ligand sur le tronçon de liaison au ligand du récepteur membranaire hétérologue, étant en mesure d'activer cette voie de signalisation Ras ou une voie similaire,
(b) étude pour déterminer si une activation du chemin de la voie de signalisation Ras ou d'une voie similaire a eu lieu,
dans laquelle, pour le cas où la liaison de la protéine ou du polypeptide effecteur dépend de la liaison d'un ligand sur le tronçon de liaison au ligand du récepteur membranaire hétérologue, une détection de l'activation de la voie de signalisation Ras ou d'une voie similaire indique que le tronçon de liaison au ligand du récepteur membranaire hétérologue, et en conséquence le polypeptide ou la protéine qui doivent être étudiés, présentent une fonction de liaison pour un ligand d'un récepteur et,
pour le cas où la liaison de la protéine ou du polypeptide effecteur dépend de l'absence de liaison d'un ligand sur le tronçon de liaison au ligand du récepteur membranaire hétérologue, des cellules telles qu'elles ont été utilisées à l'étape (a), sous des conditions sous lesquelles, en cas d'absence du récepteur membranaire hétérologue, la voie de signalisation Ras ou une voie similaire dans la cellule de levure transformée ne peut pas être activée, sont étudiées en l'absence de ligand quant à l'activation de la voie de signalisation Ras ou d'une voie similaire, dans laquelle une détection de l'activation de la voie de signalisation Ras ou d'une voie similaire en présence du ligand indiquent que le tronçon de liaison au ligand du récepteur membranaire hétérologue et en conséquence le polypeptide ou la protéine à étudier comportent une fonction de liaison au ligand d'un récepteur.

16. Kit pour l'utilisation dans une analyse selon l'une quelconque des revendications 10 à 14, lequel comporte les composants (a), (b) et (c) définis ci-après :
(a) des cellules de levure dans lesquelles la voie de signalisation Ras ou une voie similaire ne peut pas être activée, au moins sous certaines conditions,
(b) un vecteur d'acide nucléique, qui comprend dans un agencement approprié :
- un fragment d'ADN qui code pour un signal de localisation de membrane d'un récepteur membranaire hétérologue tel qu'il est défini à la revendication 1 ;
- un fragment d'ADN qui code pour un tronçon médiateur d'un récepteur membranaire hétérologue tel qu'il est défini à la revendication 1 et
- un site d'insertion disposé de façon appropriée pour l'insertion fonctionnelle d'une séquence d'ADN qui code pour un tronçon de liaison au ligand tel qu'il est défini à la revendication 1,
dans lequel après l'insertion d'une séquence d'ADN pour le tronçon de liaison au ligand, le vecteur d'acide nucléique comprend un gène complètement exprimable pour un récepteur membranaire hétérologue tel qu'il est défini à la revendication 1,
dans lequel la protéine ou le polypeptide effecteur, dont la liaison à un composant de la membrane dépend de la liaison ou, en variante, de l'absence de liaison d'un ligand sur le tronçon de liaison au ligand du récepteur membranaire hétérologue, sont en mesure d'activer la voie inactive de signalisation Ras ou une voie similaire dans les cellules de levure mentionnées en (a),
(c) un vecteur d'acide nucléique dans lequel est insérée de façon exprimable une séquence d'ADN qui code pour la protéine ou le polypeptide effecteur qui, dans le cas d'une liaison au ligand ou, en variante, d'une absence de liaison au ligand, peut se lier sur le tronçon de liaison au ligand du récepteur membranaire hétérologue sur un composant de la membrane, le cas échéant par l'intermédiaire d'autres protéines ou d'autres polypeptides (adaptateurs), et qui se présente sous la forme d'une protéine de fusion hétérologue d'un tronçon effecteur avec une protéine ou un polypeptide adaptateur qui permettent la liaison au composant de la membrane, le cas échéant par l'intermédiaire d'autres protéines ou d'autres polypeptides (adaptateurs).

17. Kit pour l'utilisation dans une analyse selon revendication 15, lequel comporte les composants (a), (b) et (c) précisés ci-après :
(a) des cellules de levure dans lesquelles la voie de signalisation Ras ou une voie similaire ne peut pas être activée, au moins sous certaines conditions,
(b) un vecteur d'acide nucléique, qui comprend dans un agencement approprié :
- un fragment d'ADN qui code pour un signal de localisation de membrane d'un récepteur membranaire hétérologue tel qu'il est défini à la revendication 1 ;
- un fragment d'ADN qui code pour un tronçon médiateur d'un récepteur membranaire hétérologue tel qu'il est défini à la revendication 1 et
- un site d'insertion disposé de façon appropriée pour l'insertion fonctionnelle d'une séquence d'ADN qui code pour un polypeptide ou une protéine à étudier quant à une fonction de liaison au ligand d'un récepteur,
dans lequel après l'insertion d'une séquence d'ADN pour le tronçon de liaison au ligand, le vecteur d'acide nucléique comprend un gène complètement exprimable pour un récepteur membranaire hétérologue, dans lequel la protéine ou le polypeptide effecteur, dont la liaison à un composant de membrane dépend de la liaison ou, en variante, de l'absence de liaison d'un ligand sur le tronçon de liaison au ligand du récepteur membranaire hétérologue, tronçon formé à partir du polypeptide ou de la protéine à étudier quant à une fonction de liaison au ligand d'un récepteur, sont en mesure d'activer la voie de signalisation Ras ou une voie similaire dans les cellules de levure mentionnées en (a),
(c) un vecteur d'acide nucléique dans lequel est insérée de façon exprimable une séquence d'ADN qui code pour la protéine ou le polypeptide effecteur qui, dans le cas d'une liaison au ligand ou, en variante, d'une absence de liaison par ligand, peut se lier au tronçon de liaison au ligand du récepteur membranaire hétérologue sur un composant de la membrane, le cas échéant par l'intermédiaire d'autres protéines ou d'autres polypeptides (adaptateurs), et qui se présente sous la forme d'une protéine de fusion hétérologue d'un tronçon effecteur avec une protéine ou un polypeptide adaptateur qui permettent la liaison au composant de la membrane, le cas échéant par l'intermédiaire d'autres protéines ou d'autres polypeptides (adaptateurs).

18. Kit selon l'une quelconque des revendications 16 ou 17,
**caractérisé en ce**
**qu'**il comprend en plus un vecteur d'acide nucléique dans lequel une séquence d'ADN est insérée de façon exprimable, qui code pour au moins une protéine adaptatrice par laquelle la protéine ou le polypeptide effecteur peut se lier sur un composant de la membrane en cas de liaison ou, en variante, d'absence de liaison, d'un ligand sur le tronçon de liaison au ligand du récepteur membranaire hétérologue.

19. Kit selon l'une quelconque des revendications 16 à 18,
**caractérisé en ce**
**qu'**il contient en plus un vecteur de transformation ou de transfection avec une construction comprenant un site de liaison pour un facteur de transcription dont l'activation s'effectue par suite d'une activation de la voie de signalisation Ras ou d'une voie similaire, dont l'activation doit être détectée par l'analyse, un promoteur minimal, et un site d'insertion pour une insertion d'un gène rapporteur disposé de façon appropriée pour une expression commandée par le promoteur minimal, le promoteur minimal étant activé à la suite d'une liaison du facteur de transcription à son site de liaison.

20. Procédé pour l'identification de polypeptides ou de protéines qui comportent une fonction de ligand d'un récepteur, qui comprend :
- de produire une cellule de levure transformée selon la revendication 1, avec un récepteur membranaire hétérologue qui présente les caractéristiques décrites à la revendication 1, et qui comprend l'ensemble d'un tel polypeptide ou d'une telle protéine ou une partie d'un tel polypeptide ou d'une telle protéine que l'on pense contenir les tronçons de séquence essentiels pour la fonction ligand, et
- au moyen de cette cellule de levure transformée, d'exécuter selon la revendication 15 un procédé d'analyse *in vivo* pour détecter si un polypeptide ou une protéine comporte une fonction de ligand d'un récepteur.
